# EUROPEAN PATENT APPLICATION

(11) **EP 2 757 086 A1**
(43) Date of publication of application: **23.07.2014**
(21) Application number: 13198696.0
(22) Date of filing: 20.12.2013
(51) Int. Cl.: C07C 13/547, H01L 51/00, C09K 11/06, C07D 403/14, C07D 263/57, C07D 209/08, C07D 277/66, C07D 209/86, C07C 211/60, C07C 49/792, C07C 13/567, C07C 13/62, C07C 13/66, C07D 213/38

(54) **Hydrocarbon-based fused ring compound and organic light emitting device using the same**

(30) Priority: 16.01.2013 KR 20130005100; 20.11.2013 KR 20130141774
(71) Applicant: Heesung Material Ltd., Yongin-City 449-884 (KR)
(72) Inventor: Lee, Jun-A, 449-884 Yongin-city (KR); Park, Geon-Yu, 200-936 Chuncheon-city (KR); Yang, Jeong-Hoon, 451-726 Pyeongtaek-city (KR); Eum, Sung-Jin, 448-150 Yongin-city (KR); Lee, Joo-Dong, 463-763 Seongnam-city (KR)
(74) Representative: Goddar, Heinz J.

(57) **Abstract**

The present invention relates to a hydrocarbon-based fused ring compound and an organic light emitting device including the same.

## Description

### Field of the Invention

The present invention relates to a novel hydrocarbon-based fused ring compound and an organic light emitting device including the same.

### Background of the Invention

An electroluminescent device is one type of self-luminescent-type display devices, and has advantages in that the device has a wide viewing angle, an excellent contrast, and quick response time.

An organic light emitting device has a structure in which an organic thin film is disposed between two electrodes. When voltage is applied to an organic light emitting device having such a structure, light emits by electrons and holes injected from the two electrodes being dissipated after the electrons and holes are bonded and make a pair in the organic thin film. The organic thin film may be formed as a monolayer or a multilayer as necessary.

Materials of an organic thin film may have a light emitting function when necessary. For example, as the material of an organic thin film, compounds capable of forming a light emitting layer alone may be used, or compounds capable of performing as a host or a dopant of a host-dopant-based light emitting layer may also be used. In addition to these, compounds capable of performing hole injection, hole transfer, electron blocking, hole blocking, electron transfer, electron injection, or the like, may also be used as the material of an organic thin film.

There have been continuous demands for the development of organic thin film materials in order to improve the performance, life span or efficiency of an organic light emitting device.

### Summary of the Invention

The present invention provides a novel hydrocarbon-based fused ring compound and an organic light emitting device including the same.

The present invention provides a compound of the following Chemical Formula 1: 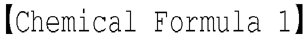 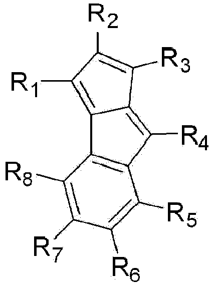 In Chemical Formula 1,
R₁ to R₈ are selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or form a substituted or unsubstituted monocyclic or multicyclic aliphatic or aromatic hydrocarbon ring, or a substituted or unsubstituted monocyclic or multicyclic aliphatic or aromatic heteroring, by being linked to an adjacent group.

In addition, the present invention provides an organic light emitting device that includes an anode, a cathode and one or more organic material layers provided between the anode and the cathode, wherein one or more layers of the organic material layers include the compound of Chemical Formula 1.

### Advantageous Effects

Compounds described in the present specification may be used as the material of an organic material layer of an organic light emitting device. The compound may be used as a hole injection material, a hole transfer material, a light emitting material, an electron transfer material, an electron injection material, or the like, in an organic light emitting device. In particular, the compound may be used as the material of a light emitting layer of an organic light emitting device. Specifically, the compound may be used alone as a light emitting material, or as a host material or a dopant material of a light emitting layer.

### Brief Description of the Drawings

Figs. 1 to 3 illustrate the laminating order of electrodes and organic material layers of an organic light emitting device according to embodiments of the present invention.
Fig. 4 shows UV data of Compound 353 by a diagram.
Fig. 5 shows PL data of Compound 353 by a diagram.

### Detailed Description of the Embodiments

Hereinafter, the present invention will be described in detail.

A compound described in the present specification may be represented by Chemical Formula 1. The compound according to the present invention may be used as the material of an organic material layer of an organic light emitting device depending on the structural and physical properties of a core structure.

In the present specification, halogen includes F, Cl, Br and I.

In the present specification, alkyl includes linear or branched alkyl having 1 to 60 carbon atoms, and may be further substituted with other substituents. The number of carbon atoms of the alkyl may be 1 to 60, specifically 1 to 40, and more specifically 1 to 20.

In the present specification, alkenyl includes linear or branched alkenyl having 2 to 60 carbon atoms, and may be further substituted with other substituents. The number of carbon atoms of the alkenyl may be 2 to 60, specifically 2 to 40, and more specifically 2 to 20.

In the present specification, alkynyl includes linear or branched alkynyl having 2 to 60 carbon atoms, and may be further substituted with other substituents. The number of carbon atoms of the alkynyl may be 2 to 60, specifically 2 to 40, and more specifically 2 to 20.

In the present specification, alkoxy includes linear or branched alkoxy having 1 to 60 carbon atoms, and may be further substituted with other substituents. The number of carbon atoms of the alkoxy may be 1 to 60, specifically 1 to 40, and more specifically 1 to 20.

In the present specification, cycloalkyl includes monocyclic or multicyclic cycloalkyl having 3 to 60 carbon atoms, and may be further substituted with other substituents. Herein, multicyclic means a group in which cycloalkyl is directly bonded to or fused with other ring groups. Herein, the other ring groups may be cycloalkyl, but may also be other types of ring groups, for example, heterocycloalkyl, aryl, heteroaryl or the like. The number of carbon atoms of the cycloalkyl may be 3 to 60, specifically 3 to 40, and more specifically 5 to 20.

In the present specification, heterocycloalkyl includes S, O or N as a heteroatom, includes monocyclic or multicyclic heterocycloalkyl having 2 to 60 carbon atoms, and may be further substituted with other substituents. Herein, multicyclic means a group in which heterocycloalkyl is directly bonded to or fused with other ring groups. Herein, the other ring groups may be heterocycloalkyl, but may also be other types of ring groups, for example, cycloalkyl, aryl, heteroaryl or the like. The number of carbon atoms of the heterocycloalkyl may be 2 to 60, specifically 2 to 40, and more specifically 3 to 20. As one example, the heterocycloalkyl group includes a 10,11-dihydro-dibenzo[b,f]azepin group, indolinyl, or a 9,10-dihydroacridine group.

In the present specification, aryl includes monocyclic or multicyclic aryl having 6 to 60 carbon atoms, and may be further substituted with other substituents. Herein, multicyclic means a group in which aryl is directly bonded to or fused with other ring groups. Herein, the other ring groups may be aryl, but may also be other types of ring groups, for example, cycloalkyl, heterocycloalkyl, heteroaryl or the like. The number of carbon atoms of the aryl may be 6 to 60, specifically 6 to 40, and more specifically 6 to 20. Specific examples of the aryl include phenyl, biphenyl, triphenyl, naphthyl, anthryl, chrysenyl, phenanthrenyl, perylenyl, fluoranthenyl, triphenylenyl, phenalenyl, pyrenyl, tetracenyl, pentacenyl, fluorenyl, indenyl, acenaphthylenyl or the like, or fused rings thereof, but are not limited thereto.

In the present specification, heteroaryl includes S, O or N as a heteroatom, includes monocyclic or multicyclic heteroaryl having 2 to 60 carbon atoms, and may be further substituted with other substituents. Herein, multicyclic means a group in which heteroaryl is directly bonded to or fused with other ring groups. Herein, the other ring groups may be heteroaryl, but may also be other types of ring groups, for example, cycloalkyl, heterocycloalkyl, aryl or the like. The number of carbon atoms of the heterocycloalkyl may be 2 to 60, specifically 2 to 40, and more specifically 3 to 20. Specific examples of the heteroaryl include pyridyl, pyrolyl, pyrimidyl, pyridazinyl, furanyl, thienyl, imidazolyl, pyrazolyl, oxazolyl, isoxazolyl, thiazolyl, isothiazolyl, triazolyl, furazanyl, oxadiazolyl, thiadiazolyl, dithiazolyl, tetrazolyl, pyranyl, thiopyranyl, diazinyl, oxazinyl, thiazinyl, dioxynyl, triazinyl, tetrazinyl, quinolyl, isoquinolyl, quinazolinyl, isoquinazolinyl, acridinyl, phenanthridinyl, imidazopyridinyl, diazanaphthalenyl, triazaindene, indolyl, benzothiazolyl, benzoxazolyl, benzimidazolyl, a benzothiophene group, a benzofuran group, a dibenzothiophene group, a dibenzofuran group, carbazolyl, benzocarbazolyl, phenazinyl or the like, or fused rings thereof, but are not limited thereto.

In the present specification, the spiro group is a group including a spiro structure, and may have 10 to 60 carbon atoms. For example, the spiro group may include a structure in which a 2,3-dihydro-1H-indene group or a cyclohexane group is spiro-bonded to a fluorene group. Specifically, the spiro group includes a group of the following structural formulae.

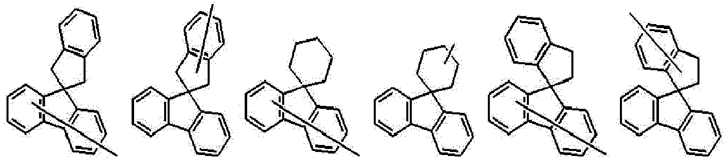

In the present specification, "substituted or unsubstituted" means being substituted with one or more substituents selected from the group consisting of halogen; cyano; linear or branched C₁ to C₆₀ alkyl; linear or branched C₂ to C₆₀ alkenyl; linear or branched C₂ to C₆₀ alkynyl; linear or branched C₁ to C₆₀ haloalkyl; linear or branched C₂ to C₆₀ haloalkenyl; linear or branched C₂ to C₆₀ haloalkynyl; linear or branched C₁ to C₆₀ alkoxy; linear or branched C₂ to C₆₀ alkenyloxy; linear or branched C₂ to C₆₀ alkynyloxy; monocyclic or multicyclic C₃ to C₆₀ cycloalkyl; monocyclic or multicyclic C₂ to C₆₀ heterocycloalkyl; monocyclic or multicyclic C₆ to C₆₀ aryl; monocyclic or multicyclic C₂ to C₆₀ heteroaryl; monocyclic or multicyclic C₂ to C₆₀ heterocycloalkyl; monocyclic or multicyclic C₆ to C₆₀ aryloxy; monocyclic or multicyclic C₂ to C₆₀ heteroaryloxy; an acetophenone group; a benzophenone group; a C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or being unsubstituted. These additional substituents may be further substituted additionally.

According to one embodiment of the present invention, in Chemical Formula 1, R₁ to R₈ are the same as or different from each other, and each is selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aliphatic or aromatic hydrocarbon ring or a substituted or unsubstituted monocyclic or multicyclic aliphatic or aromatic heteroring, by being linked to an adjacent group, however, not all of R₁ to R₈ are hydrogen.

According to one embodiment of the present invention, in Chemical Formula 1, at least one of R₁ to R₈ is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aromatic hydrocarbon ring, or a substituted or unsubstituted monocyclic or multicyclic aromatic heteroring, by being linked to an adjacent group.

According to one embodiment of the present invention, in Chemical Formula 1, R₁ to R₃ are the same as or different from each other, and each is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aromatic hydrocarbon ring, or a substituted or unsubstituted monocyclic or multicyclic aromatic heteroring, by being linked to an adjacent group.

According to one embodiment of the present invention, in Chemical Formula 1, R₁ to R₃ are the same as or different from each other, each is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₂ and R₃ are the same as each other, and are substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₁ is the same as R₂ and R₃, and is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₁ is different from R₂ and R₃, and is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₄ is selected from the group consisting of hydrogen; substituted or unsubstituted linear or branched C₁ to C₆₀ alkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₄ is selected from the group consisting of hydrogen; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, at least one of R₆ and R₇ is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aromatic hydrocarbon ring, or a substituted or unsubstituted monocyclic or multicyclic aromatic heteroring, by being linked to an adjacent group.

According to one embodiment of the present invention, in Chemical Formula 1, R₆ and R₇ are the same as or different from each other, and each is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aromatic hydrocarbon ring, or a substituted or unsubstituted monocyclic or multicyclic aromatic heteroring, by being linked to an adjacent group.

According to one embodiment of the present invention, in Chemical Formula 1, R₆ and R₇ are the same as each other, and are substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1, R₅ and R₈ are hydrogen; substituted or unsubstituted linear or branched C₁ to C₆₀ alkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, Chemical Formula 1 may be represented by any one of the following Chemical Formulae 1a to 1g. 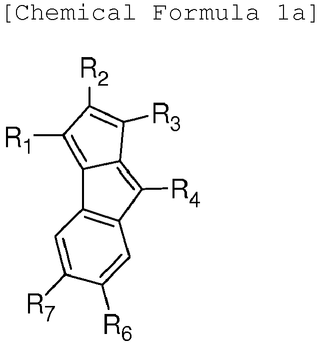 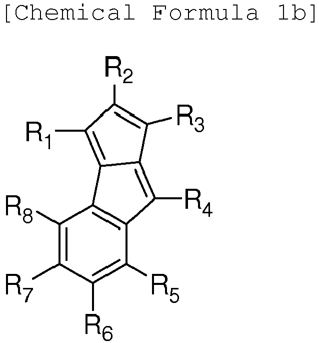 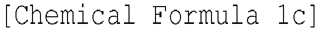 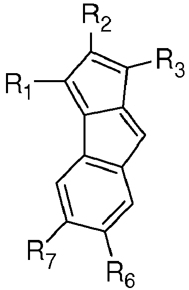 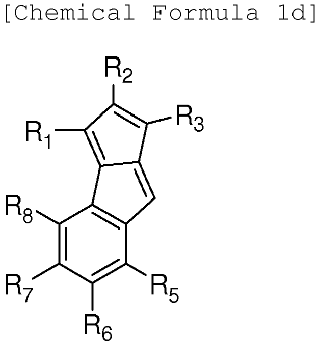 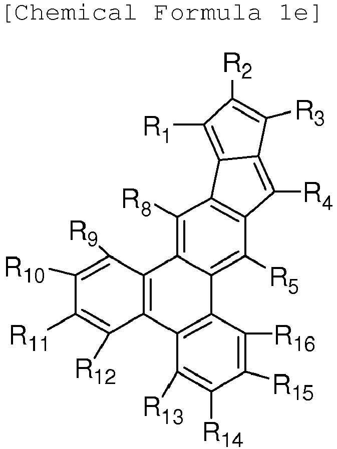 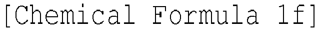 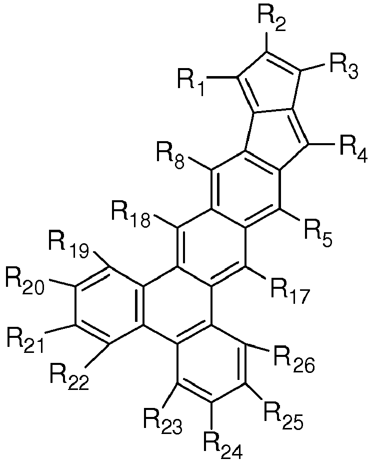 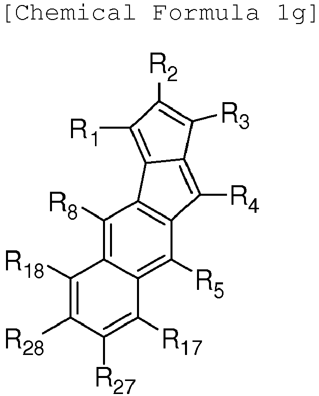 In Chemical Formulae 1a to 1g,
R₁ to R₈ are the same as or different from each other, each independently selected from the group consisting of halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl,
R₉ to R₂₈ are selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or form a substituted or unsubstituted monocyclic or multicyclic aliphatic or aromatic hydrocarbon ring, or a substituted or unsubstituted monocyclic or multicyclic aliphatic or aromatic heteroring, by being linked to an adjacent group.

According to one embodiment of the present invention, in Chemical Formulae 1a to 1g, R₁ to R₈ are the same as or different from each other, and each is substituted or unsubstituted phenyl, substituted or unsubstituted biphenyl, substituted or unsubstituted triphenyl, substituted or unsubstituted naphthyl, substituted or unsubstituted anthryl, substituted or unsubstituted phenanthrenyl, substituted or unsubstituted indenyl, substituted or unsubstituted perylenyl, substituted or unsubstituted pyrenyl, substituted or unsubstituted acenaphthalenyl, substituted or unsubstituted fluorenyl, substituted or unsubstituted fluoranthenyl, substituted or unsubstituted triphenylenyl, substituted or unsubstituted phenalenyl, substituted or unsubstituted pyrrole, substituted or unsubstituted pyridyl, substituted or unsubstituted pyrimidyl, substituted or unsubstituted pyridazinyl, substituted or unsubstituted triazinyl, substituted or unsubstituted thienyl, substituted or unsubstituted furanyl, substituted or unsubstituted benzothiazole, substituted or unsubstituted benzoxazole, substituted or unsubstituted indolyl, substituted or unsubstituted carbazolyl, substituted or unsubstituted benzocarbazolyl, substituted or unsubstituted quinolyl, substituted or unsubstituted isoquinolyl, a substituted or unsubstituted dibenzothiophene group, a substituted or unsubstituted dibenzofuran group, substituted or unsubstituted indolinyl, a substituted or unsubstituted 10,11-dihydro-dibenzo[b,f]azepine group, a substituted or unsubstituted 9,10-dihydroacridine group, a substituted or unsubstituted spiro group in which 2,3-dihydro-1H-indene or cyclohexane is spiro-bonded to fluorene, substituted or unsubstituted dialkylamine, substituted or unsubstituted diarylamine, substituted or unsubstituted alkylarylamine, a substituted or unsubstituted acetophenone group, or a substituted or unsubstituted benzophenone group.

In Chemical Formulae 1a to 1g, R₁ to R₈ may have one or more additional substituents, and at this time, the additional substituents are linear or branched C₁ to C₂₀ alkyl, phenyl, biphenyl, triphenyl, naphthyl, anthryl, phenanthrenyl, indenyl, perylenyl, pyrenyl, acenaphthalenyl, fluorenyl, fluoranthenyl, triphenylenyl, phenalenyl, pyrrole, pyridyl, pyrimidyl, pyridazinyl, triazinyl, thienyl, furanyl, benzothiazole, benzoxazole, indolyl, carbazolyl, benzocarbazolyl, quinolyl, isoquinolyl, a dibenzothiopen group, a dibenzofuran group, indolinyl, a 10,11-dihydro-dibenzo[b,f]azepine group, a 9,10-dihydroacridine group, a spiro group in which 2,3-dihydro-1H-indene or cyclohexane is spiro-bonded to fluorene, dialkylamine, diarylamine, or alkylarylamine. These additional substituents may be unsubstituted or additionally substituted with linear or branched C₁ to C₂₀ alkyl, phenyl, biphenyl, triphenyl, naphthyl, anthryl, phenanthrenyl, indenyl, perylenyl, pyrenyl, acenaphthalenyl, fluorenyl, fluoranthenyl, triphenylenyl, phenalenyl, pyrrole, pyridyl, pyrimidyl, pyridazinyl, triazinyl, thienyl, furanyl, benzothiazole, benzoxazole, indolyl, carbazolyl, benzocarbazolyl, quinolyl, isoquinolyl, a dibenzothiopen group, a dibenzofuran group, indolinyl, a 10,11-dihydro-dibenzo[b,f]azepine group, a 9,10-dihydroacridine group, a spiro group in which 2,3-dihydro-1H-indene or cyclohexane is spiro-bonded to fluorene, dialkylamine, diarylamine, or alkylarylamine.

According to one embodiment of the present invention, in Chemical Formula 1e, R₉ to R₁₆ are hydrogen.

According to one embodiment of the present invention, in Chemical Formula 1f, R₁₇ to R₂₆ are hydrogen.

According to one embodiment of the present invention, in Chemical Formula 1g, R₁₇ and R₁₈ are hydrogen.

According to one embodiment of the present invention, in Chemical Formula 1g, R₂₇ and R₂₈ are selected from the group consisting of hydrogen; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl.

According to one embodiment of the present invention, in Chemical Formula 1g, R₂₇ and R₂₈ are substituted or unsubstituted monocyclic or multicyclic C₆ to C₆₀ aryl; or substituted or unsubstituted monocyclic or multicyclic C₂ to C₆₀ heteroaryl.

Specific examples of the compound of Chemical Formula 1 are represented by the following structural formulae, but the compound is not limited thereto.

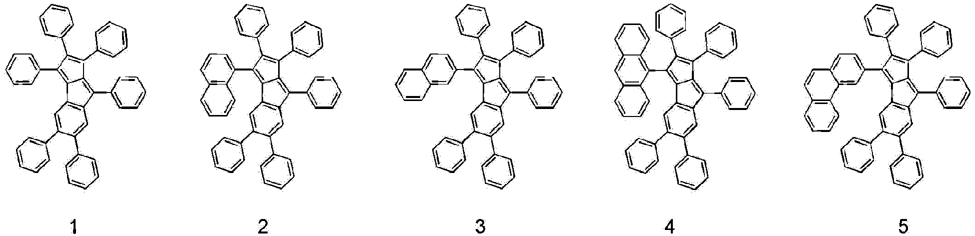

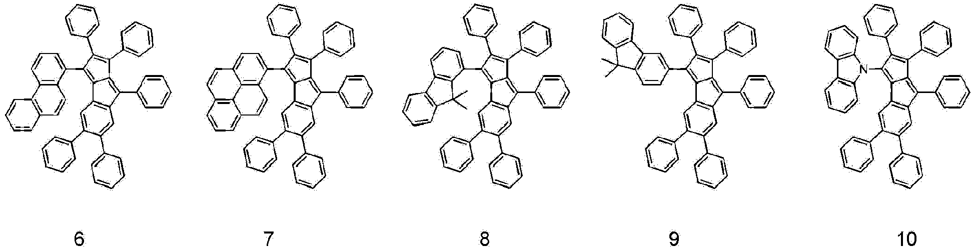

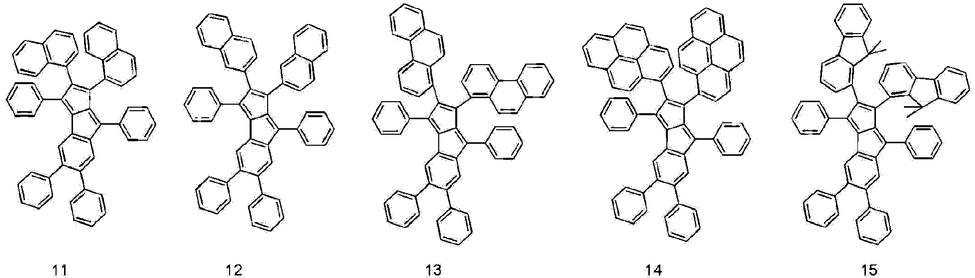

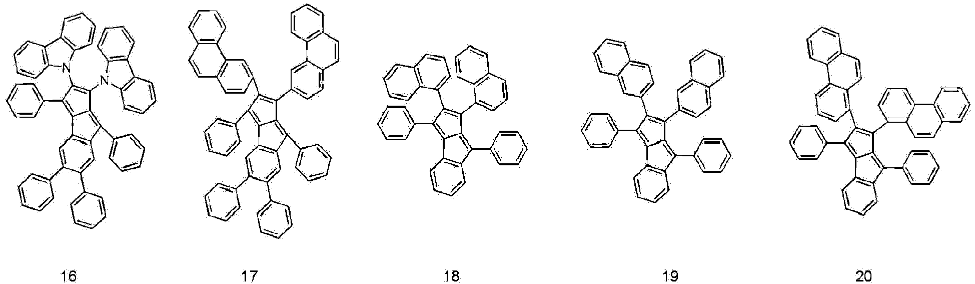

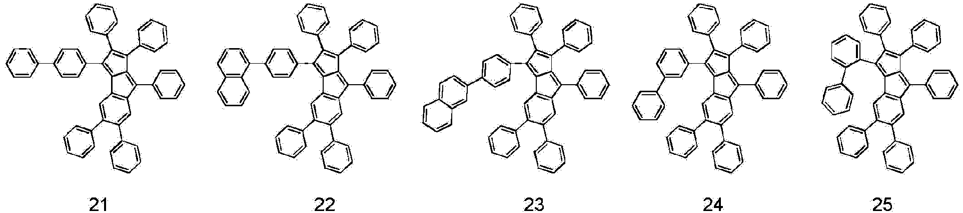

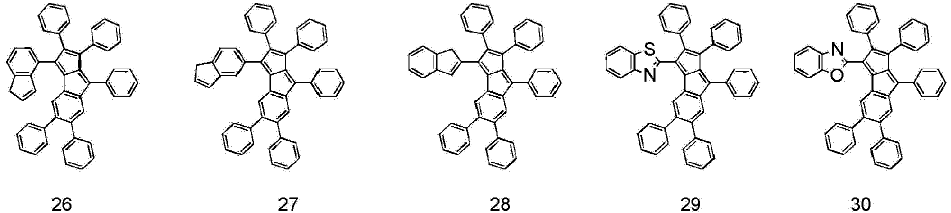

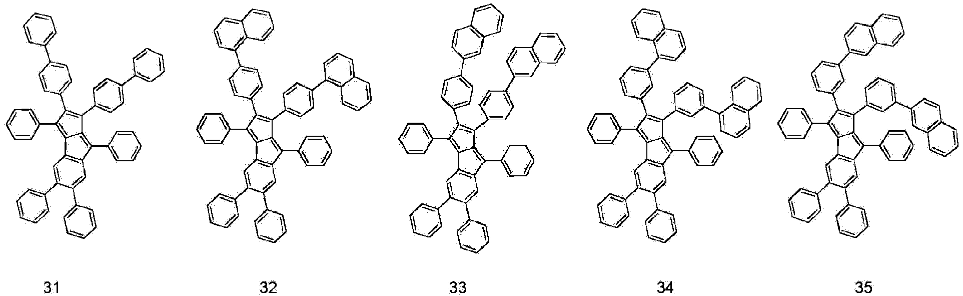

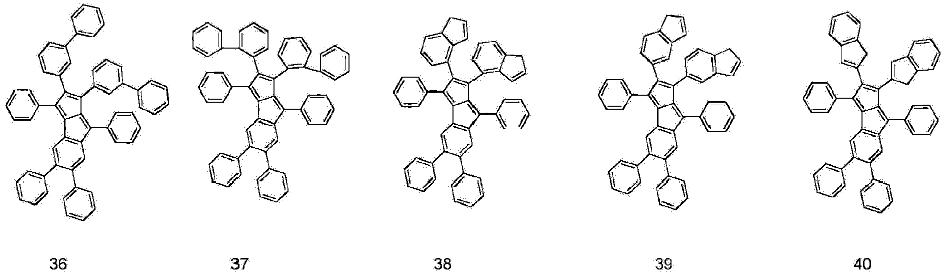

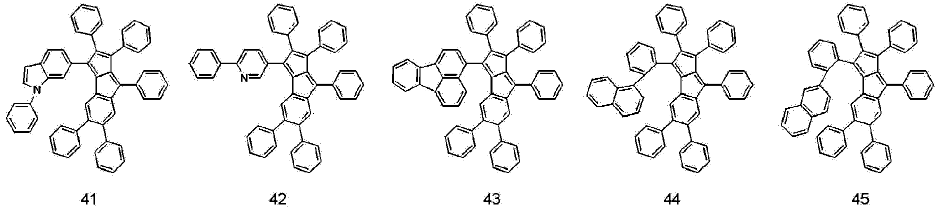

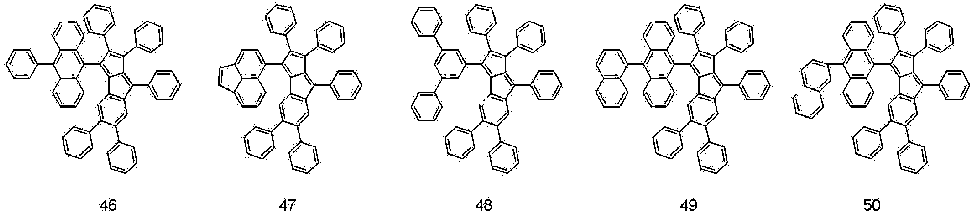

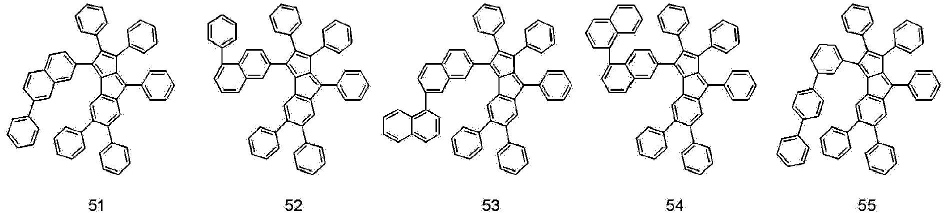

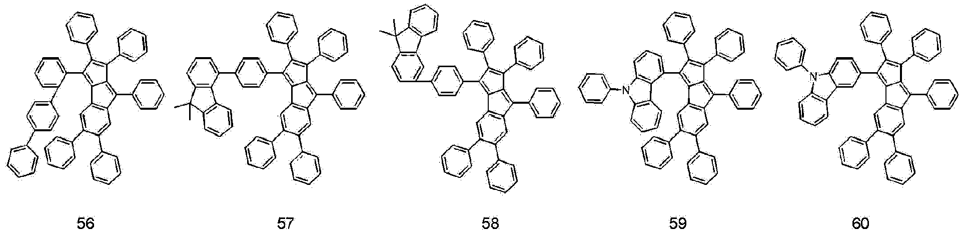

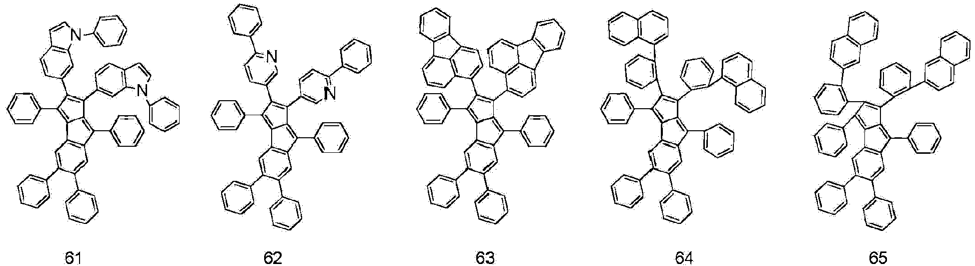

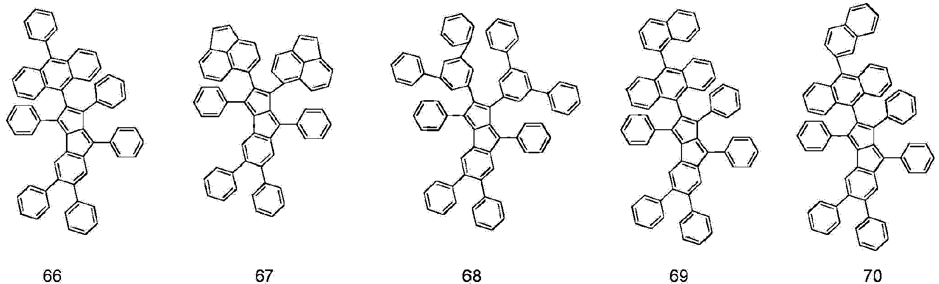

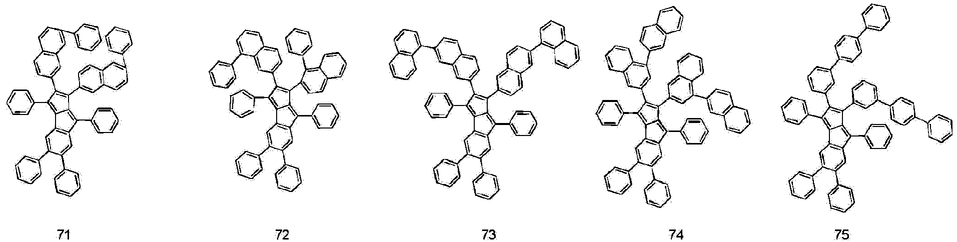

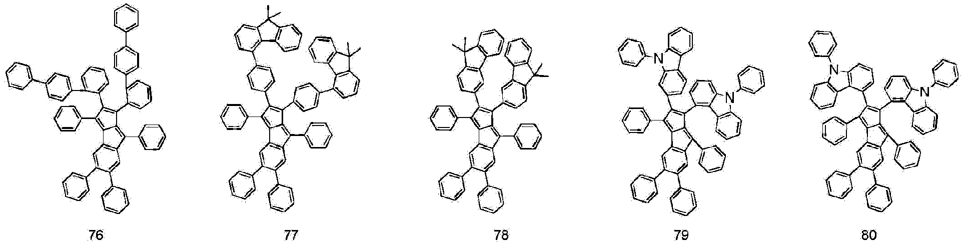

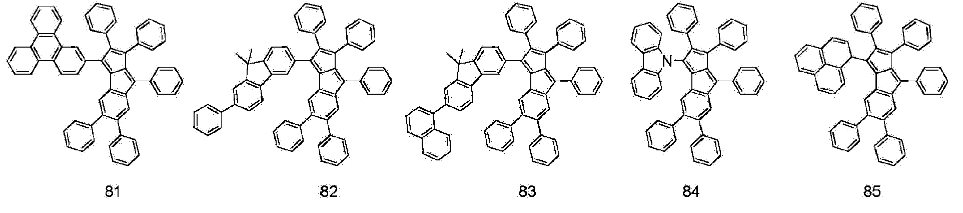

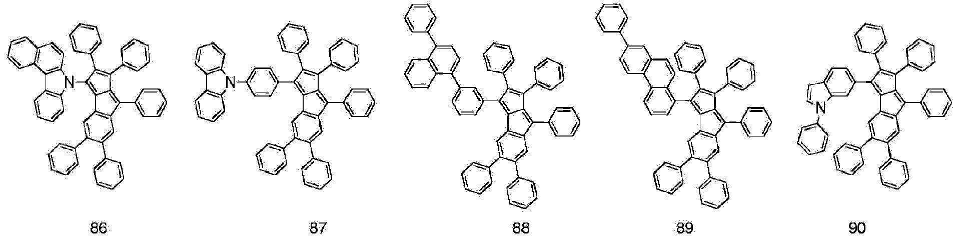

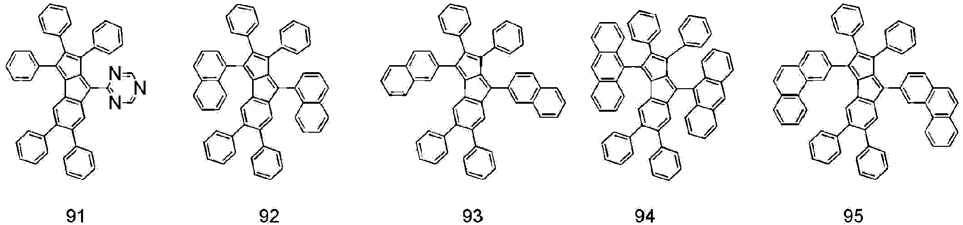

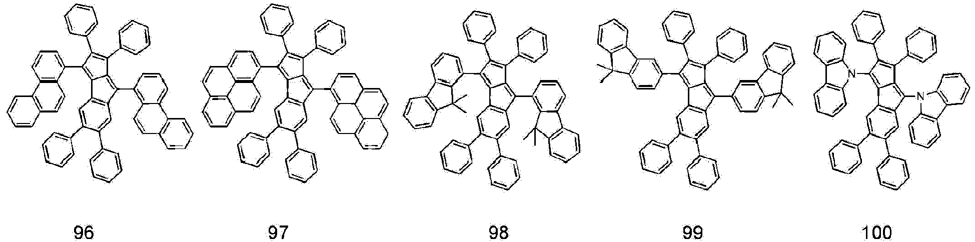

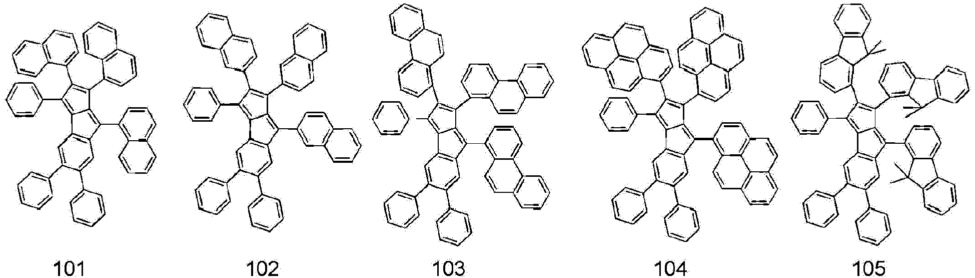

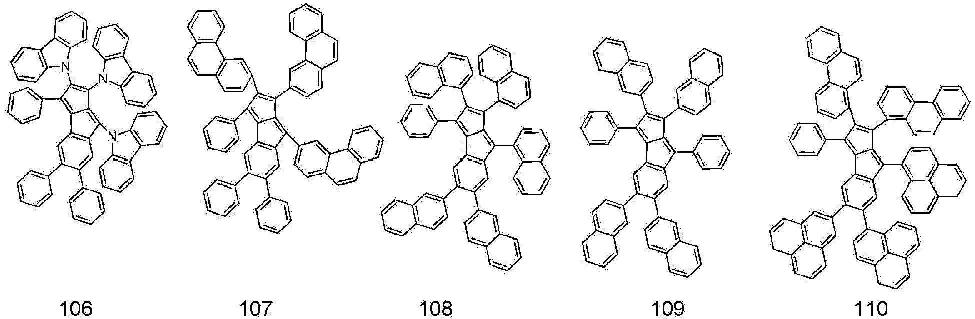

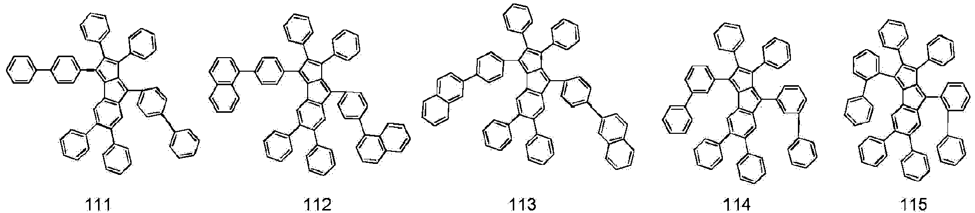

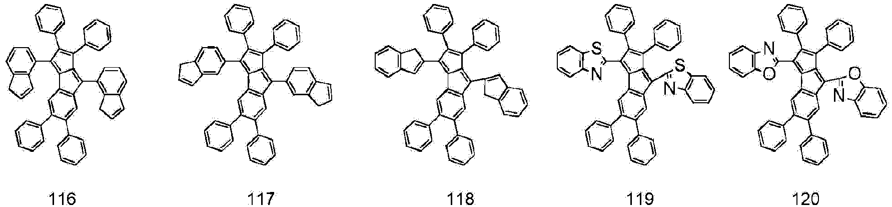

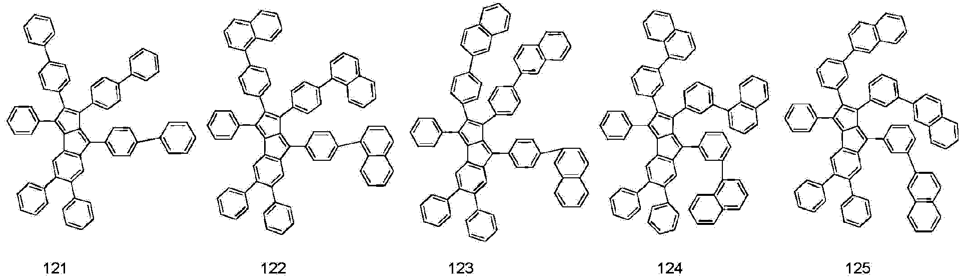

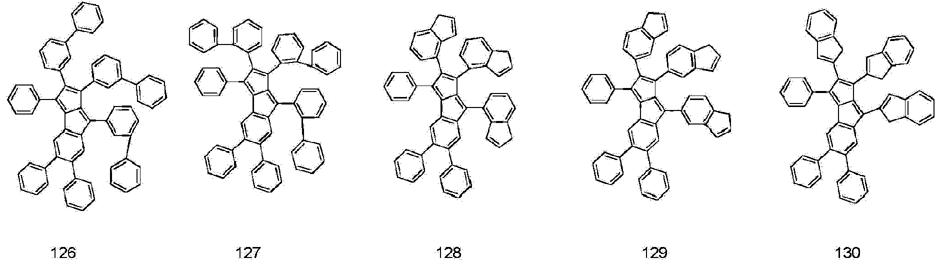

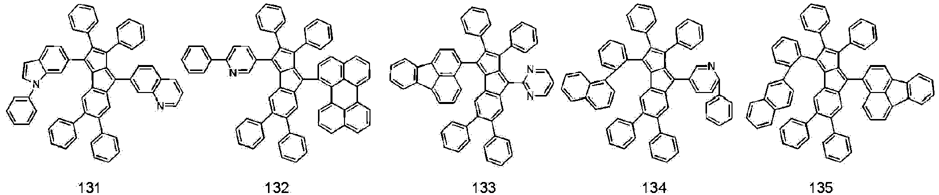

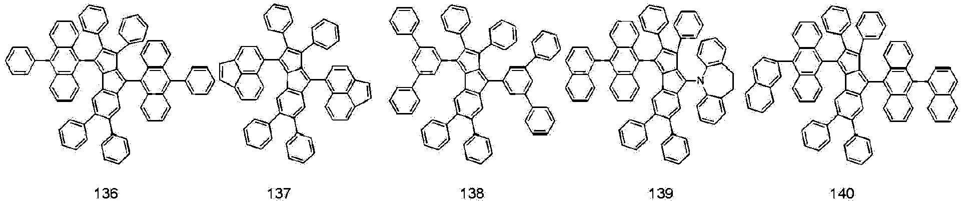

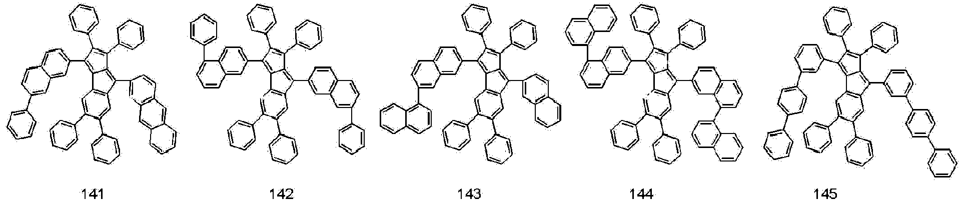

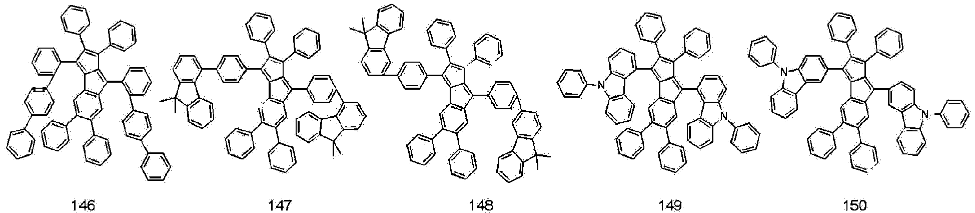

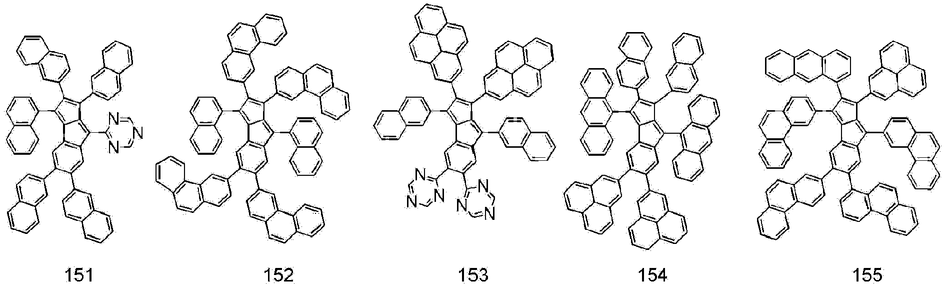

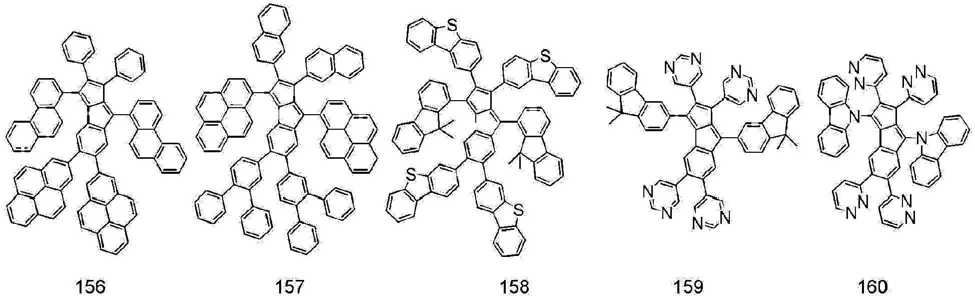

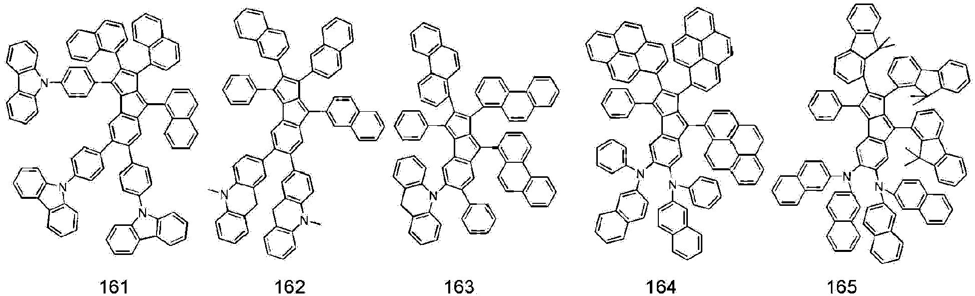

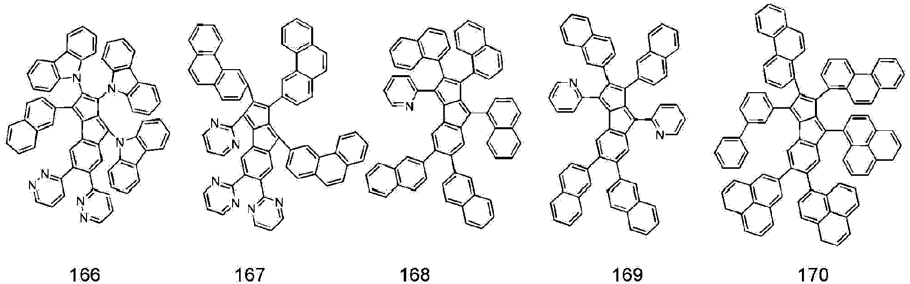

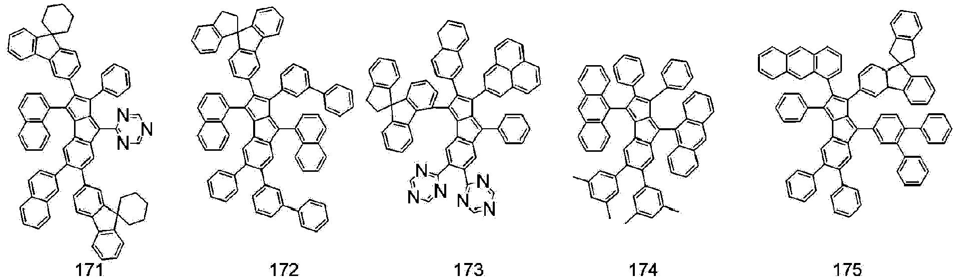

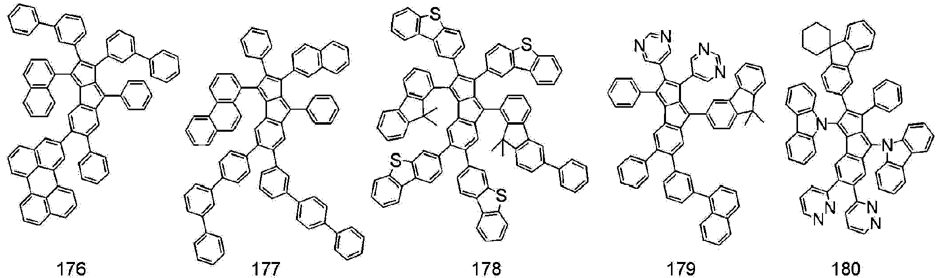

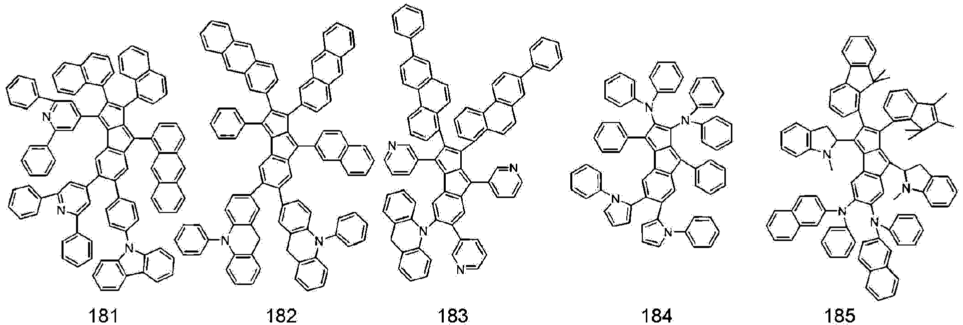

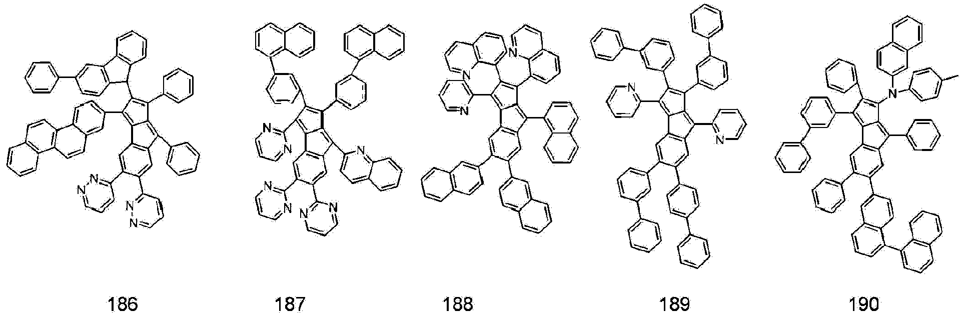

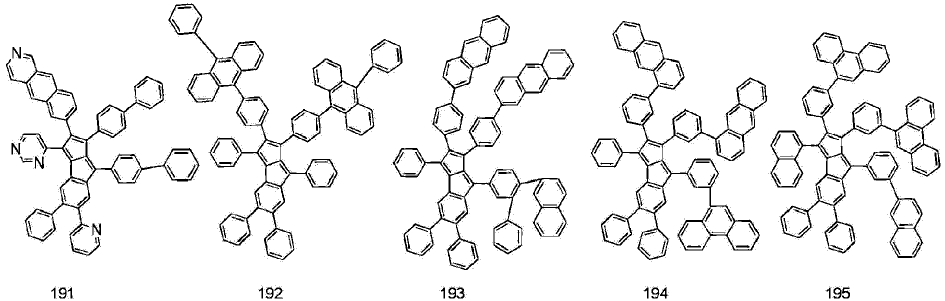

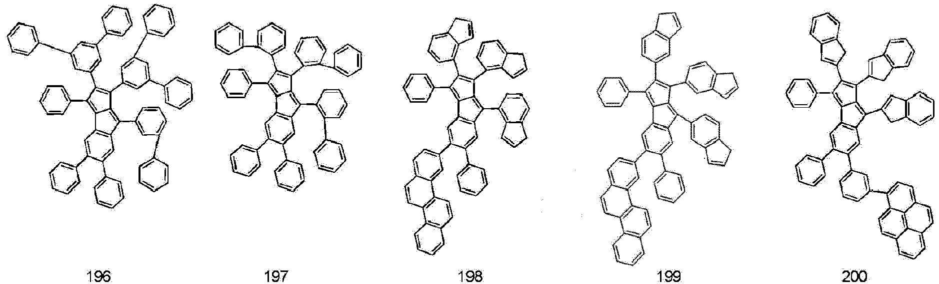

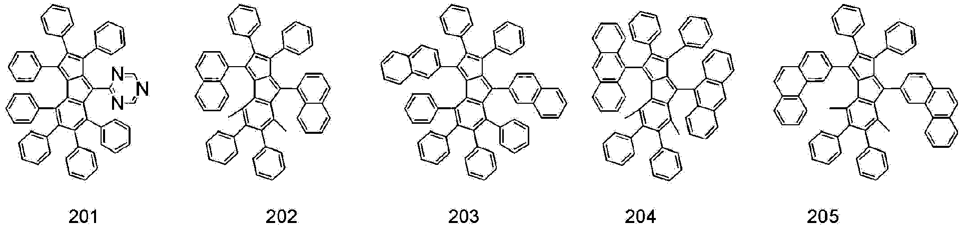

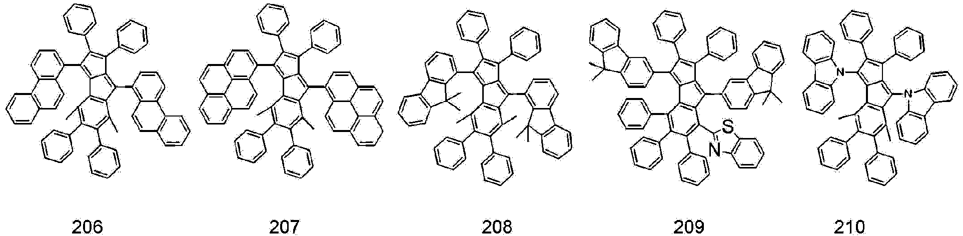

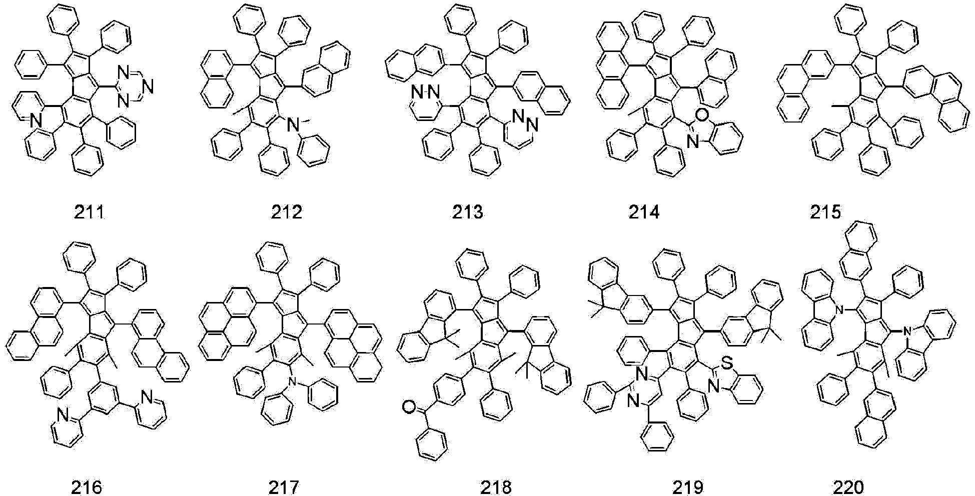

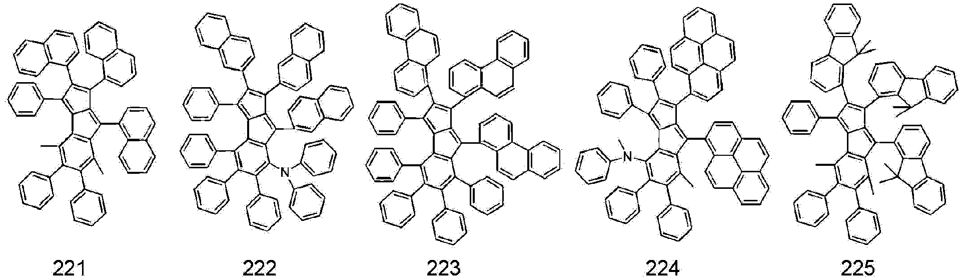

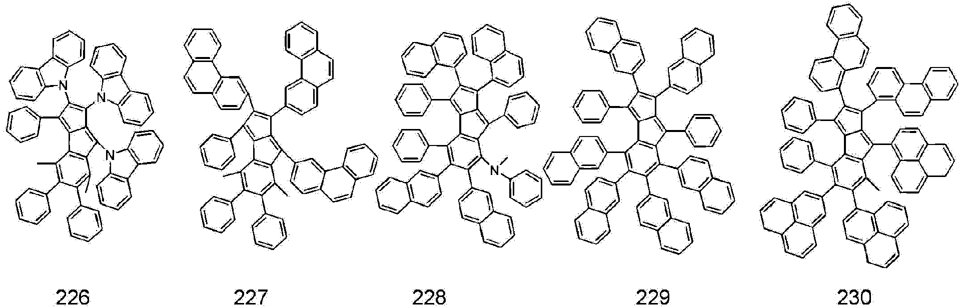

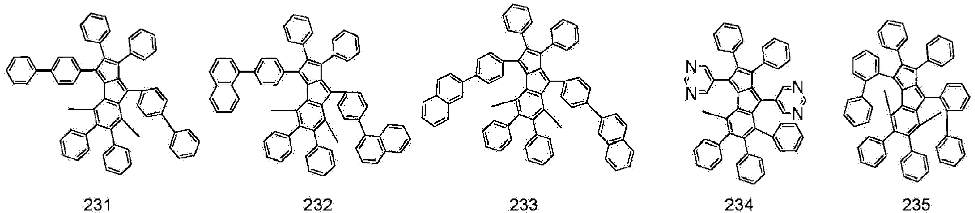

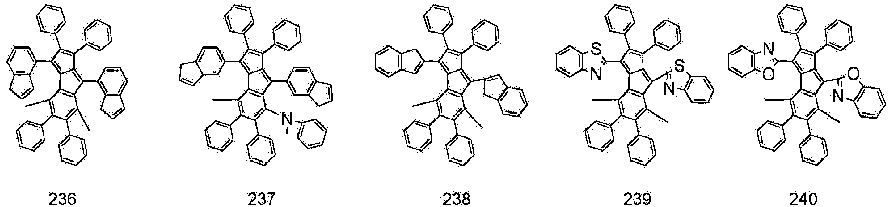

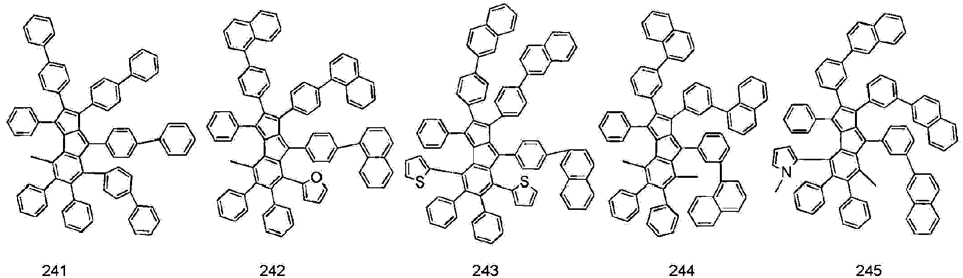

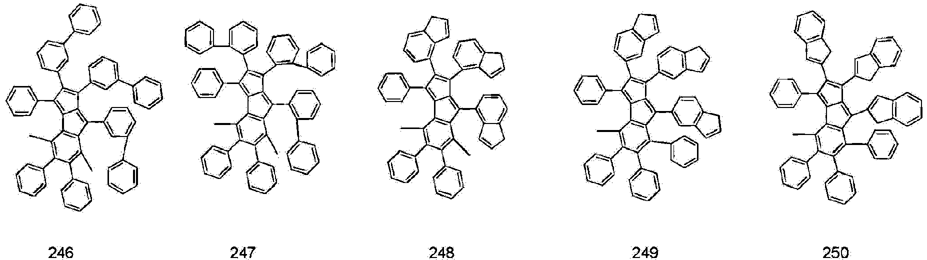

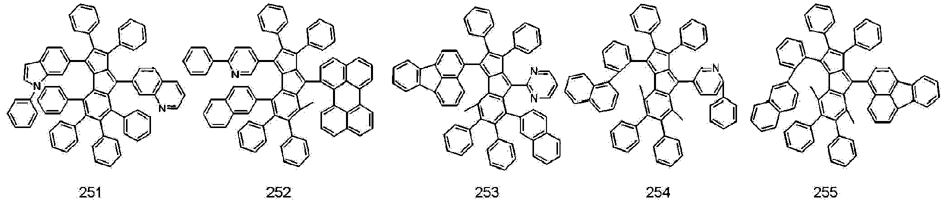

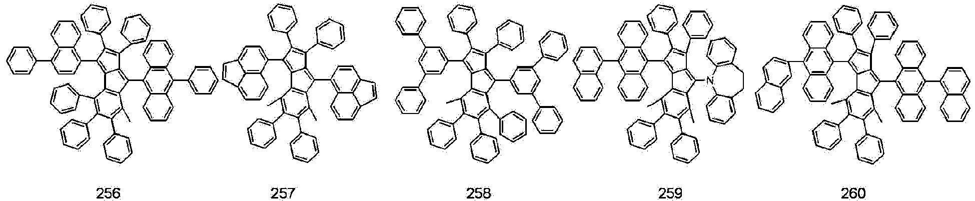

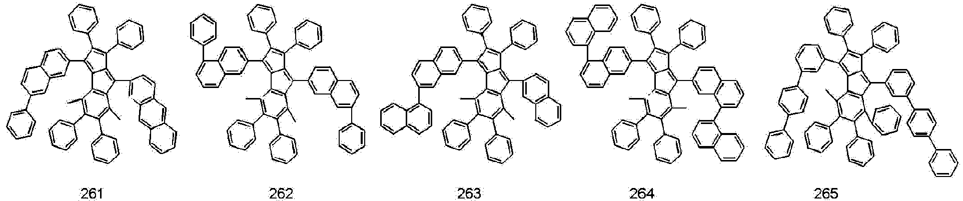

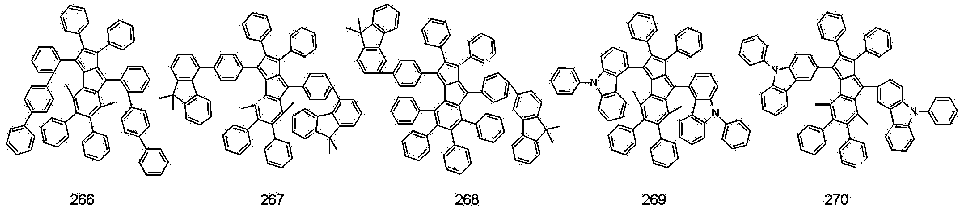

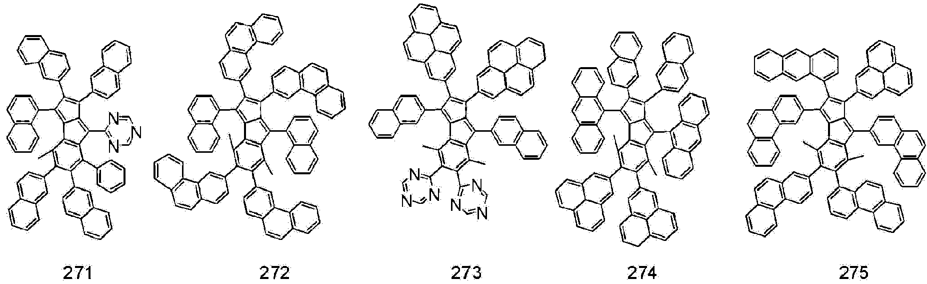

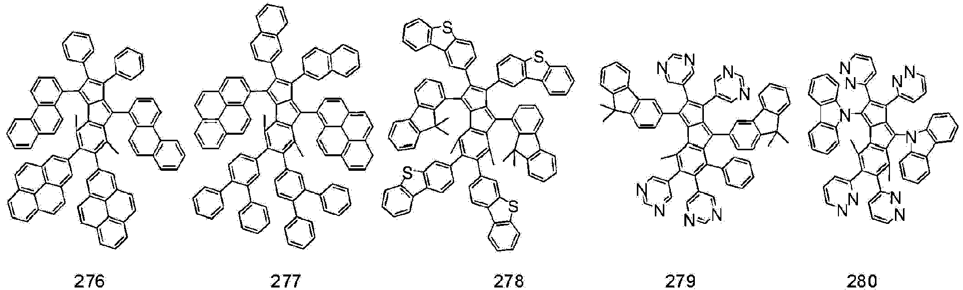

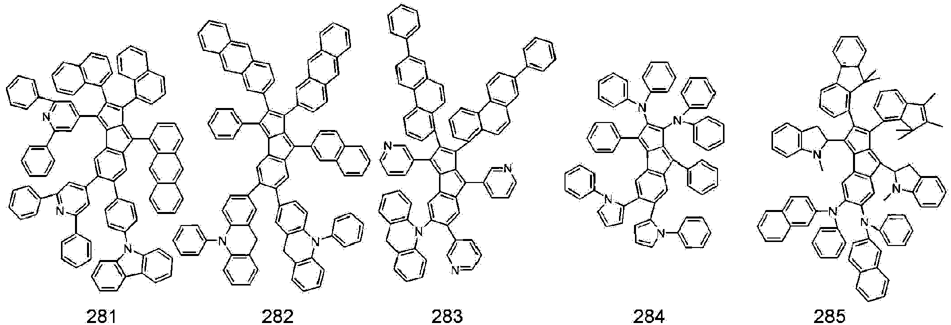

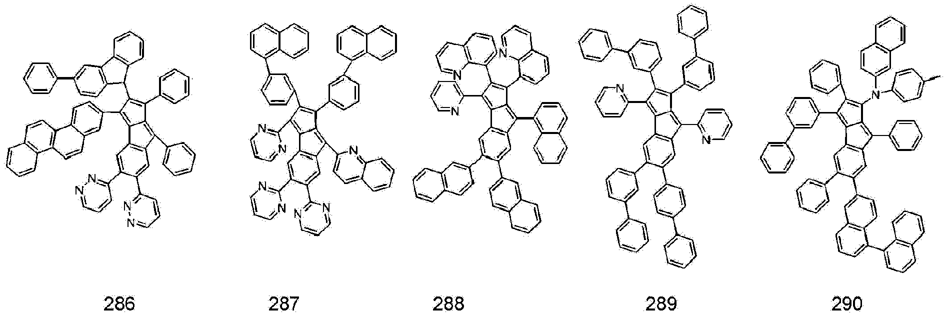

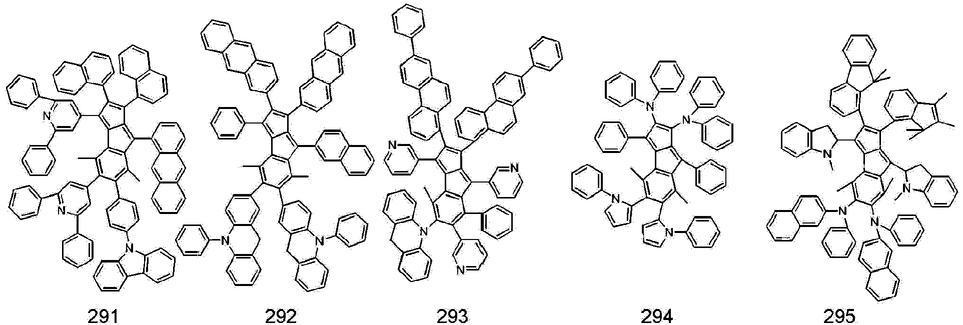

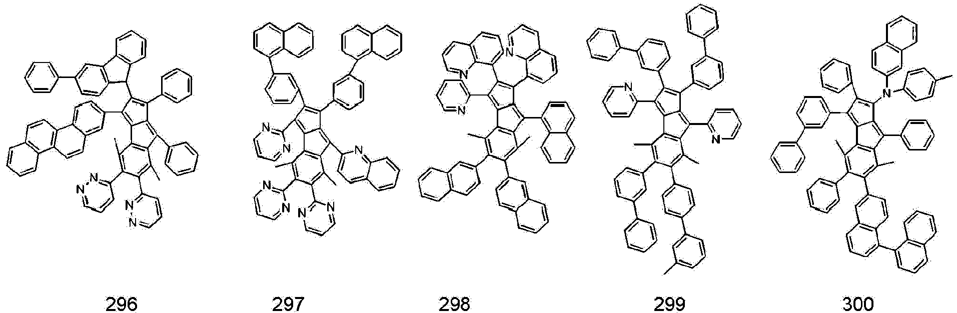

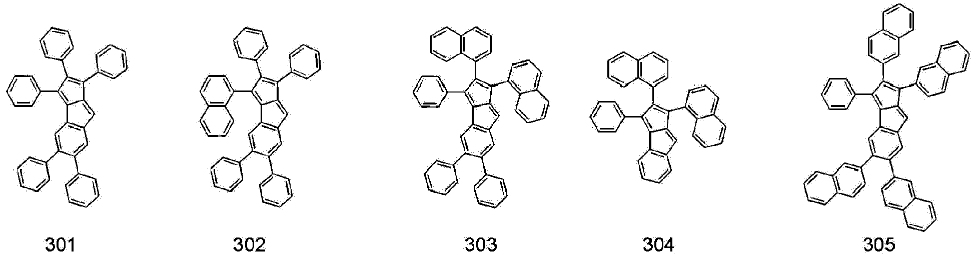

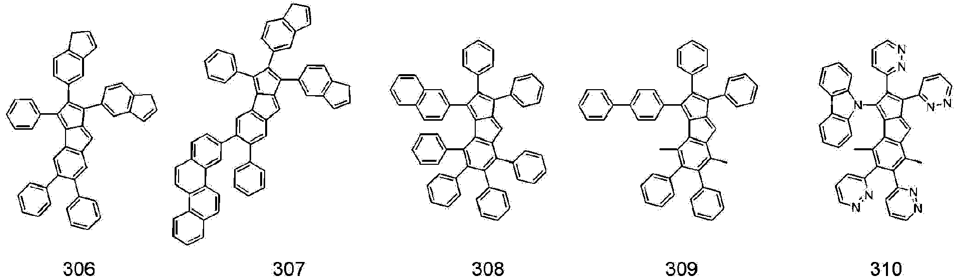

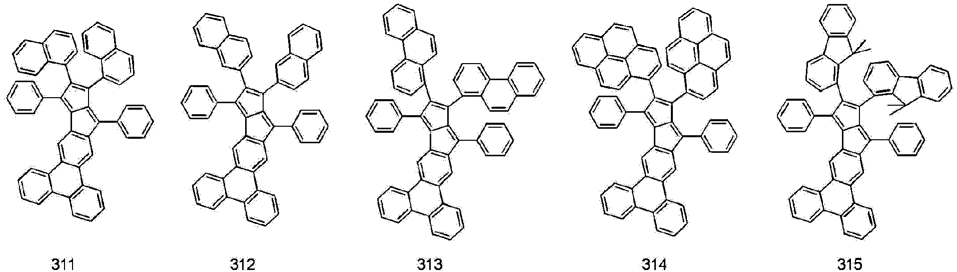

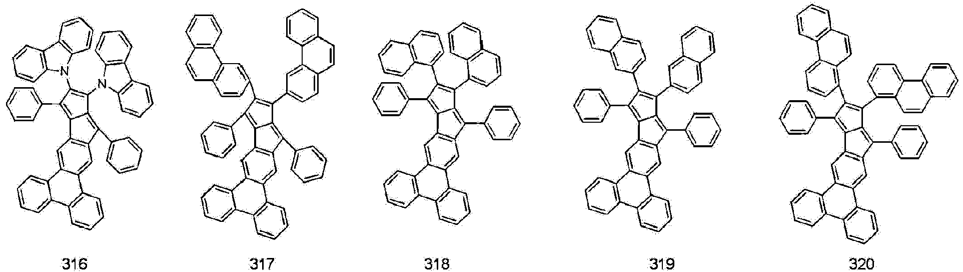

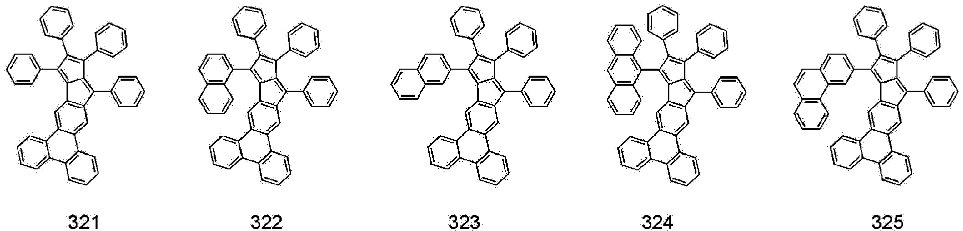

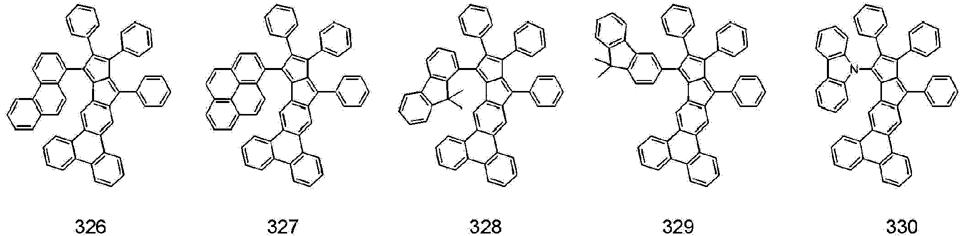

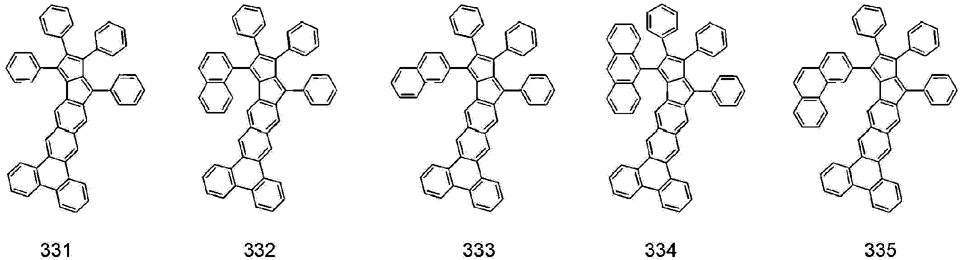

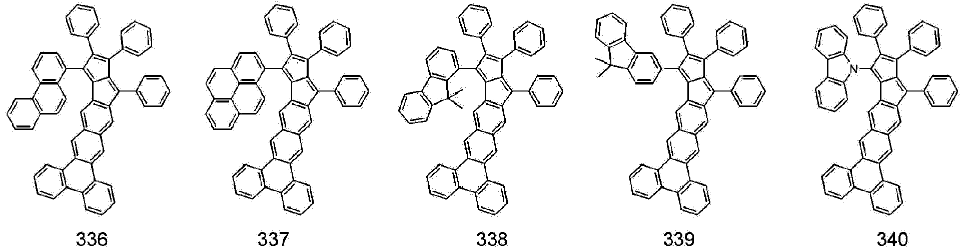

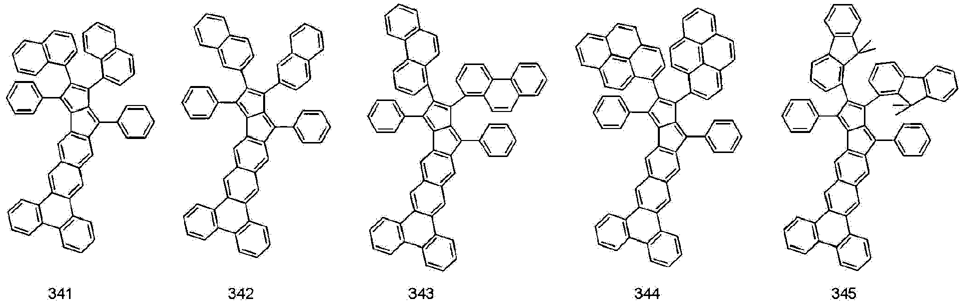

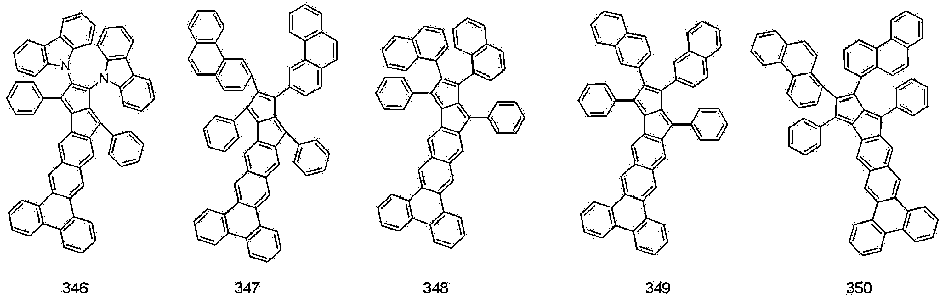

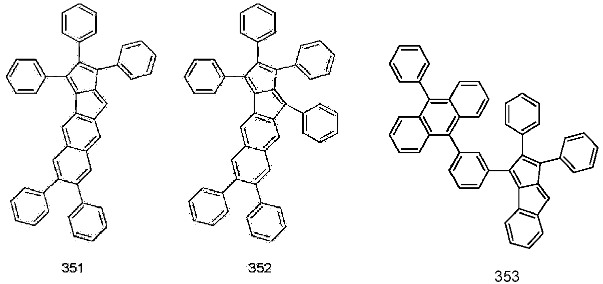

The compounds described above may be prepared based on the preparation examples described later. For example, the compound of Chemical Formula 1 may be prepared using methods such as Reaction Equation 1. As necessary, substituents may be added or excluded. In addition, based on technologies known in the related art, starting materials, reactants, reaction conditions and the like may be changed.

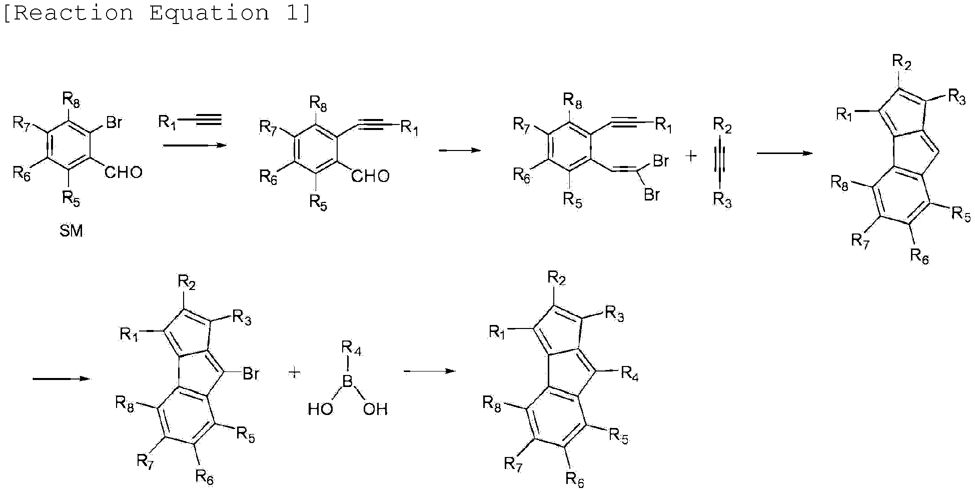

Another embodiment of the present invention provides an organic light emitting device that includes the compound of Chemical Formula 1 described above. Specifically, an organic light emitting device according to the present invention includes an anode, a cathode, and one or more layers of organic material layers provided between the anode and the cathode, and one or more layers of the organic material layers include the compound of Chemical Formula 1.

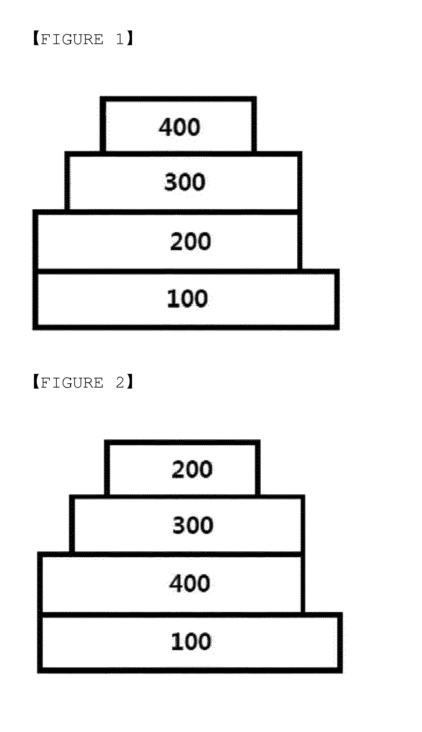

The laminating order of the electrodes and the organic material layers of an organic light emitting device according to embodiments of the present invention is illustrated in Figs. 1 to 3. However, these diagrams are not intended to limit the scope of the present invention, and the structures of organic light emitting devices known in the related art may also be applied to the present invention.

According to Fig. 1, an organic light emitting device in which an anode (200), an organic material layer (300) and a cathode (400) are laminated on a substrate (100) in consecutive order is shown by the diagram. However, the structure of the organic light emitting device is not limited to this structure only, and as shown in Fig. 2, an organic light emitting device in which a cathode, an organic material layer and an anode are laminated on a substrate in consecutive order may also be included.

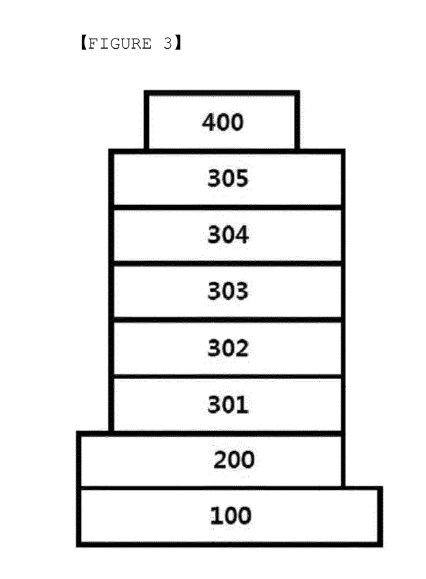

Fig. 3 illustrates the case in which the organic material layer is a multilayer. An organic light emitting device according to Fig. 3 includes a hole injection layer (301), a hole transfer layer (302), a light emitting layer (303), an electron transfer layer (304) and an electron injection layer (305). However, the scope of the present invention is not limited to this laminated structure, and when necessary, other layers except the light emitting layer may not be included, and other necessary layers having other functions may be added.

An organic light emitting device according to the present invention may be prepared using materials and methods known in the related art except that the compound of Chemical Formula 1 is included in one or more layers of the organic material layers.

The compound of Chemical Formula 1 may form one or more layers of the organic material layers alone in an organic light emitting device. However, when necessary, the compound of Chemical Formula 1 may be mixed with other materials to form the organic material layers.

The compound of Chemical Formula 1 may be used as a hole injection material, a hole transfer material, a light emitting material, an electron transfer material, an electron injection material, or the like, in an organic light emitting device. Particularly, the compound of Chemical Formula 1 may be used as the material of a light emitting layer of an organic light emitting device. Specifically, the compound of Chemical Formula 1 may be used as the light emitting material of a light emitting layer. In addition, the compound of Chemical Formula 1 may be used as a host material or a dopant material of the light emitting layer. The compound of Chemical Formula 1 may be used either alone or as a mixture of two or more types. In addition, the compound of Chemical Formula 1 may be used by being mixed with other types of compounds.

As one example, the organic material layer that includes the compound of Chemical Formula 1 is a light emitting layer.

As another example, the organic material layer that includes the compound of Chemical Formula 1 is a light emitting layer, and this light emitting layer further includes a dopant material.

As another example, the organic material layer that includes the compound of Chemical Formula 1 is a fluorescent blue light emitting layer, and this light emitting layer further includes a fluorescent blue dopant material.

The dopant material is not particularly limited, and examples thereof include TBP (2,5,8,11-tetra-tert-butylperylene), DSAPh (p-bis(p-N,N-diphenyl-aminostyryl)-benzene), DPAVBi (4,4-bis[4-(di-p-tolylamino)styryl]biphenyl), BCzVBi (4,4'-(bis(9-ethyl-3-carbazovinylene)-1,1'-biphenyl), DPVBi (1,4-bis(2,2-diphenylvinyl)biphenyl), TBPe (2,5,8,11-tetra-tert-butylperylene), N-BDAVBi (N-(4-((E)-2-(6-((E)-4-(diphenylamino)styryl)naphthalen-2-yl)vinyl)phenyl)-N-phenylbenzenamine), or the like.

In the organic light emitting device according to the present invention, materials other than the compound of Chemical Formula 1 are illustrated below, however, these are for illustrative purposes only, and do not intend to limit the scope of the present invention, and these materials may be substituted with materials known in the related art.

As the anode material, materials having relatively large work function may be used, and transparent conductive oxides, metals, conductive polymers or the like may be used.

As the cathode material, materials having relatively small work function may be used, and metals, metal oxides, conductive polymers or the like may be used.

As the hole injection material, known hole injection materials may be used, and for example, phthalocyanine compounds such as copper phthalocyanine disclosed in US Patent No. 4,356,429, or starbust-type amine derivatives disclosed in a literature [Advanced Material, 6, p.677 (1994)], such as TCTA, m-MTDATA, m-MTDAPB, Pani/DBSA (polyaniline/dodecylbenzenesulfonic acid) or PEDOT/PSS (poly(3,4-ethylenedioxythiophene)/poly(4-styrenesulfonate)), Pani/CSA (polyaniline/camphor sulfonic acid) or PANI/PSS (polyaniline/poly(4-styrene-sulfonate), which is a conductive polymer having solubility, or the like, may be used.

As the hole transfer material, a pyrazoline derivative, an arylamine-based derivative, a stilbene derivative, a triphenyldiamine derivative or the like may be used, and a low molecular or high molecular material may also be used.

As the electron transfer material, an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, 8-hydroxyquinoline and a metal complex of a derivative thereof, or the like, may be used, and a high molecular material as well as a low molecular material may also be used.

As the electron injection material, for example, LiF is typically used in the related industry, however, the present invention is not limited thereto.

As the light emitting material, a red, green or blue light emitting material may be used, and when necessary, two or more light emitting materials may be mixed and used. In addition, as the light emitting material, a fluorescent material may be used, but a phosphorescent material may also be used. As the light emitting material, materials that emit light alone by bonding the holes and the electrons injected from an anode and a cathode, respectively, may be used, however, materials in which a host material and a dopant material are both involved in light emitting may also be used.

Hereinafter, the present invention will be described in more detail with reference to examples, however, it is to be understood that these are for illustrative purposes only, and are not intended to limit the scope of the present invention.

### [Preparation Example 1] Preparation of Compound 1

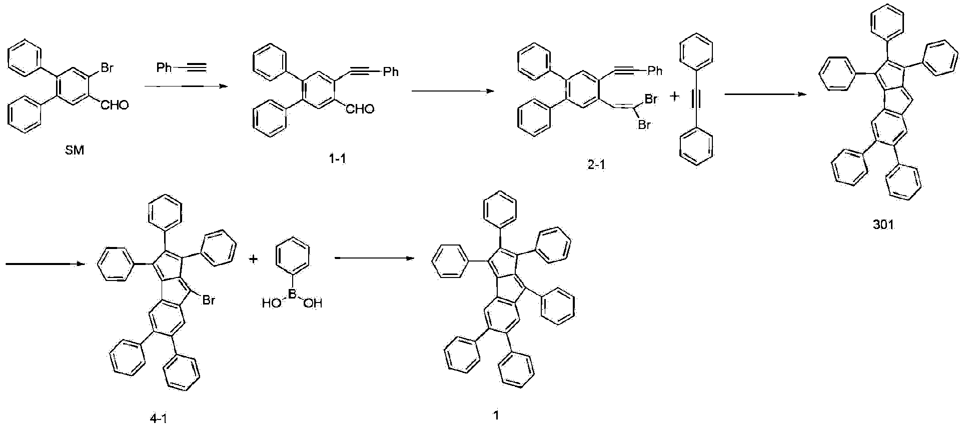

### Preparation of Compound 1-1

After 1 g (2.97 mmol) of Compound SM and 363 mg (3.6 mmol) of ethynylbenzene were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 862 mg (81%) of target Compound 1-1 was obtained.

### Preparation of Compound 2-1

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1 g (2.8 mmol) of Compound 1-1 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.15 g (80%) of target Compound 2-1 was obtained.

### Preparation of Compound 301

After 1 g (1.9 mmol) of Compound 2-1, 6.9 g (38.8 mmol) of 1,2-diphenylethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 620 mg (60%) of target Compound 301 was obtained.

### Preparation of Compound 4-1

After 1 g (1.9 mmol) of Compound 301 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.9 g (95%) of target Compound 4-1 was obtained.

### Preparation of Compound 1

After 1 g (1.6 mmol) of Compound 4-1, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 740 mg (76%) of target Compound 1 was obtained.

### [Preparation Example 2] Preparation of Compound 2

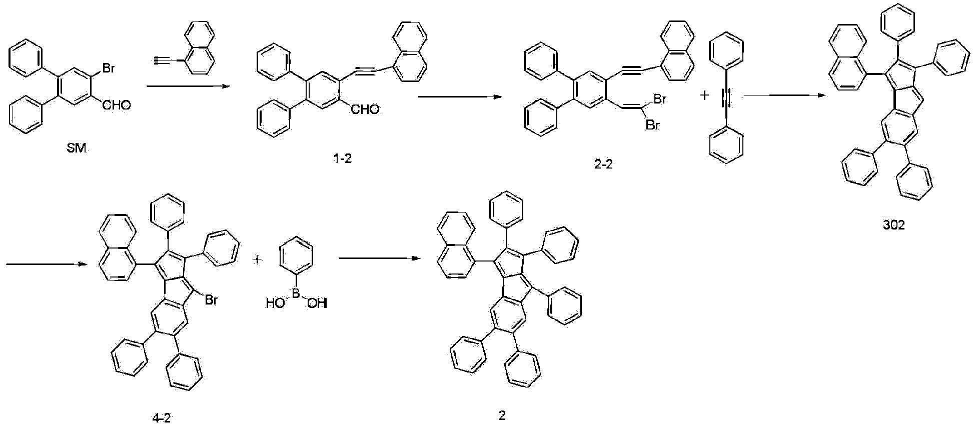

### Preparation of Compound 1-2

After 1 g (2.97 mmol) of Compound SM and 542 mg (3.6 mmol) of ethynylnaphthalene were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 983 mg (81%) of target Compound 1-2 was obtained.

### Preparation of Compound 2-2

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.1 g (2.8 mmol) of Compound 1-2 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.26 g (80%) of target Compound 2-2 was obtained.

### Preparation of Compound 302

After 1 g (1.8 mmol) of Compound 2-2, 6.3 g (35 mmol) of 1,2-diphenylethyne, 70 mg (0.1 mmol) of Pd(PPh₃)₂Cl₂ and 118 mg (1.8 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 629 mg (60%) of target Compound 302 was obtained.

### Preparation of Compound 4-2

After 1.1 g (1.9 mmol) of Compound 302 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.2 g (95%) of target Compound 4-2 was obtained.

### Preparation of Compound 2

After 1.06 g (1.6 mmol) of Compound 4-2, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 801 mg (76%) of target Compound 2 was obtained.

### [Preparation Example 3] Preparation of Compound 11

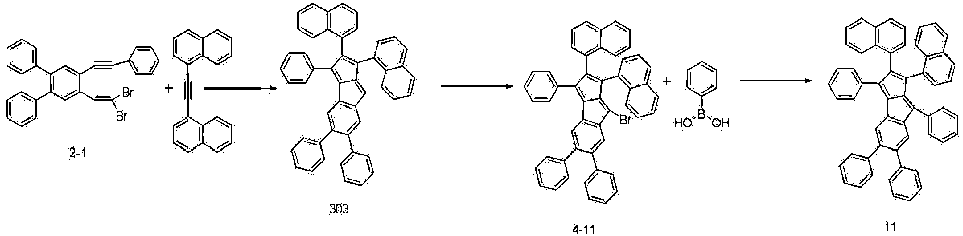

### Preparation of Compound 303

After 1 g (1.9 mmol) of Compound 2-1, 10.8 g (38.8 mmol) of 1,2-di(naphthalen-1-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 721 mg (60%) of target Compound 303 was obtained.

### Preparation of Compound 4-11

After 1.2 g (1.9 mmol) of Compound 303 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.3 g (95%) of target Compound 4-11 was obtained.

### Preparation of Compound 11

After 1.14 g (1.6 mmol) of Compound 4-11, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 862 mg (76%) of target Compound 11 was obtained.

### [Preparation Example 4] Preparation of Compound 18

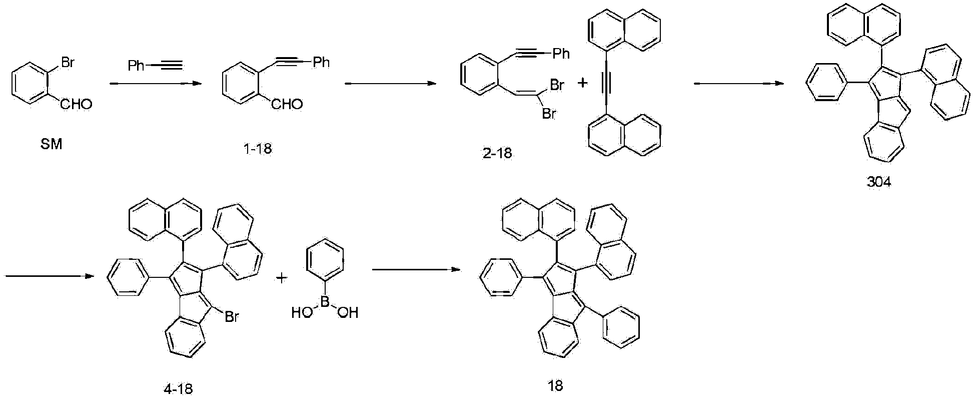

### Preparation of Compound 1-18

After 1 g (5,4 mmol) of Compound SM and 662 mg (6.48 mmol) of ethynylbenzene were dissolved in 22 ml of TEA, 10.3 mg (0.05 mmol) of CuI and 701.9 mg (0.1 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 913 mg (82%) of target Compound 1-18 was obtained.

### Preparation of Compound 2-18

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 577 mg (2.8 mmol) of Compound 1-18 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 811 mg (80%) of target Compound 2-18 was obtained.

### Preparation of Compound 304

After 688 mg (1.9 mmol) of Compound 2-18, 10.8 g (38.8 mmol) of 1,2-di(naphthalen-1-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 548 mg (60%) of target Compound 304 was obtained.

### Preparation of Compound 4-18

After 910 mg (1.9 mmol) of Compound 304 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1 g (94%) of target Compound 4-1 was obtained.

### Preparation of Compound 18

After 895 mg (1.6 mmol) of Compound 4-18, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 677 mg (76%) of target Compound 18 was obtained.

### [Preparation Example 5] Preparation of Compound 109

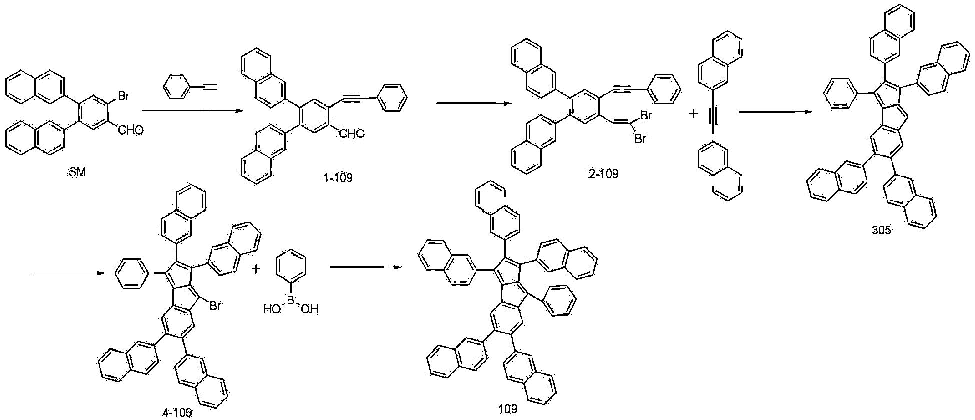

### Preparation of Compound 1-109

After 1.3 g (2.97 mmol) of Compound SM and 363 mg (3.6 mmol) of ethynylbenzene were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 1.13 g (83%) of target Compound 1-109 was obtained.

### Preparation of Compound 2-109

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.3 g (2.8 mmol) of Compound 1-109 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.29 g (75%) of target Compound 2-109 was obtained.

### Preparation of Compound 305

After 1.17 g (1.9 mmol) of Compound 2-109, 10.8 g (38.8 mmol) of 1,2-di(naphthalen-2-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 891 mg (64%) of target Compound 305 was obtained.

### Preparation of Compound 4-109

After 1.39 g (1.9 mmol) of Compound 305 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.4 g (92%) of target Compound 4-109 was obtained.

### Preparation of Compound 109

After 1.3 g (1.6 mmol) of Compound 4-109, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 1 g (73%) of target Compound 109 was obtained.

### [Preparation Example 6] Preparation of Compound 129

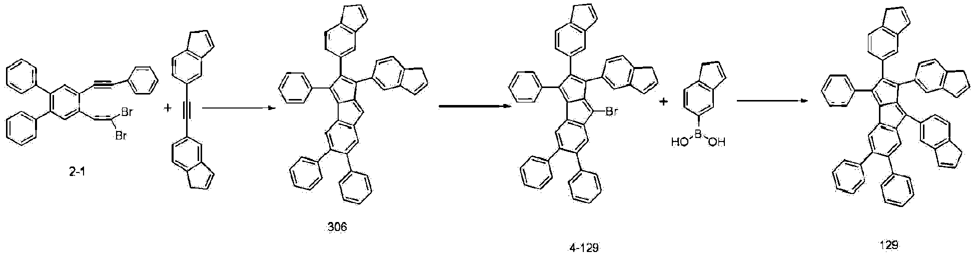

### Preparation of Compound 306

After 1 g (1.9 mmol) of Compound 2-1, 9.8 g (38.8 mmol) of 1,2-di(1H-inden-5-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 752 mg (65%) of target Compound 306 was obtained.

### Preparation of Compound 4-129

After 1.16 g (1.9 mmol) of Compound 306 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.2 g (92%) of target Compound 4-129 was obtained.

### Preparation of Compound 129

After 1.1 g (1.6 mmol) of Compound 4-18, 288 mg (1.8 mmol) of (1H-inden-5-yl)boronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 578 mg (80%) of target Compound 129 was obtained.

### [Preparation Example 7] Preparation of Compound 199

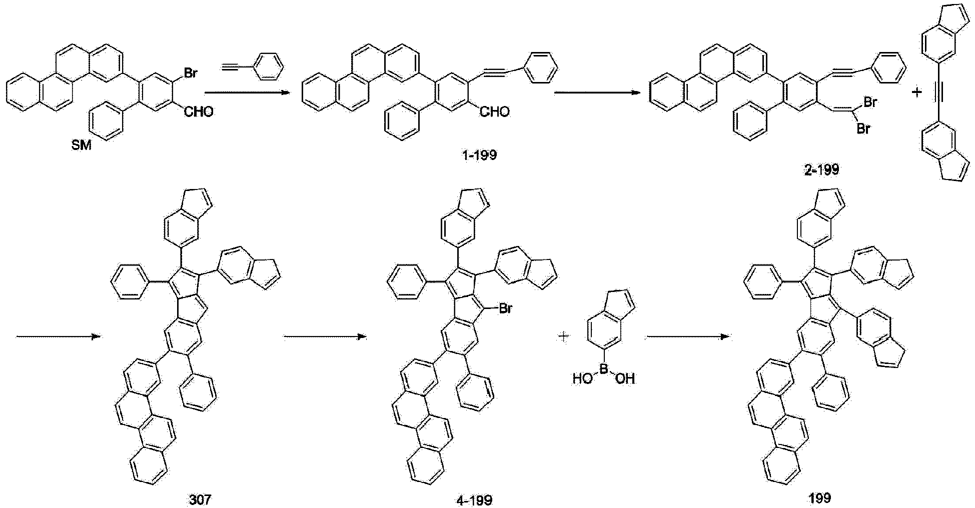

### Preparation of Compound 1-199

After 1.4 g (2.97 mmol) of Compound SM and 363 mg (3.6 mmol) of ethynylbenzene were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 1.3 g (86%) of target Compound 1-199 was obtained.

### Preparation of Compound 2-199

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.4 g (2.8 mmol) of Compound 1-199 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.3 g (70%) of target Compound 2-199 was obtained.

### Preparation of Compound 307

After 1.26 g (1.9 mmol) of Compound 2-199, 9.8 g (38.8 mmol) of 1,2-di(1H-inden-5-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 951 mg (66%) of target Compound 307 was obtained.

### Preparation of Compound 4-199

After 1.4 g (1.9 mmol) of Compound 307 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.4 g (90%) of target Compound 4-199 was obtained.

### Preparation of Compound 199

After 1.3 g (1.6 mmol) of Compound 4-199, 287 mg (1.8 mmol) of 1H-inden-5-ylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 1 g (73%) of target Compound 199 was obtained.

### [Preparation Example 8] Preparation of Compound 203

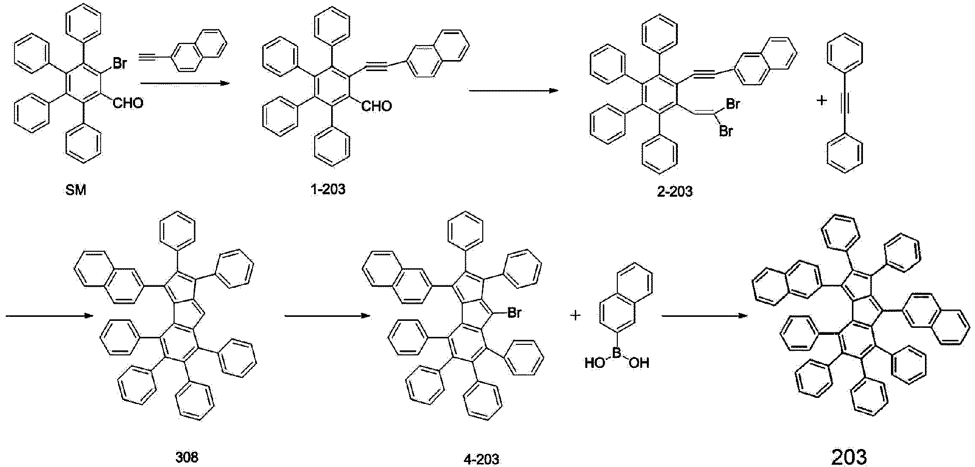

### Preparation of Compound 1-203

After 1.48 g (2.97 mmol) of Compound SM and 542 mg (3.6 mmol) of 1-ethynylnaphthalene were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 1.3 g (82%) of target Compound 1-203 was obtained.

### Preparation of Compound 2-203

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.57 g (2.8 mmol) of Compound 1-203 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.38 g (69%) of target Compound 2-203 was obtained.

### Preparation of Compound 308

After 2 g (1.9 mmol) of Compound 2-203, 9.8 g (38.8 mmol) of 1,2-di(1H-inden-5-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 940 mg (69%) of target Compound 308 was obtained.

### Preparation of Compound 4-203

After 1.4 g (1.9 mmol) of Compound 308 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.4 g (90%) of target Compound 4-203 was obtained.

### Preparation of Compound 203

After 1.3 g (1.6 mmol) of Compound 4-203, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 973 mg (75%) of target Compound 203 was obtained.

### [Preparation Example 9] Preparation of Compound 231

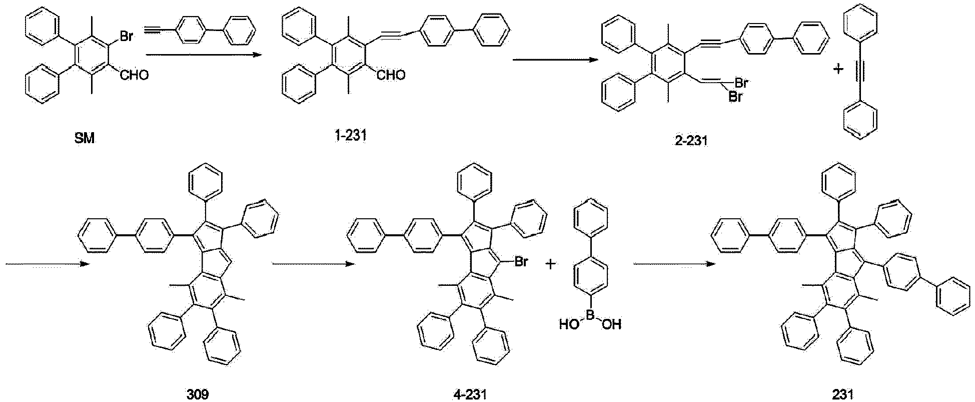

### Preparation of Compound 1-231

After 1.08 g (2.97 mmol) of Compound SM and 641 mg (3.6 mmol) of 4-ethynylbiphenyl were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 1.1 g (81%) of target Compound 1-231 was obtained.

### Preparation of Compound 2-231

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.3 g (2.8 mmol) of Compound 1-231 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.2 g (70%) of target Compound 2-231 was obtained.

### Preparation of Compound 309

After 1.2 g (1.9 mmol) of Compound 2-231, 9.8 g (38.8 mmol) of 1,2-di(1H-inden-5-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 834 mg (69%) of target Compound 309 was obtained.

### Preparation of Compound 4-231

After 1.2 g (1.9 mmol) of Compound 309 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.2 g (90%) of target Compound 4-231 was obtained.

### Preparation of Compound 231

After 1.1 g (1.6 mmol) of Compound 4-231, 356 mg (1.8 mmol) of biphenyl-4-ylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 946 mg (75%) of target Compound 231 was obtained.

### [Preparation Example 10] Preparation of Compound 280

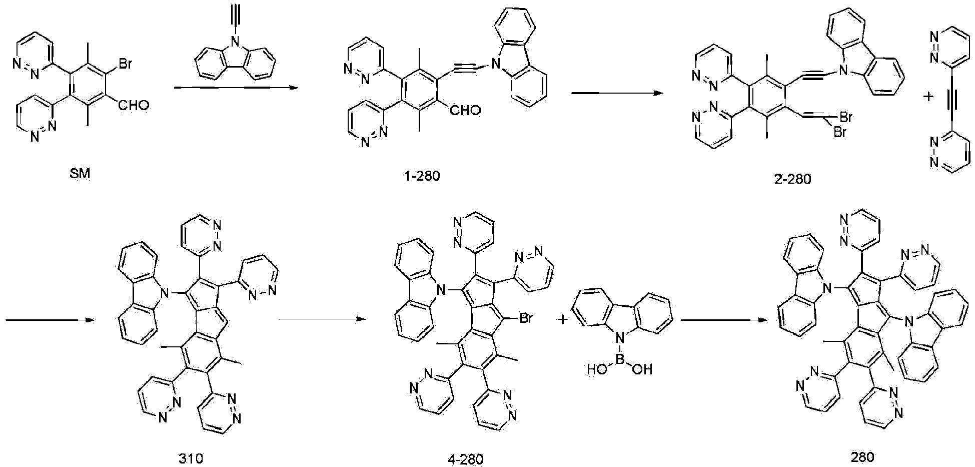

### Preparation of Compound 1-280

After 1.1 g (2.97 mmol) of Compound SM and 688 mg (3.6 mmol) of 9-ethynyl-9H-carbazole were dissolved in 22 ml of TEA, 28 mg (0.15 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 1.1 g (82%) of target Compound 1-280 was obtained.

### Preparation of Compound 2-280

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.33 g (2.8 mmol) of Compound 1-280 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.3 g (74%) of target Compound 2-280 was obtained.

### Preparation of Compound 310

After 1.2 g (1.9 mmol) of Compound 2-280, 7 g (38.8 mmol) of 1,2-di(pyridazin-3-yl)ethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 887 mg (71%) of target Compound 310 was obtained.

### Preparation of Compound 4-280

After 1.2 g (1.9 mmol) of Compound 310 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.2 g (90%) of target Compound 4-280 was obtained.

### Preparation of Compound 280

After 1.18 g (1.6 mmol) of Compound 4-280, 380 mg (1.8 mmol) of 9H-carbazol-9-ylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 1 g (78%) of target Compound 280 was obtained.

### [Preparation Example 11] Preparation of Compound 321

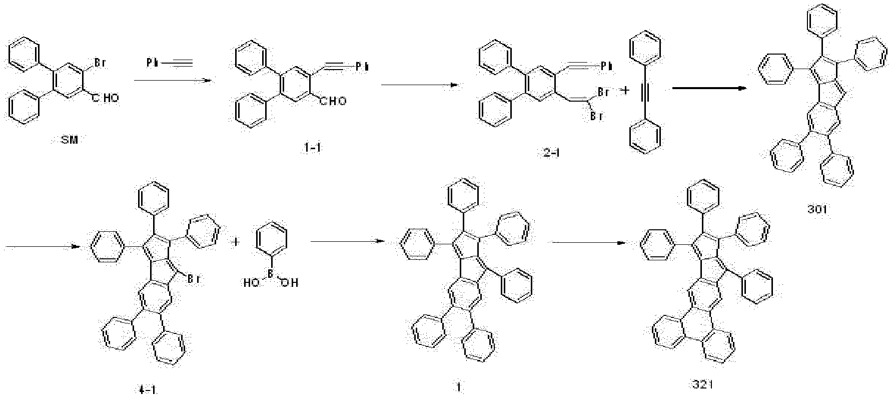

### Preparation of Compound 321

3 g (4.9 mmol) of Compound 1 was dissolved in 60 ml of MC. After 15.9 g (98 mmol) of anhydrous ferric chloride was dissolved in 675 ml of nitromethane, it was quickly added to Compound 1, and the mixture was shaken for 15 seconds. After the result was immersed in a sonicator for 30 minutes, it was stirred for 2 days at room temperature. After the reaction completed, the result was washed with a 0.1M HCl solution, and then with a 0.1M NH₄OH solution. After the result was extracted with ethyl acetate and water, the organic layer was dried using anhydrous sodium sulfate and filtered. After the solvent was removed by vacuum distillation again, the result was separated and purified using column chromatography, and 2.26 g (76%) of target Compound 321 was obtained.

### [Preparation Example 12] Preparation of Compound 331

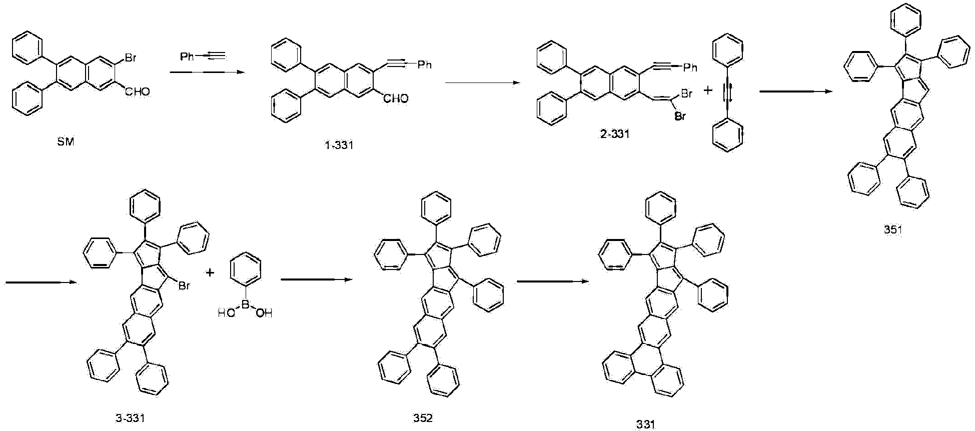

### Preparation of Compound 1-331

After 1 g (2.6 mmol) of Compound SM and 315 mg (3.1 mmol) of ethynylbenzene were dissolved in 22 ml of TEA, 24 mg (0.13 mmol) of CuI and 211 mg (0.3 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 956 mg (90%) of target Compound 1-331 was obtained.

### Preparation of Compound 2-331

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.14 g (2.8 mmol) of Compound 1-331 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.2 g (79%) of target Compound 2-331 was obtained.

### Preparation of Compound 351

After 1 g (1.9 mmol) of Compound 2-331, 6.9 g (38.8 mmol) of 1,2-diphenylethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 686 mg (62%) of target Compound 351 was obtained.

### Preparation of Compound 3-331

After 1 g (1.9 mmol) of Compound 351 and 368 mg (2 mmol) of NBS were dissolved in DMF, the mixture was stirred for 6 hours at room temperature. The result was extracted with ethyl acetate and water. The organic layer was dried using anhydrous sodium sulfate and filtered, and after the solvent was removed by vacuum distillation again, the result was separated and purified using column chromatography, and 1.15 g (92%) of target Compound 3-331 was obtained.

### Preparation of Compound 352

After 1.2 g (1.6 mmol) of Compound 3-331, 219 mg (1.8 mmol) of phenylboronic acid and 663 mg (4.8 mmol) of K₂CO₃ were dissolved in 15 ml/3 ml/3 ml of toluene/H₂O/EtOH, 92 mg (0.08 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 811 mg (77%) of target Compound 352 was obtained.

### Preparation of Compound 331

3.2 g (4.9 mmol) of Compound 352 was dissolved in 60 ml of MC. After 15.9 g (98 mmol) of anhydrous ferric chloride was dissolved in 675 ml of nitromethane, it was quickly added to Compound 352, and the mixture was shaken for 15 seconds. After the result was immersed in a sonicator for 30 minutes, it was stirred for 2 days at room temperature. After the reaction completed, the result was washed with a 0.1M HCl solution, and then with a 0.1M NH₄OH solution. After the result was extracted with ethyl acetate and water, the organic layer was dried using anhydrous sodium sulfate and filtered. After the solvent was removed by vacuum distillation again, the result was separated and purified using column chromatography, and 2.47 g (77%) of target Compound 331 was obtained.

### [Preparation Example 13] Preparation of Compound 353

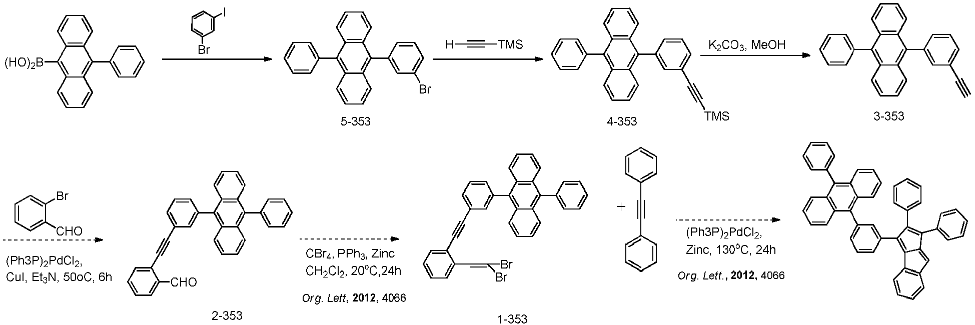

### Preparation of Compound 5-353

After 1.2 g (4.1 mmol) of 1-bromo-3-iodobenzene, 1 g (3.4 mmol) of (10-phenylanthracen-9-yl)boronic acid and 939 mg (6.8 mmol) of K₂CO₃ were dissolved in 20 ml/4 ml/4 ml of toluene/H₂O/EtOH, 195 mg (0.17 mmol) of Pd(PPh₃)₄ was added dropwise thereto, and the mixture was vacuum distilled for one day. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.1 g (80%) of target Compound 5-353 was obtained.

### Preparation of Compound 4-353

After 1 g (2.6 mmol) of Compound 5-353 and 281 mg (2.9 mmol) of ethynyltrimethylsilane were dissolved in 22 ml of TEA, 24 mg (0.13 mmol) of CuI and 42 mg (0.06 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 776 mg (70%) of target Compound 4-353 was obtained.

### Preparation of Compound 3-353

After 1 g (2.3 mmol) of Compound 4-353 and 28 mg (0.2 mmol) of K₂CO₃ were dissolved in 20 ml of MeOH, the mixture was stirred for 4 hours at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 587 mg (72%) of target Compound 3-353 was obtained.

### Preparation of Compound 2-353

After 1 g (2.9 mmol) of Compound 3-353 and 592 mg (3.2 mmol) of 2-bromobenzaldehyde were dissolved in 22 ml of TEA, 28 mg (0.15 mmol) of CuI and 42 mg (0.06 mmol) of (Ph₃P)₂PdCl₂ were added dropwise thereto. The mixture was stirred for 3 hours at 50°C. After the reaction completed, the result was extracted with ethyl acetate (EA) and water, and the EA layer was washed with 1N-HCl. The organic layer was dried using anhydrous sodium sulfate and filtered, and after the solvent was removed by vacuum distillation again, the result was separated and purified using column chromatography, and 930 mg (70%) of target Compound 2-353 was obtained.

### Preparation of Compound 1-353

After 5.9 g (22 mmol) of PPh₃ and 1.5 g (22 mmol) of zinc powder were dissolved in 22 ml of dichloromethane at 0°C, 7.4 g (22 mmol) of CBr₄ compound was added thereto in small fractions for 30 minutes while stirring. The mixture was further stirred for 1 hour at room temperature. The temperature was lowered to 0°C, and then 1.28 g (2.8 mmol) of Compound 2-353 was added thereto in small fractions for 30 minutes. After that, the result was stirred for one day at room temperature. After the reaction completed, the result was extracted with ethyl acetate and water. The organic layer was dried using anhydrous sodium sulfate and filtered, and after the solvent was removed by vacuum distillation again, the result was separated and purified using column chromatography, and 1.3 g (75%) of target Compound 1-353 was obtained.

### Preparation of Compound 353

After 1.2 g (1.9 mmol) of Compound 1-353, 6.9 g (38.8 mmol) of 1,2-diphenylethyne, 70 mg (0.1 mmol) of Pd(PPh₃)₂Cl₂ and 127 mg (1.9 mmol) of zinc powder were dissolved in toluene, the mixture was vacuum distilled for one day. After the reaction completed, the result was cooled and filtered using florisil/silica, and extracted with ethyl acetate and water. The organic layer was dried using anhydrous sodium sulfate and filtered, and after the solvent was removed by vacuum distillation again, the result was separated and purified using column chromatography, and 480 mg (40%) of target Compound 353 was obtained.

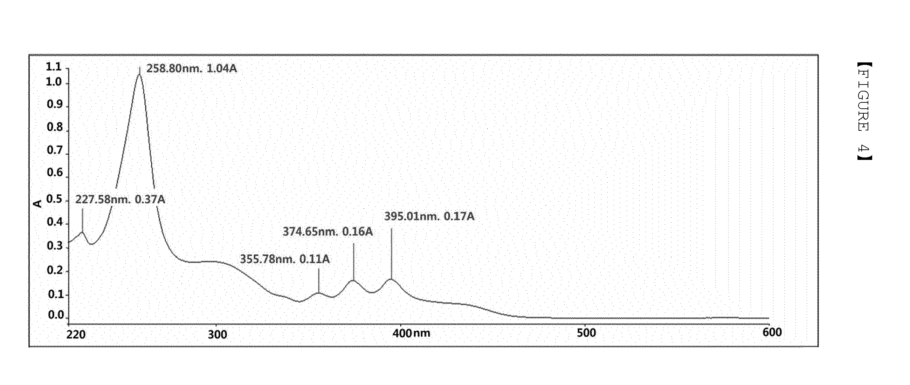

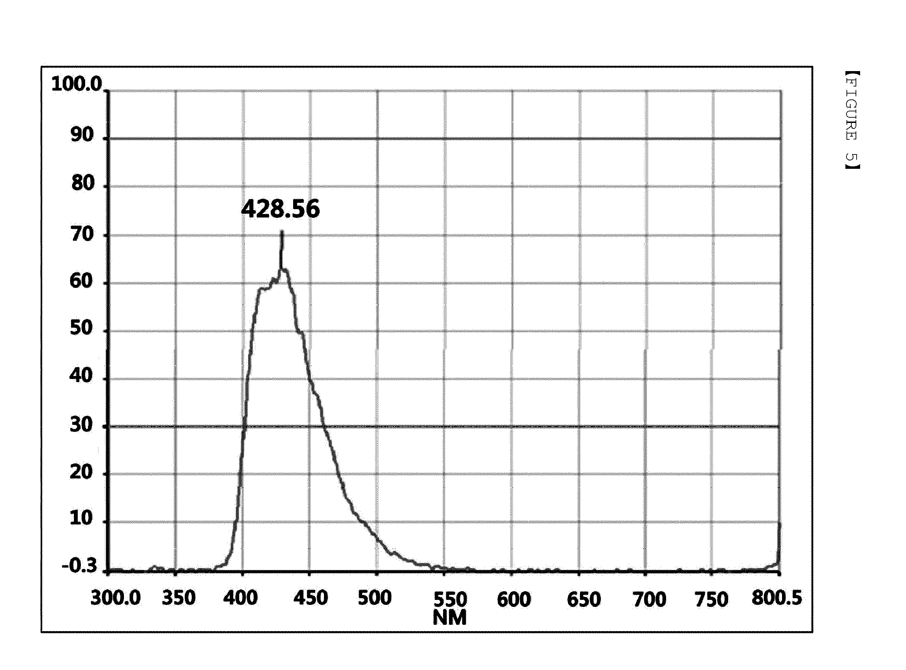

UV data of Compound 353 are shown by a diagram in Fig. 4, and PL data of Compound 353 are shown by a diagram in Fig. 5. In Fig. 4, the y axis is intensity, and the x axis is wavelength (unit: nm). In Fig. 5, the y axis is intensity, and the x axis is wavelength (unit: nm).

The HOMO, the LUMO, and the band gap of Compound 353 are shown in the following Table 1.

**[Table 1]**

| HOMO | LUMO | Band Gap |
|---|---|---|
| -5.43 eV | -2.74 eV | 2.68 eV |

The compounds were prepared using the methods of the preparation examples, and identification results of the synthesis are shown in Table 2.

**[Table 2]**

| Compound | ¹H NMR (CDCl₃, 200 MHz) | MS/FAB | |
|---|---|---|---|
| | | found | calculated |
| 1 | δ=7.41(6H, t), 7.51(12H, t), 7.52(8H, d), 7.54(2H, s), 7.79(4H, d). | 608.7 7 | 608.25 |
| 2 | δ=7.41(5H, t), 7.51(10H, t), 7.52(6H, d), 7.54(2H, s), 7.55(2H, t), 7.61(1H, t), 7.79(4H, d), 8.04(1H, d), 8.08(1H, d), 8.42(1H, d), 8.55(1H, d). | 658.8 3 | 658.27 |
| 3 | δ=7.41(5H, t), 7.51(10H, t), 7.52(6H, d), 7.54(2H, s), 7.58(1H, s), 7.59(2H, t), 7.73(1H, d), 7.79(4H, d), 7.92(1H, d), 8.00(2H, d). | 658.8 3 | 658.27 |
| 4 | δ=7.39(4H, t), 7.41(5H, t), 7.51(10H, t), 7.52(6H, d), 7.54(2H, s), 7.79(4H, d), 7.91(4H, d), 8.27(1H, s). | 708.8 9 | 708.28 |
| 5 | δ=7.41(5H, t), 7.51(10H, t), 7.52(6H, d), 7.54(2H, s), 7.71(2H, d), 7.79(4H, d), 7.82(1H, t), 7.88(1H, d), 8.04(1H, d), 8.12(1H, d), 8.18(1H, d), 8.93(1H, d), 9.15(1H, s). | 708.8 9 | 708.28 |
| 6 | δ=7.41(5H, t), 7.51(10H, t), 7.52(6H, d), 7.54(2H, s), 7.71(2H, d), 7.79(4H, d),7.82(1H, t), 7.88(1H, d), 7.94(1H, d), 8.04(1H, d), 8.12(1H, d), 8.89(1H, d), 8.93(1H, d). | 708.8 9 | 708.28 |
| 7 | δ=7.41(5H, t), 7.51(10H, t), 7.52(6H, d), 7.54(2H, s), 7.71(4H, d), 7.79(4H, d), 7.82(1H, t), 7.88(1H, d), 8.04(1H, d), 8.12(1H, d), 8.18(1H, d). | 732.9 1 | 732.28 |
| 11 | δ=7.41(4H, t), 7.51(8H, t), 7.52(4H, d), 7.54(2H, s), 7.55(4H, t), 7.61(2H, t), 7.79(4H, d), 8.04(2H, d), 8.08(2H, d), 8.42(2H, d), 8.55(2H, d). | 708.8 9 | 708.28 |
| 12 | δ=7.41(4H, t), 7.51(8H, t), 7.52(4H, d), 7.54(2H, s), 7.58(2H, s), 7.59(4H, t), 7.73(1H, d), 7.78(1H, s), 7.79(4H, d), 7.92(2H, d), 8.00(4H, d). | 708.8 9 | 708.28 |
| 13 | δ=7.41(4H, t), 7.51(8H, t), 7.52(4H, d), 7.54(2H, s), 7.71(4H, d), 7.79(4H, d), 7.82(2H, t), 7.88(2H, t), 7.94(2H, t), 7.94(2H, t), 8.04(2H, d), 8.12(2H, d), 8.89(2H, d), 8.93(2H, d). | 809.0 0 | 808.31 |
| 14 | δ=7.41(4H, t), 7.51(8H, t), 7.52(4H, d), 7.54(2H, s), 7.71(8H, d), 7.79(4H, d), 7.82(2H, t), 7.88(2H, t), 8.04(2H, d), 8.12(2H, d), 8.89(2H, d). | 857.0 5 | 856.31 |
| 15 | δ=1.72(12H, s), 7.28(2H, t), 7.38(2H, t), 7.41(4H, t), 7.44(2H, t), 7.51(8H, t), 7.52(4H, d), 7.53(2H, d), 7.54(2H, s), 7.55(2H, d), 7.79(4H, d), 7.83(2H, d), 7.87(2H, d). | 841.0 9 | 840.38 |
| 16 | δ=7.25(2H, t), 7.51(8H, t), 7.29(2H, t), 7.33(2H, t), 7.41(4H, t), 7.50(2H, t), 7.51(8H, t), 7.52(4H, d), 7.54(2H, s), 7.63(2H, d), 7.79(4H, d), 7.94(2H, t), 8.12(2H, d), 8.12(2H, d), 8.55(2H, d). | 786.9 6 | 786.30 |
| 17 | δ=7.41(4H, t), 7.51(8H, t), 7.52(4H, d), 7.54(2H, s), 7.71(4H, d), 7.79(4H, d), 7.82(2H, t), 7.88(2H, t), 8.04(2H, d), 8.12(2H, d), 8.18(2H, d), 8.93(2H, d), 9.15(2H, s). | 809.0 0 | 808.31 |
| 18 | δ=7.41(2H, t), 7.51(4H, t), 7.52(4H, d), 7.55(4H, t), 7.61(2H, t), 8.04(2H, d), 8.08(2H, d), 8.10(2H, t), 8.42(4H, d), 8.55(2H, d). | 556.6 9 | 556.22 |
| 19 | δ=7.41(2H, t), 7.51(4H, t), 7.52(4H, d), 7.58(2H, s), 7.59(4H, t), 7.73(2H, d), 7.92(2H, d), 8.00(4H, d), 8.10(2H, t), 8.42(2H, d). | 556.6 9 | 556.22 |
| 20 | δ=7.41(2H, t), 7.51(4H, t), 7.52 (4H, d),7.71(4H, d), 7.82(2H, t), 7.88(2H, t), 7.94(2H, t), 8.04(1H, d), 8.10(2H, t), 8.12(2H, d), 8.42(2H, d), 8.89(2H, s), 8.93(2H, d). | 656.81 | 565.25 |
| 21 | δ=7.25(4H, d), 7.41 (6H, t), 7.51(12H, t), 7.52 (8H, d), 7.54(2H, s), 7.79(4H, d). | 684.8 6 | 684.28 |
| 22 | δ=7.25(4H, d), 7.41 (5H, t), 7.51(10H, t), 7.52 (6H, d), 7.54(2H, s), 7.55(2H, d), 7.61(1H, t), 7.79(4H, d), 8.04(1H, d), 8.08(1H, s), 8.42(1H, d), 8.55(1H, s). | 734.9 2 | 734.30 |
| 23 | δ=7.25(4H, d), 7.41 (5H, t), 7.51(10H, t), 7.52 (6H, d), 7.54(2H, s), 7.58(1H, s), 7.59(2H, t), 7.73(1H, d), 7.79(4H, d), 7.92(1H, d), 8.00(2H, d). | 734.9 2 | 734.30 |
| 24 | δ=7.41(6H, t), 7.51(12H, t), 7.52(8H, d), 7.54(2H, s), 7.48(2H, d), 7.57(1H, t), 7.70(1H, s), 7.79(4H, d). | 684.8 6 | 684.28 |
| 25 | δ=7.41(6H, t), 7.51(12H, t), 7.47(2H, d), 7.52(6H, d), 7.54(2H, s), 7.79(6H, d), 7.85(2H, d). | 684.8 6 | 684.28 |
| 26 | δ=3.22(1H, s), 6.39(1H, d), 6.58(1H, d), 7.27(1H, t), 7.29(1H, d), 7.41(5H, t), 7.51(10H, t), 7.52(6H, d), 7.54(2H, s), 7.79(4H, d), 7.83(1H, s). | 646.8 2 | 646.27 |
| 27 | δ=3.22(1H, d), 6.39(1H, t), 6.58(1H, d), 7.21(1H, d), 7.29(1H, d), 7.41(5H, t), 7.51(10H, t), 7.52 (6H, d), 7.54(2H, s), 7.79(4H, d), 8.10(1H, d). | 646.8 2 | 646.27 |
| 28 | δ=3.22(1H, s), 6.94(1H, s), 7.21(2H, t), 7.26(1H, d), 7.33(1H, d), 7.41(5H, t), 7.51(10H, t), 7.52(4H, d), 7.16(2H, d), 7.54(2H, s), 7.79(4H, d). | 646.8 2 | 646.27 |
| 29 | δ=7.41(5H, t), 7.51(10H, t), 7.52(6H, d), 7.53(12H, t), 7.54(2H, s), 7.79(4H, d), 8.01(1H, d), 8.18(1H, d). | 665.8 4 | 665.22 |
| 30 | δ=7.39(2H, d), 7.41(5H, t), 7.51(10H, t), 7.52(6H, d), 7.54(2H, s), 7.74(2H, t), 7.79(4H, d). | 649.7 8 | 649.24 |
| 31 | δ=7.25(8H, d), 7.41(6H, t), 7.51(12H, t), 7.52(8H, d), 7.54(2H, s), 7.79(4H, d). | 760.9 6 | 760.31 |
| 33 | δ=7.25(8H, d), 7.41(4H, t), 7.51(8H, t), 7.52(4H, d), 7.54(2H, s), 7.58(2H, s), 7.59(4H, t), 7.73(2H, d), 7.79(4H, d), 7.92(2H, d), 8.00(4H, d). | 861.0 8 | 860.34 |
| 35 | δ=7.41(4H, t), 7.48(4H, d), 7.51(8H, t), 7.52(4H, d), 7.54(2H, s), 7.57(2H, t), 7.58(2H, s), 7.59(4H, t), 7.70(2H, s), 7.73(2H, d), 7.79(4H, d), 7.92(2H, d), 8.00(4H, d). | 861.0 8 | 860.34 |
| 37 | δ=7.41(6H, t), 7.47(4H, t), 7.51(12H, t), 7.52(4H, d), 7.54(2H, s), 7.79(8H, d), 7.85(4H, d). | 760.9 6 | 760.31 |
| 39 | δ=3.22(2H, s), 6.39(2H, t), 6.58(2H, d), 7.21(2H, d), 7.24(2H, d), 7.41(4H, t), 7.51(8H, t), 7.52(4H, d), 7.54(2H, s), 7.79(4H, d), 7.83(2H, s). | 684.8 6 | 684.28 |
| 41 | δ=6.52(1H, d), 7.41(5H, t), 7.45(1H, t), 7.50(2H, d), 7.51(10H, t), 7.52(6H, d), 7.54(2H, s), 7.58(2H, t), 7.60(1H, d), 7.62(1H, s), 7.79(4H, d), 8.10(1H, d), 8.49(1H, d). | 723.9 0 | 723.29 |
| 43 | δ=7.41(5H, t), 7.51(10H, t), 7.52(6H, d), 7.54(2H, s), 7.58(1H, t), 7.79(5H, d), 7.80(1H, d), 7.90(1H, d), 7.96(1H, d), 8.10(2H, t), 8.42(2H, d). | 732.9 1 | 732.28 |
| 44 | δ=7.41(5H, t), 7.47(2H, t), 7.51(10H, t), 7.52(6H, d), 7.54(2H, s), 7.58(1H, s), 7.59(2H, t), 7.73(1H, d), 7.79(4H, d), 7.85(2H, d), 7.92(1H, d), 8.00(2H, d). | 734.9 2 | 734.30 |
| 47 | δ=7.15(2H, d), 7.41(5H, t), 7.51(10H, t), 7.52(6H, d), 7.54(2H, s), 7.58(1H, t), 7.79(5H, d), 7.80(1H, d), 7.90(1H, d), 7.96(1H, d). | 682.85 | 682.27 |
| 49 | δ=7.39(4H, t), 7.41(5H, t), 7.51(10H, t), 7.52(6H, d), 7.54(2H, s), 7.55(2H, t), 7.61(1H, t), 7.79(4H, d), 7.91(4H, d), 8.04(1H, d), 8.08(1H, d), 8.42(1H, d), 8.55(1H, d). | 835.0 5 | 834.33 |
| 51 | δ=7.41(6H, t), 7.51(12H, t), 7.52(8H, d), 7.54(2H, s), 7.58(2H, s), 7.73(2H, d), 7.79(4H, d), 7.92(2H, d). | 734.9 2 | 734.30 |
| 53 | δ=7.41(5H, t), 7.51(10H, t), 7.52(6H, d), 7.54(2H, s), 7.55(2H, t), 7.58(2H, s), 7.61(1H, t), 7.73(4H, d), 7.92(2H, d), 8.04(1H, d), 8.08(1H, d), 8.42(1H, d), 8.55(1H, d). | 784.9 8 | 784.31 |
| 54 | δ=7.41(5H, t), 7.51(10H, t), 7.52(6H, d), 7.54(2H, s), 7.55(2H, t), 7.61(2H, t), 7.79(4H, d), 7.89(1H, s), 7.95(1H, d), 8.04(2H, d), 8.08(2H, d), 8.14(1H, d), 8.42(2H, d), 8.55(1H, d). | 784.9 8 | 784.31 |
| 55 | δ=7.25(4H, d), 7.41(6H, t), 7.48(2H, d), 7.51(12H, t), 7.52(8H, d), 7.54(2H, s), 7.57(1H, t), 7.70(1H, s), 7.79(4H, d). | 760.9 6 | 760.31 |
| 56 | δ=7.25(4H, d), 7.41(6H, t), 7.47(2H, t), 7.51(12H, t), 7.52(8H, d), 7.54(2H, s), 7.79(4H, d), 7.85(2H, d). | 760.9 6 | 760.31 |
| 57 | δ=1.72(2H, d), 7.25(4H, d), 7.28(1H, t), 7.34(1H, t), 7.38(1H, t), 7.41(5H, t), 7.51(1H, t), 7.52(6H, d), 7.54(2H, s), 7.55(1H, d), 7.63(1H, d), 7.79(4H, d), 7.87(1H, d). | 801.0 2 | 800.34 |
| 59 | δ=7.25(1H, t), 7,33(1H, t), 7.41(5H, t), 7.43(1H, t), 7.45(1H, t), 7.50(2H, d), 7.51(10H, t), 7.52(6H, d), 7.54(2H, s), 7.58(2H, t), 7.59(1H, d), 7.79(5H, d), 7.94(1H, d), 8.55(1H, d). | 773.9 6 | 773.31 |
| 60 | δ=7.29(1H, t), 7,41(5H, t), 7.45(1H, t), 7.50(3H, d), 7.51(10H, t), 7.52(6H, d), 7.54(2H, s), 7.58(2H, t), 7.79(4H, d), 7.77(1H, s), 7.63(1H, d), 8.00(1H, d), 8.18(1H, d). | 773.9 6 | 773.31 |
| 61 | δ=6.52(2H, d), 7.41(4H, t), 7.45(2H, t), 7.50(4H, d), 7.51(8H, t), 7.52(4H, d), 7.54(2H, s), 7.58(4H, t), 7.60(2H, d), 7.62(2H, d), 7.79(4H, d), 8.10(2H, d), 8.49(2H, d). | 839.0 3 | 838.33 |
| 62 | δ=7.41(4H, t), 7.47(2H, t), 7.51(8H, t), 7.52(4H, d), 7.54(6H, m), 7.69(2H, d), 7.79(4H, d), 8.03(2H, d), 8.30(4H, d), 8.78(2H, d). | 762.94 | 762.30 |
| 63 | δ=7.41(4H, t), 7.51(8H, t), 7.52(4H, d), 7.54(2H, s), 7.58(2H, d), 7.69(2H, d), 7.79(6H, d), 7.80(2H, d), 7.90(2H, d), 7.96(2H, d), 8.10(4H, t), 8.42(4H, d). | 857.0 5 | 856.31 |
| 66 | δ=7.39(4H, t), 7.41(6H, t), 7.51(12H, t), 7.52(8H, d), 7.54(2H, s), 7.79(4H, d), 7.91(4H, d). | 784.9 8 | 874.31 |
| 67 | δ=7.15(4H, d), 7.41(4H, t), 7.51(8H, t), 7.52(4H, d), 7.54(2H, s), 7.58(2H, d), 7.79(6H, d), 7.80(2H, d), 7.90(2H, d), 7.96(2H, d). | 756.9 3 | 756.28 |
| 69 | δ=7.39(4H, d), 7.41(5H, t), 7.51(10H, t), 7.52(6H, d), 7.54(2H, s), 7.55(2H, t), 7.61(1H, t), 7.79(4H, d), 7.91(4H, d) 8.04(1H, d), 8.08(1H, d), 8.42(1H, d), 8.55(1H, d). | 835.0 4 | 834.33 |
| 109 | δ=7.41(2H, t), 7.51(4H, t), 7.52(4H, d), 7.54(2H, s), 7.58(4H, s), 7.59(8H, t), 7.73(4H, d), 7.92(4H, d) 8.00(8H, d). | 809.0 0 | 808.31 |
| 129 | δ=3.22(3H, s), 6.39(3H, s), 6.58(3H, s), 7.21(3H, d), 7.24(3H, d), 7.41(3H, t), 7.51(6H, t), 7.52(2H, d), 7.54(2H, s), 7.79(4H, d), 7.83(3H, d). | 722.9 1 | 722.30 |
| 199 | δ=3.22(3H, s), 6.39(3H, s), 6.58(3H, s), 7.21(3H, m), 7.24(3H, d), 7.41(2H, t), 7.51(4H, t), 7.52(2H, d), 7.54(2H, s), 7.71(2H, d), 7.79(2H, d), 7.82(2H, t), 7.83(3H, d), 7.88(2H, d), 8.10(1H, d), 8.12(1H, d), 8.34(1H, s), 8.93(2H, d). | 872.3 4 | 873.09 |
| 203 | δ=7.41(6H, t), 7.51(12H, t), 7.52(12H, d), 7.58(2H, d), 7.59(4H, t), 7.73(2H, s), 7.92(2H, d) 8.00(3H, d). | 861.0 8 | 860.34 |
| 231 | δ=2.34(6H, s), 7.25(8H, d), 7.41(6H, t), 7.51(12H, t), 7.52(11H, d). | 789.0 1 | 788.34 |
| 280 | δ=2.34(6H, s), 7.25(2H, t), 7.27(2H, t), 7.29(2H, t), 7.33(2H, t), 7.61(2H, t), 7.94(1H, d), 8.03(1H, d), 8.03(1H, d), 8.09(2H, d), 8.12(2H, d), 8.55(1H, d), 8.98(2H, d), 9.34(1H, d). | 822.9 1 | 822.30 |
| 301 | δ=7.41(5H, t), 7.51(10H, t), 7.52(6H, d), 7.54(2H, s), 7.79(4H, d). | 532.6 7 | 532.22 |
| 302 | δ=7.41(4H, t), 7.51(8H, t), 7.52(4H, d), 7.54(2H, s), 7.61(1H, t), 7.79(4H, d), 8.04(1H, d), 8.08(1H, d), 8.42(1H, d), 8.55(1H, d). | 582.7 3 | 582.23 |
| 303 | δ=7.41(3H, t), 7.51(7H, t), 7.52(2H, d), 7.54(2H, s), 7.55(4H, t), 7.79(4H, d), 8.04(2H, d), 8.08(2H, d), 8.55(2H, d). | 632.7 9 | 632.25 |
| 304 | δ=7.41(1H, t), 7.51(2H, t), 7.52(2H, d), 7.54(4H, t), 7.61(2H, t), 8.04(2H, d), 8.08(2H, d), 8.1(2H, t), 8.42(2H, d), 8.55(2H, d). | 480.6 0 | 480.19 |
| 305 | δ=7.41(1H, t), 7.51(2H, t), 7.52(2H, d), 7.54(2H, s), 7.58(4H, s), 7.59(8H, t), 7.73(4H, d), 7.92(4H, d), 8.00(8H, d). | 732.9 1 | 732.28 |
| 306 | δ=3.22(4H, s), 6.39(2H, d), 6.58(2H, d), 7.21(2H, d), 7.24(2H, d), 7.41(3H, t), 7.51(6H, t), 7.52(2H, d), 7.54(2H, s), 7.79(4H, d), 7.83(2H, d). | 608.7 7 | 608.25 |
| 307 | δ=3.22(4H, s), 6.31(2H, s), 6.58(2H, d), 7.21(2H, d), 7.24(2H, d), 7.41(2H, t), 7.51(4H, t), 7.52(2H, d), 7.54(2H, s), 7.88(2H, t), 8.04(1H, d), 8.12(1H, d), 8.18(1H, d), 8.93(2H, d), 9.15(1H, d). | 758.9 4 | 8758.3 0 |
| 308 | δ=7.41(6H, t), 7.51(12H, t), 7.52(12H, d), 7.58(1H, s), 7.59(2H, t), 8.00(2H, d), 8.12(1H, d), 8.18(1H, d), 8.93(2H, d), 9.15(1H, d). | 734.9 2 | 734.30 |
| 309 | δ=2.34(6H, s), 7.25(4H, d), 7.41(5H, t), 7.51(10H, t), 7.52(10H, d). | 636.82 | 636.28 |
| 310 | δ=2.34(6H, s), 7.25(1H, t), 7.27(2H, t), 7.29(1H, t), 7.33(1H, t), 7.61(2H, t), 7.63(1H, t), 8.03(1H, d), 8.09(3H, d), 8.12(1H, d), 8.55(1H, d), 8.98(2H, d), 9.34(1H, d). | 657.7 2 | 657.24 |
| 321 | δ=7.41(4H, t), 7.51(8H, t), 7.52(8H, d), 7.82(2H, t), 7.88(2H, t), 8.12(3H, d), 8.93(3H, d). | 606.7 5 | 606.23 |
| 331 | δ=7.41(4H, t), 7.51(8H, t), 7.52(8H, d), 7.82(2H, t), 7.88(2H, t), 8.12(2H, d), 8.31(2H, s), 8.93(2H, d). | 656.8 1 | 656.25 |
| 353 | δ=7.39(3H, t), 7.41(3H, t), 7.48(4H, d), 7.51(6H, t), 7.52(6H, d), 7.57(1H, t), 7.70(1H, s), 7.91(4H, d), 8.10(2H, t), 8.42(2H, d). | 632.7 9 | 632.25 |

### Comparative Example: Manufacture of OLED Device

First, a transparent electrode ITO thin film obtained from an OLED glass (manufactured by Samsung Corning Co. Ltd.) was ultrasonic cleaned using trichloroethylene, acetone, ethanol and distilled water in consecutive order, and was used after being cleaned using isopropyl alcohol.

Next, after an ITO substrate was installed on the substrate folder of vacuum deposition apparatus, and was exhausted until the degree of vacuum within the vacuum deposition apparatus reaches 10⁻⁷ torr, a hole injection layer having a thickness of 600 Å was deposited on the ITO substrate by vapor depositing the following 4,4',4"-tris(N,N-(2-naphthyl)-phenylamino)triphenyl amine (2-TNATA).

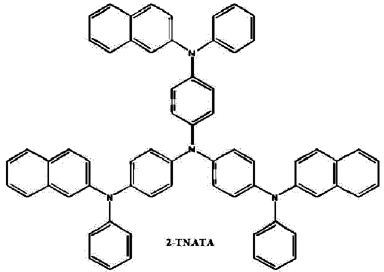

Subsequently, a hole transfer layer having a thickness of 250 Å was deposited on the hole injection layer by placing the following N,N'-bis(α-naphthyl)-N,N'-diphenyl-4,4'-diamine (NPB) in another cell within the vacuum deposition apparatus, and evaporating NPB through the application of current to the cell.

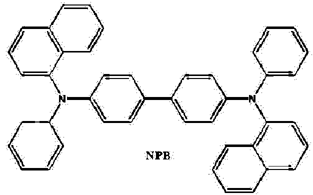

After the hole injection layer and the hole transfer layer were formed, a light emitting layer was deposited thereon as follows. In one cell within the vacuum deposition apparatus, the following host was placed as a light emitting material, and the following dopant was placed in another cell.

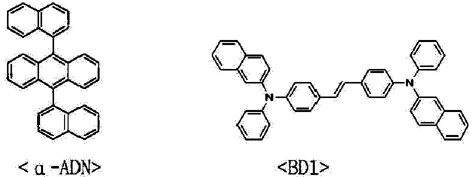

Subsequently, a light emitting layer was deposited to a thickness of 200 Å on the hole transfer layer by heating the two cells together, and depositing with the deposition rate ratio of the dopant to be 5% by weight (host:dopant=95:5). Next, the following tris(8-hydroxyquinoline)aluminum(III) (Alq) was deposited to a thickness of 200 Å as an electron transfer layer.

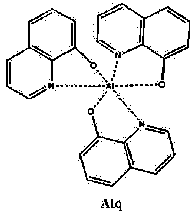

After that, lithium fluoride (LiF) was deposited to a thickness of 10 Å as an electron injection layer. Next, an OLED was manufactured by depositing an Al cathode to a thickness of 1200 Å.

Meanwhile, each of all the organic compound materials necessary for the manufacture of an OLED device was vacuumed, sublimed, and purified under 10⁻⁶ to 10⁻⁸ torr, and used in the manufacture.

### Example: Manufacture of OLED Device

The OLED was manufactured using the same method as in a comparative example except that the compounds on the following table 3 were used as the material of the light emitting layer instead of α-AND in the comparative example.

### Experimental Example 1: Evaluation of OLED Characteristics

Driving voltage(V), power efficiency (cd/A) and driving life span of the OLED device manufactured as describe above were measured at 1,000cd/m², and as the time taken for the efficiency to drop to 50%, and the results are shown in the following Table 3.

**[Table 3]**

| Example No. | Compound No. | Op.V | Cd/A | T50 |
|---|---|---|---|---|
| 1 | 1 | 5.12 | 4.3 | 410 |
| 2 | 2 | 5.11 | 4.5 | 430 |
| 3 | 3 | 5.04 | 4.6 | 460 |
| 4 | 4 | 5.04 | 4.7 | 480 |
| 5 | 5 | 5.08 | 4.5 | 440 |
| 6 | 6 | 5.06 | 4.5 | 440 |
| 7 | 7 | 5.04 | 4.7 | 480 |
| 8 | 11 | 5.04 | 4.7 | 480 |
| 9 | 12 | 5.08 | 4.6 | 470 |
| 10 | 13 | 5.11 | 4.3 | 390 |
| 11 | 14 | 5.10 | 4.5 | 460 |
| 12 | 15 | 5.11 | 4.4 | 420 |
| 13 | 16 | 5.09 | 4.5 | 440 |
| 14 | 17 | 5.09 | 4.5 | 450 |
| 15 | 18 | 5.00 | 4.6 | 460 |
| 16 | 19 | 4.99 | 4.6 | 470 |
| 17 | 20 | 4.98 | 4.6 | 480 |
| 18 | 21 | 5.09 | 4.6 | 440 |
| 19 | 22 | 4.99 | 4.7 | 480 |
| 20 | 23 | 5.06 | 4.7 | 480 |
| 21 | 24 | 5.08 | 4.7 | 460 |
| 22 | 25 | 5.06 | 4.7 | 460 |
| 23 | 26 | 5.06 | 4.7 | 460 |
| 24 | 27 | 5.06 | 4.7 | 470 |
| 25 | 28 | 4.94 | 4.8 | 510 |
| 26 | 29 | 5.08 | 4.7 | 460 |
| 27 | 30 | 4.96 | 4.7 | 500 |
| 28 | 31 | 5.05 | 4.8 | 490 |
| 29 | 33 | 5.08 | 4.5 | 440 |
| 30 | 35 | 5.07 | 4.6 | 470 |
| 31 | 37 | 5.06 | 4.8 | 480 |
| 32 | 39 | 4.98 | 4.7 | 470 |
| 33 | 41 | 4.94 | 4.8 | 510 |
| 34 | 43 | 4.95 | 4.8 | 510 |
| 35 | 44 | 5.03 | 4.6 | 450 |
| 36 | 47 | 5.01 | 4.6 | 460 |
| 37 | 49 | 4.98 | 4.7 | 470 |
| 38 | 51 | 5.01 | 4.8 | 510 |
| 39 | 53 | 5.07 | 4.5 | 430 |
| 40 | 54 | 5.06 | 4.5 | 450 |
| 41 | 57 | 5.04 | 4.6 | 470 |
| 42 | 59 | 5.04 | 4.8 | 490 |
| 43 | 60 | 4.95 | 4.9 | 510 |
| 44 | 61 | 4.93 | 4.9 | 520 |
| 45 | 62 | 5.03 | 4.7 | 460 |
| 46 | 63 | 5.01 | 4.7 | 480 |
| 47 | 66 | 4.98 | 4.8 | 500 |
| 48 | 67 | 4.97 | 4.8 | 510 |
| 49 | 69 | 4.98 | 4.9 | 530 |
| 50 | 109 | 5.06 | 4.8 | 450 |
| 51 | 129 | 5.04 | 4.8 | 490 |
| 52 | 199 | 5.01 | 4.8 | 490 |
| 53 | 203 | 4.99 | 5.1 | 540 |
| 54 | 231 | 4.89 | 4.9 | 520 |
| 55 | 280 | 4.88 | 5.0 | 530 |
| 56 | 301 | 4.87 | 5.1 | 540 |
| 57 | 302 | 4.91 | 5.0 | 530 |
| 58 | 303 | 4.88 | 5.1 | 540 |
| 59 | 304 | 4.86 | 5.1 | 540 |
| 60 | 305 | 4.83 | 5.2 | 550 |
| 61 | 306 | 4.89 | 4.9 | 520 |
| 62 | 307 | 4.93 | 5.3 | 580 |
| 63 | 308 | 4.83 | 5.2 | 580 |
| 64 | 309 | 4.99 | 4.6 | 570 |
| 65 | 310 | 4.98 | 4.6 | 580 |
| 66 | 321 | 5.09 | 4.6 | 540 |
| 67 | 331 | 4.99 | 4.7 | 580 |
| 68 | 353 | 4.83 | 5.0 | 580 |
| Comparative Example 1 | α-ADN | 6.78 | 4.4 | 370 |

### [Reference]

- 100: Substrate
- 200: Anode
- 300: Organic Material Layer
- 301: Hole Injection Layer
- 302: Hole Transfer Layer
- 303: Light Emitting Layer
- 304: Electron Transfer Layer
- 305: Electron Injection Layer
- 400: Cathode

## Claims

1. A compound of the following Chemical Formula 1: 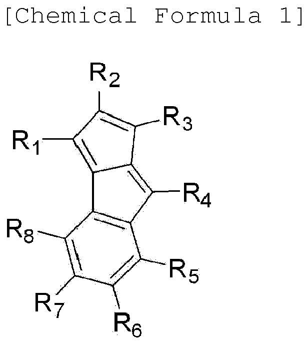 wherein, in Chemical Formula 1,
R₁ to R₈ are selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or form a substituted or unsubstituted monocyclic or multicyclic aliphatic or aromatic hydrocarbon ring, or a substituted or unsubstituted monocyclic or multicyclic aliphatic or aromatic heteroring, by being linked to an adjacent group.

2. The compound of Claim 1, wherein R₁ to R₈ are the same as or different from each other, and each is selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aliphatic or aromatic hydrocarbon ring or a substituted or unsubstituted monocyclic or multicyclic aliphatic or aromatic heteroring, by being linked to an adjacent group, however, not all of R₁ to R₈ are hydrogen.

3. The compound of Claim 1, wherein at least one of R₁ to R₈ is substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; a substituted or unsubstituted acetophenone group; a substituted or unsubstituted benzophenone group; a substituted or unsubstituted C₁₀ to C₆₀ spiro group; or amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or forms a substituted or unsubstituted monocyclic or multicyclic aromatic hydrocarbon ring, or a substituted or unsubstituted monocyclic or multicyclic aromatic heteroring, by being linked to an adjacent group.

4. The compound of Claim 1, wherein Chemical Formula 1 is represented by any one of the following Chemical Formulae 1a to 1g: 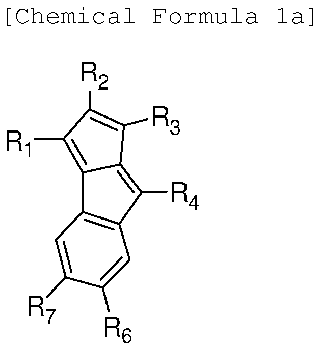 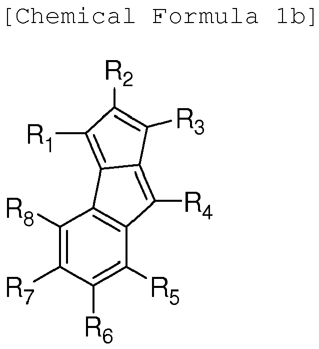 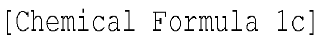 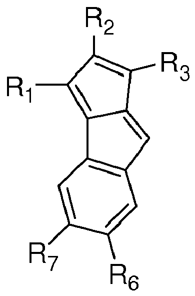 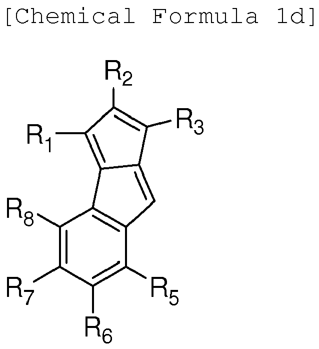 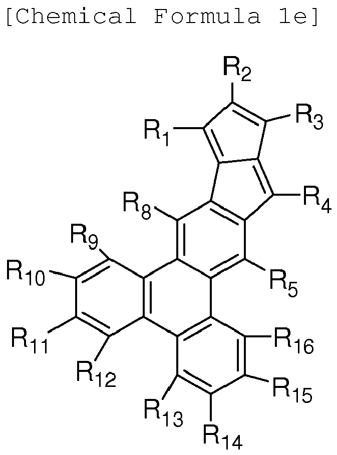 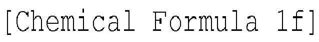 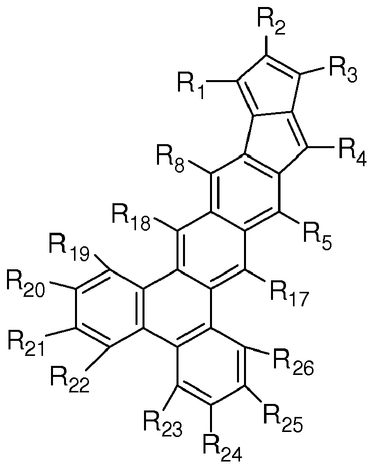 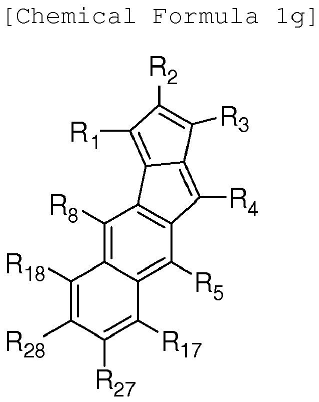 wherein, in Chemical Formulae 1a to 1g,
R₁ to R₈ are the same as or different from each other, each independently selected from the group consisting of halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl; and
R₉ to R₂₈ are selected from the group consisting of hydrogen; halogen; substituted or unsubstituted linear or branched C₁ to C₆₀ alkyl; substituted or unsubstituted linear or branched C₂ to C₆₀ alkenyl; substituted or unsubstituted linear or branched C₂ to C₆₀ alkynyl; substituted or unsubstituted linear or branched C₁ to C₆₀ alkoxy; substituted or unsubstituted monocyclic or multicyclic C₃ to C₆₀ cycloalkyl; substituted or unsubstituted monocyclic or multicyclic C₂ to C₆₀ heterocycloalkyl; substituted or unsubstituted monocyclic or multicyclic C₆ to C₆₀ aryl; substituted or unsubstituted monocyclic or multicyclic C₂ to C₆₀ heteroaryl; and amine unsubstituted or substituted with C₁ to C₂₀ alkyl, substituted or unsubstituted monocyclic or multicyclic C₆ to C₆₀ aryl, or substituted or unsubstituted monocyclic or multicyclic C₂ to C₆₀ heteroaryl, or form a substituted or unsubstituted monocyclic or multicyclic aliphatic or aromatic hydrocarbon ring, or a substituted or unsubstituted monocyclic or multicyclic aliphatic or aromatic heteroring, by being linked to an adjacent group.

5. The compound of Claim 4, wherein, in Chemical Formulae 1a to 1g, R₁ to R₈ are the same as or different from each other, and each is substituted or unsubstituted phenyl, substituted or unsubstituted biphenyl, substituted or unsubstituted triphenyl, substituted or unsubstituted naphthyl, substituted or unsubstituted anthryl, substituted or unsubstituted phenanthrenyl, substituted or unsubstituted indenyl, substituted or unsubstituted perylenyl, substituted or unsubstituted pyrenyl, substituted or unsubstituted acenaphthalenyl, substituted or unsubstituted fluorenyl, substituted or unsubstituted fluoranthenyl, substituted or unsubstituted triphenylenyl, substituted or unsubstituted phenalenyl, substituted or unsubstituted pyrrole, substituted or unsubstituted pyridyl, substituted or unsubstituted pyrimidyl, substituted or unsubstituted pyridazinyl, substituted or unsubstituted triazinyl, substituted or unsubstituted thienyl, substituted or unsubstituted furanyl, substituted or unsubstituted benzothiazole, substituted or unsubstituted benzoxazole, substituted or unsubstituted indolyl, substituted or unsubstituted carbazolyl, substituted or unsubstituted benzocarbazolyl, substituted or unsubstituted quinolyl, substituted or unsubstituted isoquinolyl, a substituted or unsubstituted dibenzothiophene group, a substituted or unsubstituted dibenzofuran group, substituted or unsubstituted indolinyl, a substituted or unsubstituted 10,11-dihydro-dibenzo[b,f]azepine group, a substituted or unsubstituted 9,10-dihydroacridine group, a substituted or unsubstituted spiro group in which 2,3-dihydro-1H-indene or cyclohexane is spiro-bonded to fluorene, substituted or unsubstituted dialkylamine, substituted or unsubstituted diarylamine, substituted or unsubstituted alkylarylamine, a substituted or unsubstituted acetophenone group, or a substituted or unsubstituted benzophenone group.

6. The compound of Claim 4, wherein, in Chemical Formulae 1a to 1g, R₉ to R₂₆ are hydrogen.

7. The compound of Claim 4, wherein, in Chemical Formulae 1a to 1g, R₂₇ and R₂₈ are substituted or unsubstituted monocyclic or multicyclic C₆ to C₆₀ aryl; or substituted or unsubstituted monocyclic or multicyclic C₂ to C₆₀ heteroaryl.

8. The compound of Claim 1, the compound of Chemical Formula 1 is selected from among the following compounds:

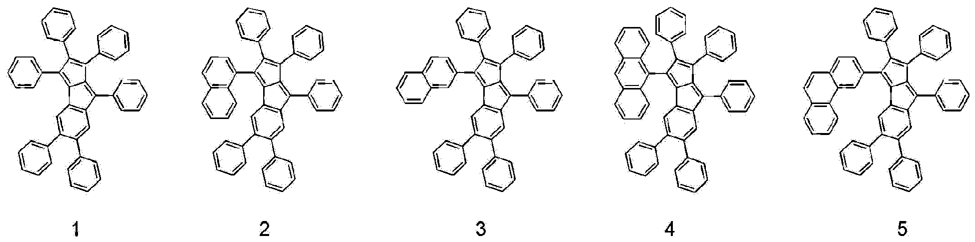

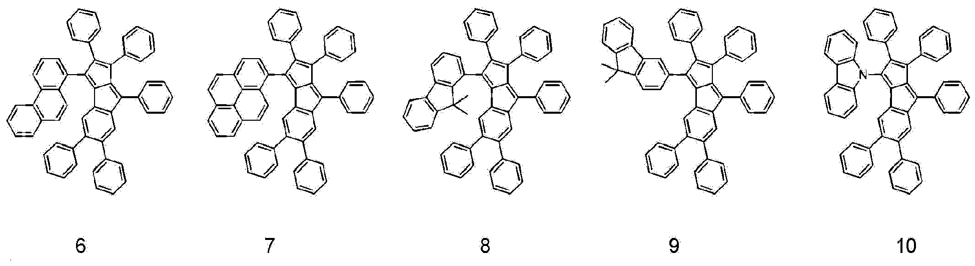

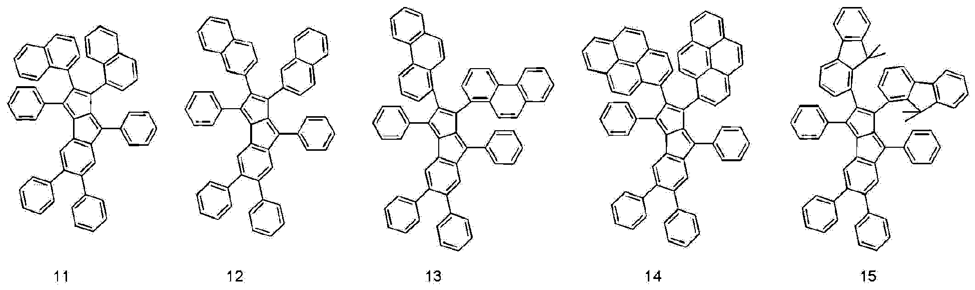

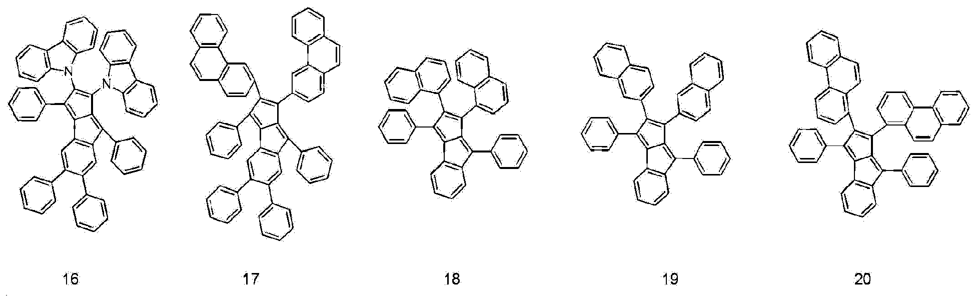

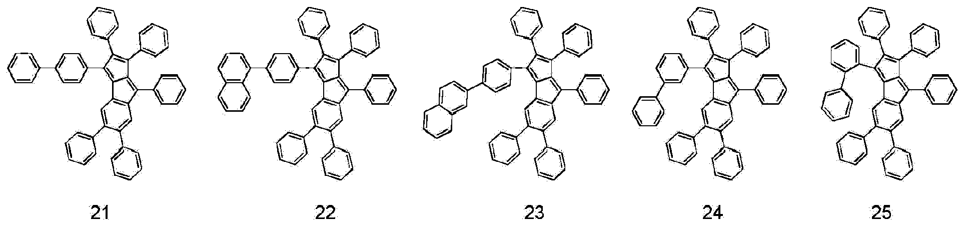

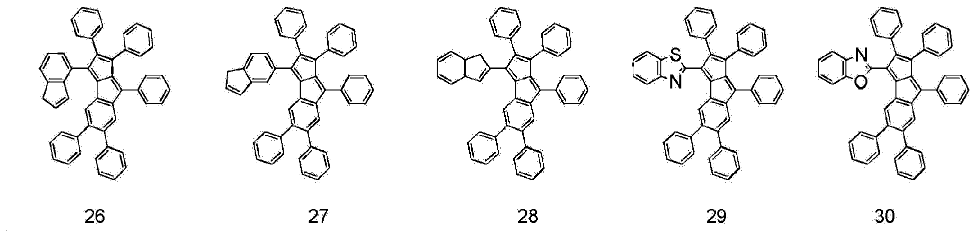

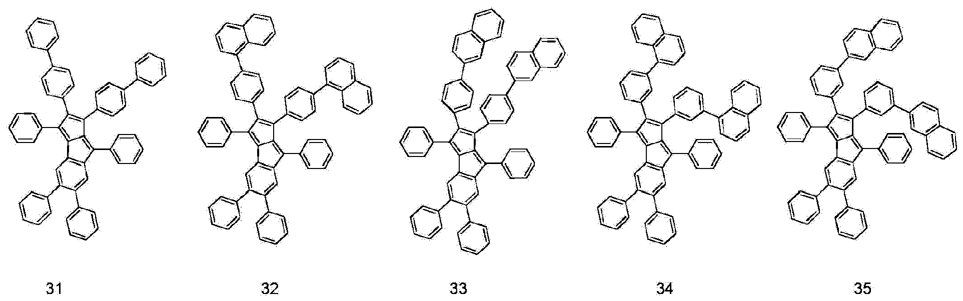

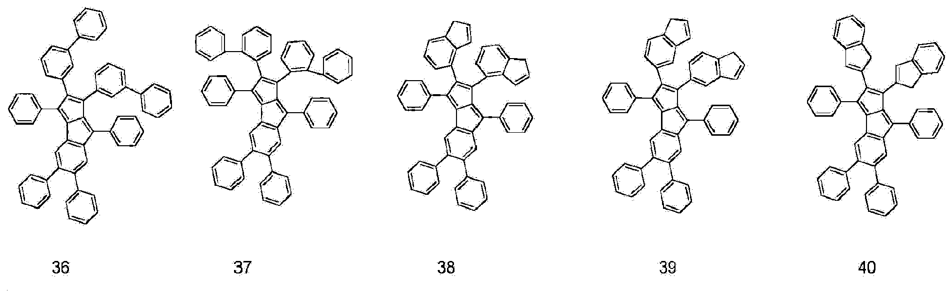

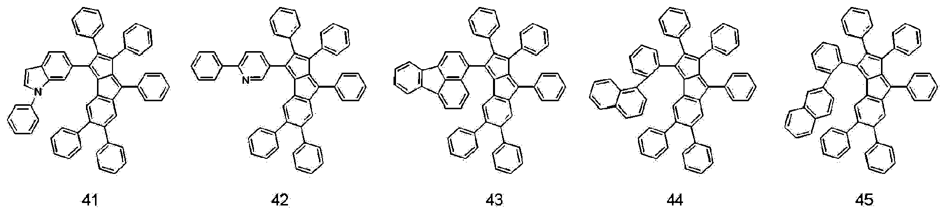

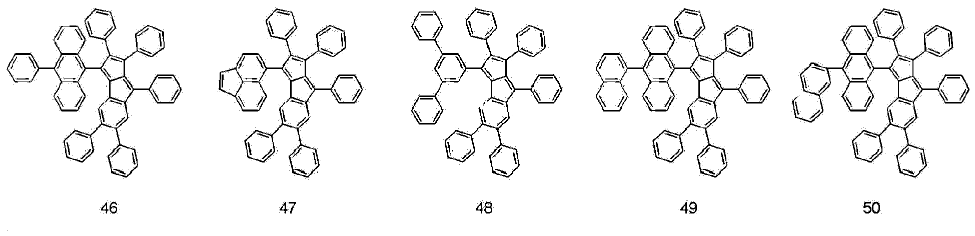

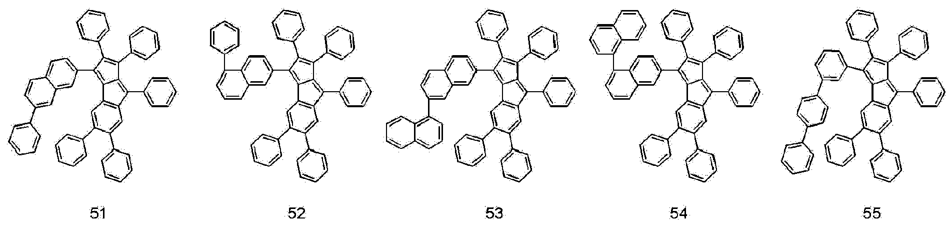

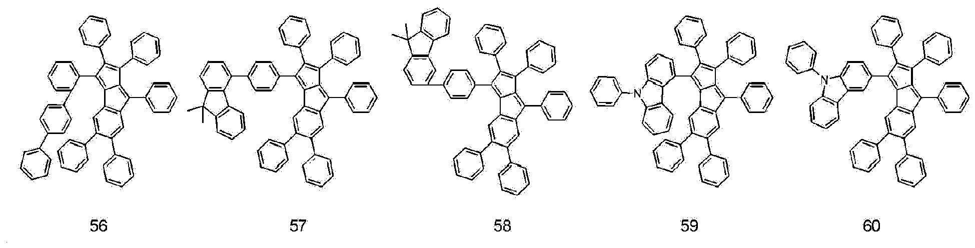

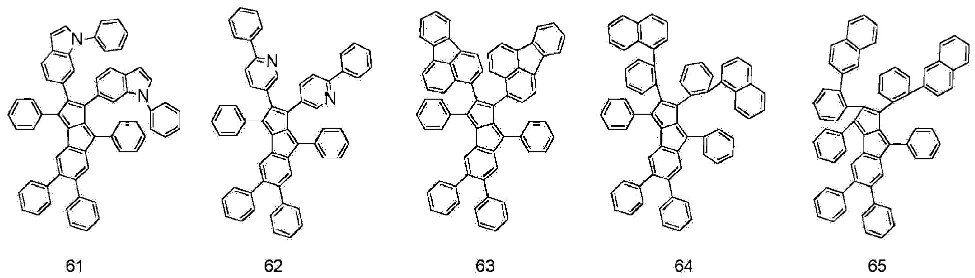

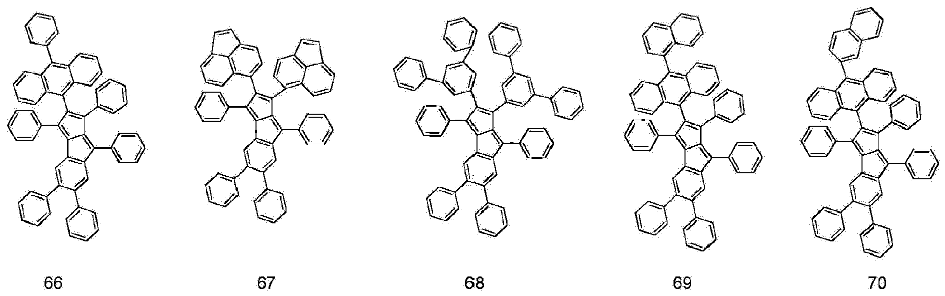

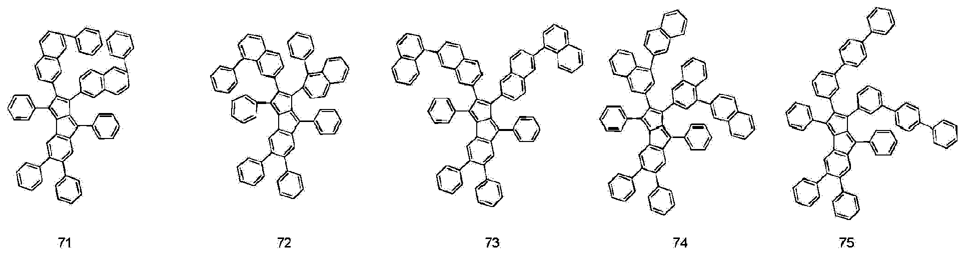

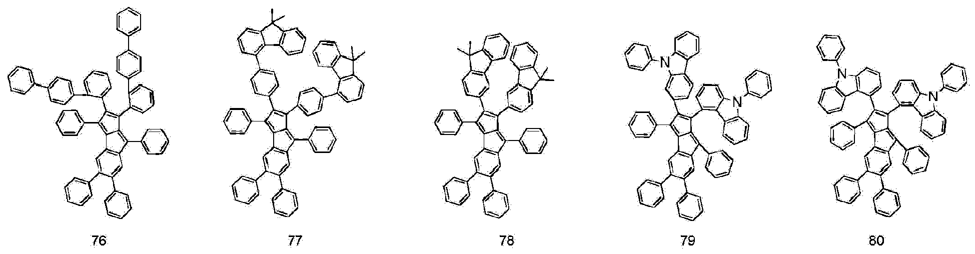

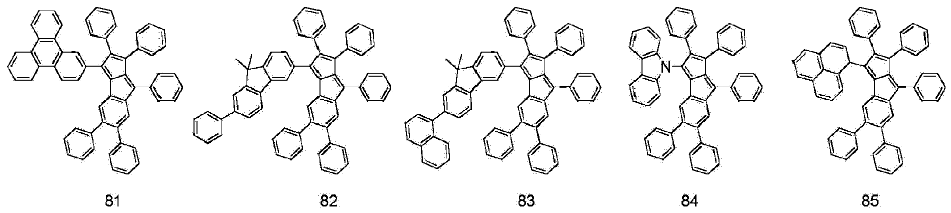

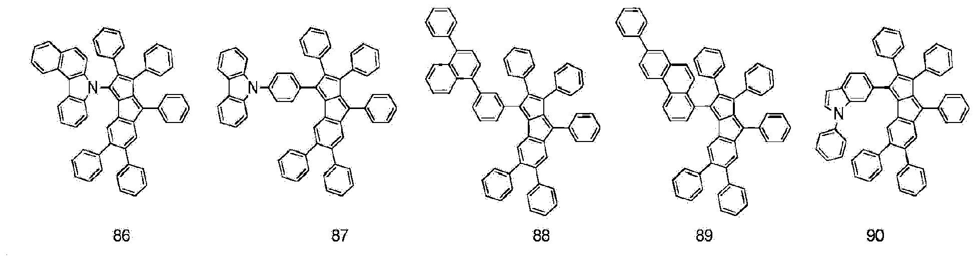

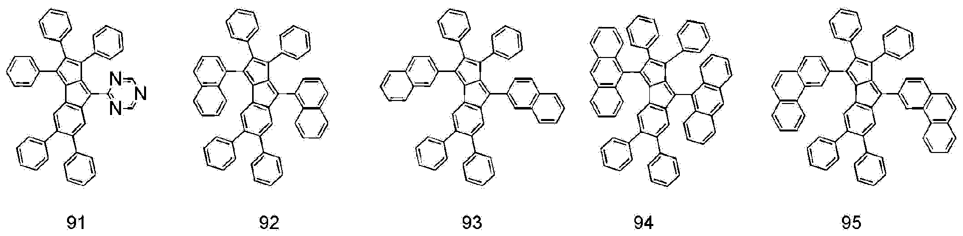

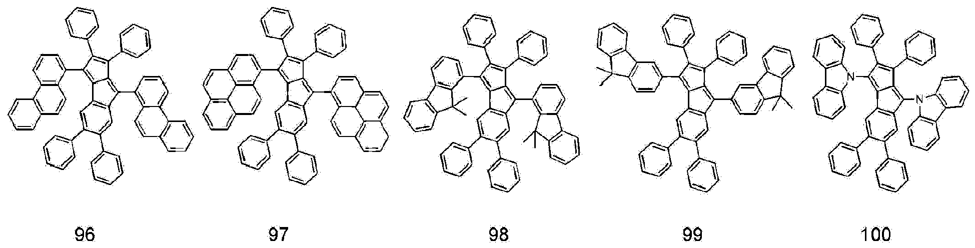

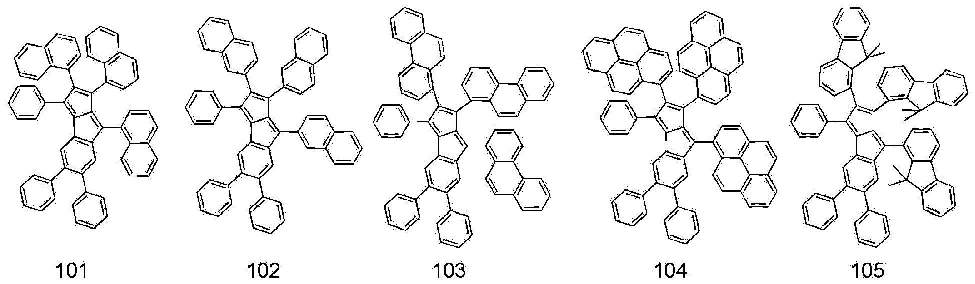

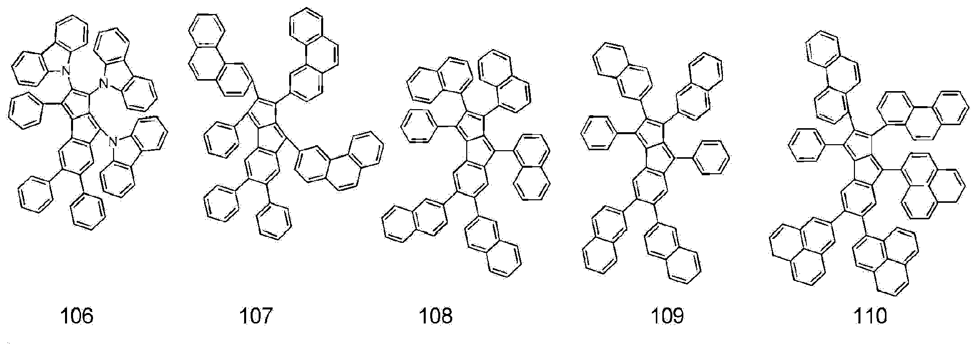

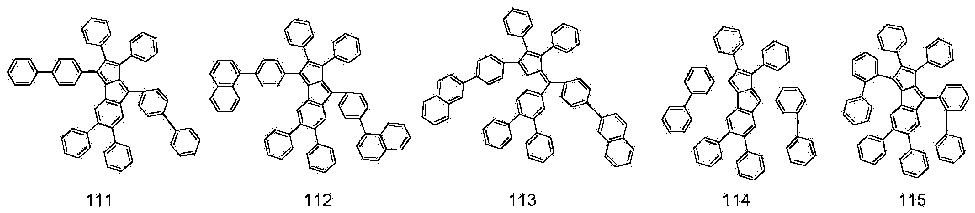

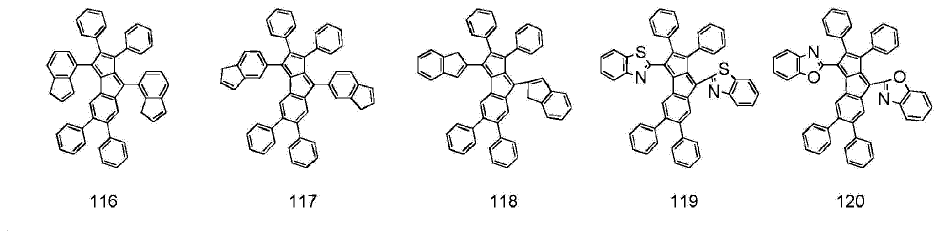

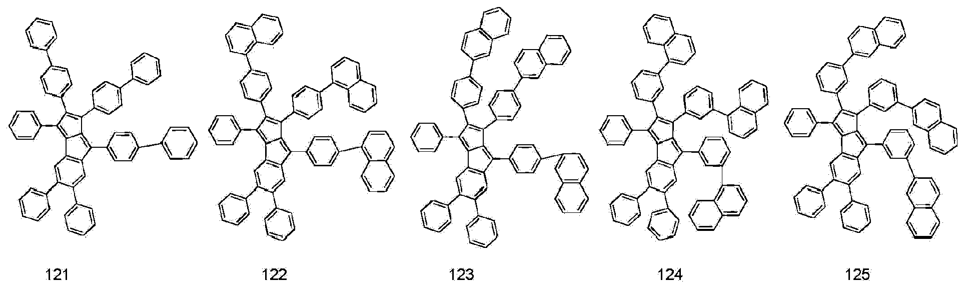

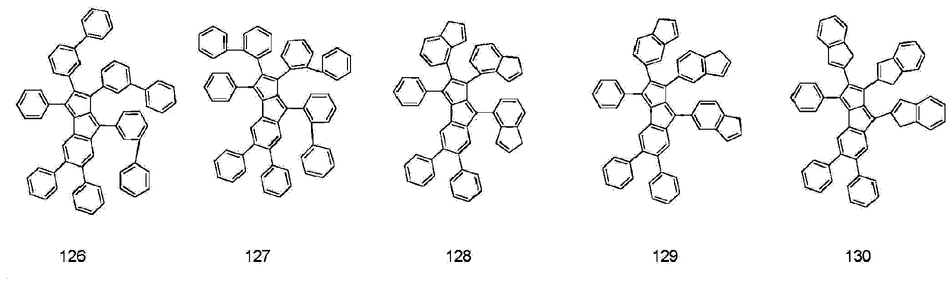

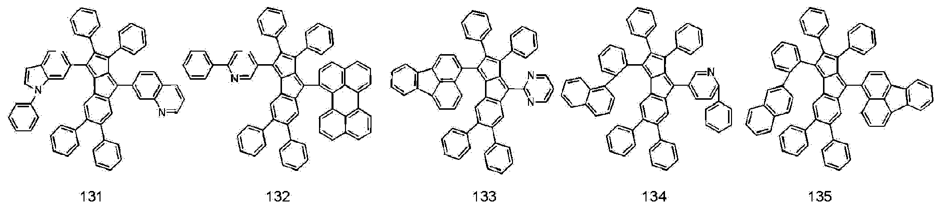

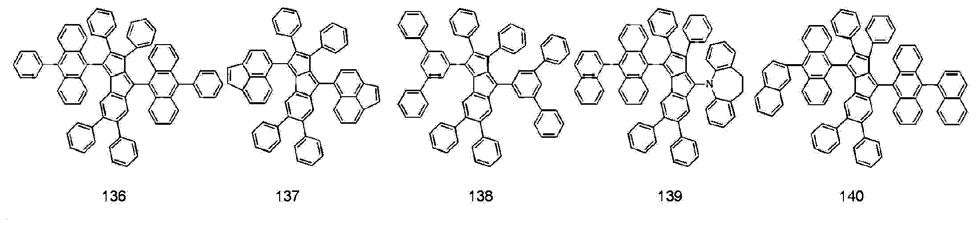

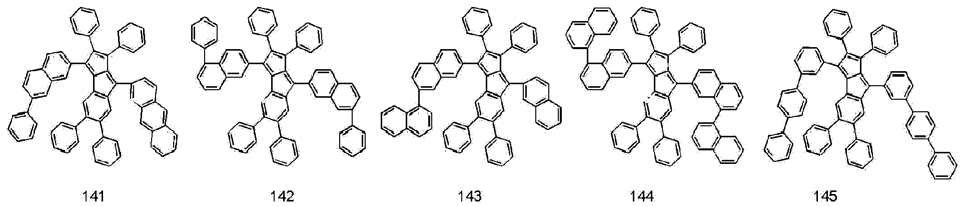

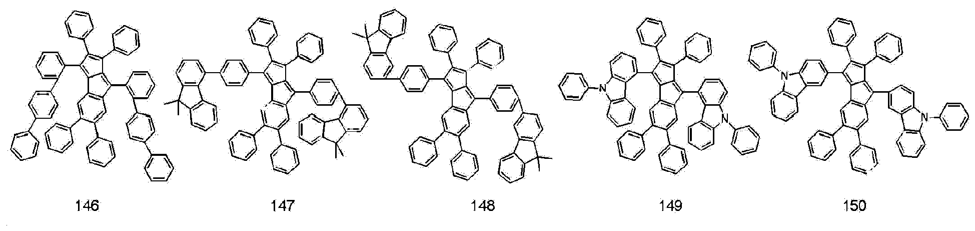

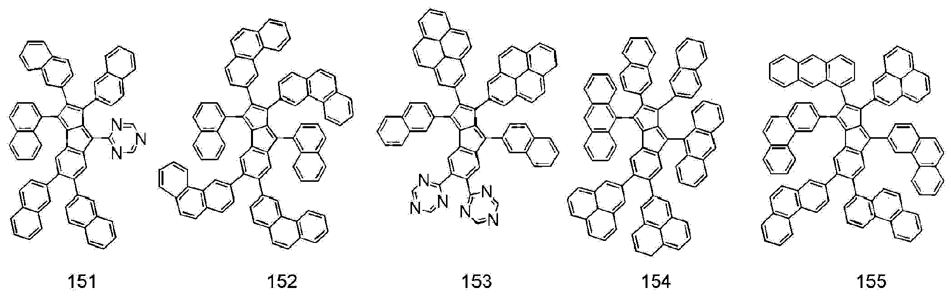

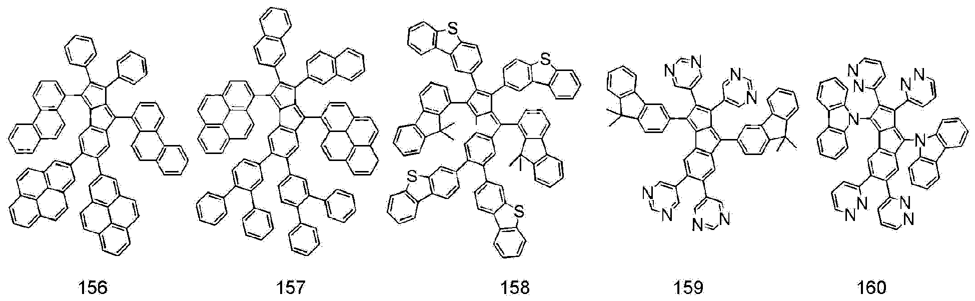

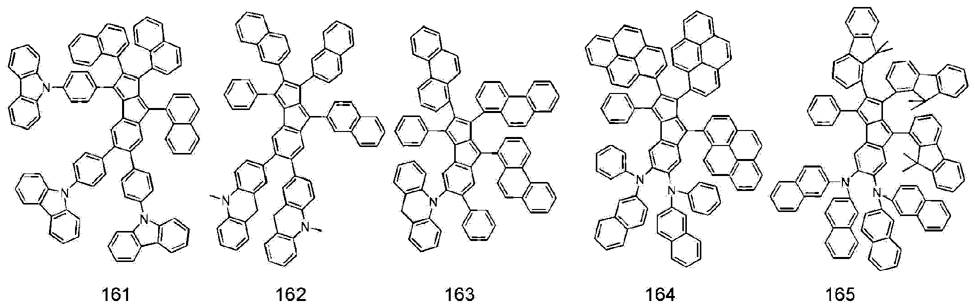

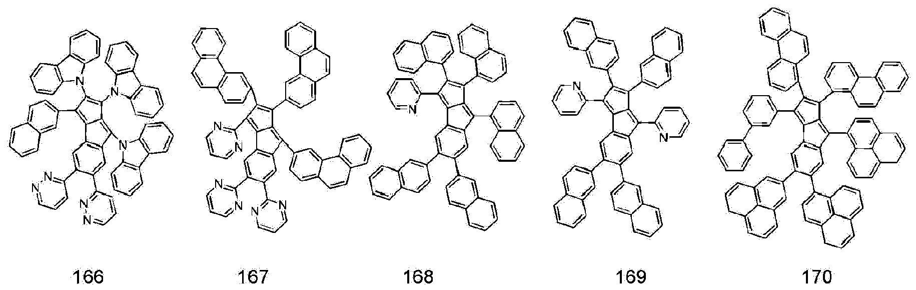

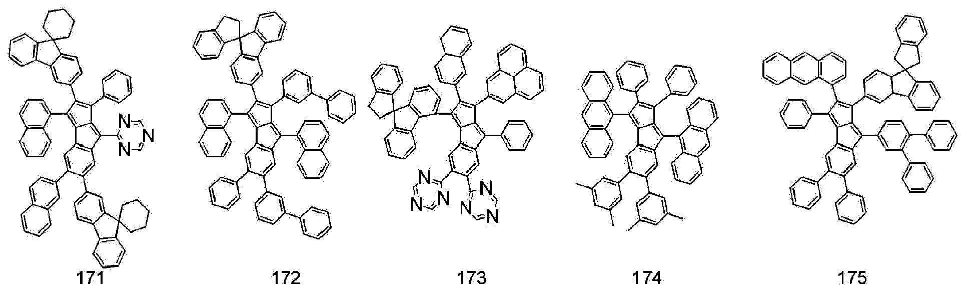

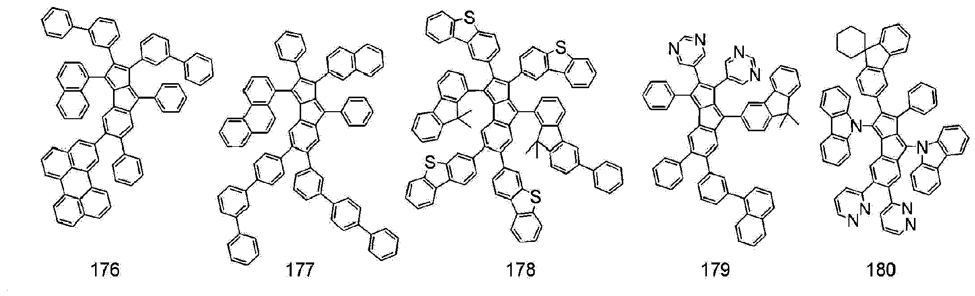

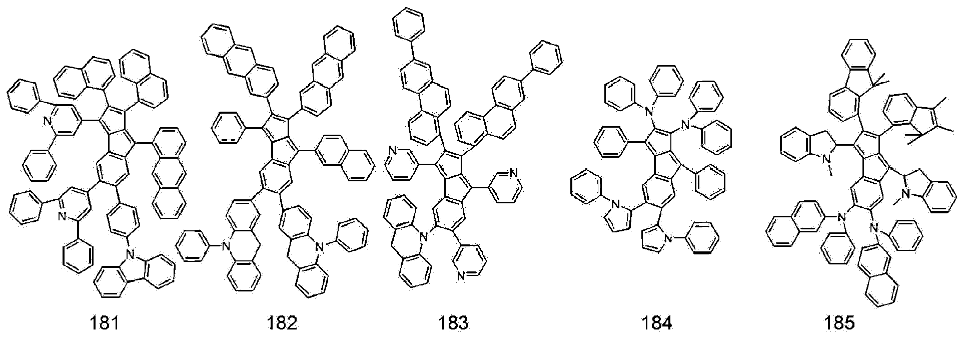

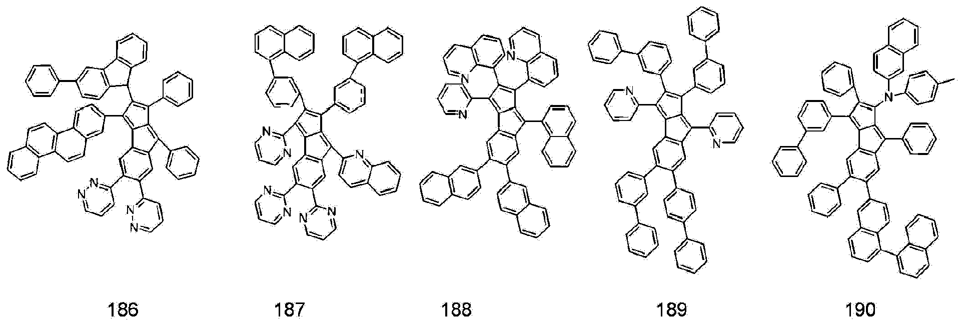

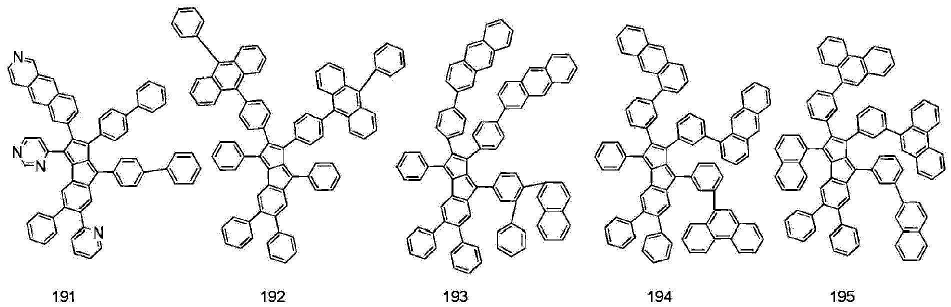

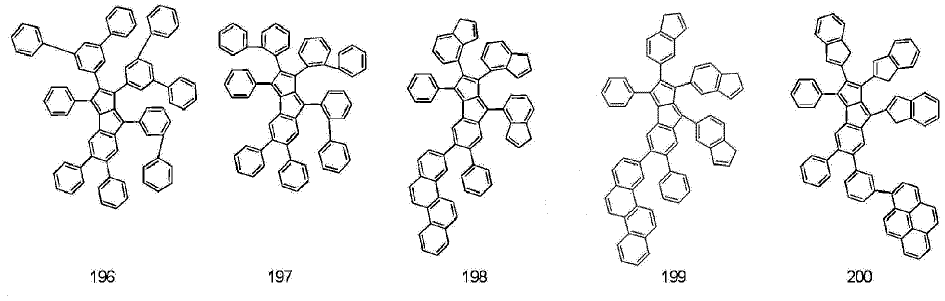

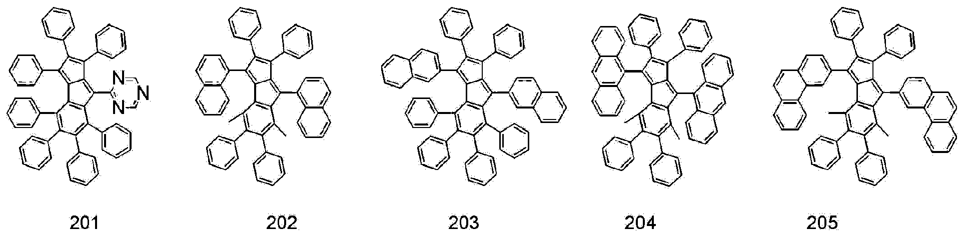

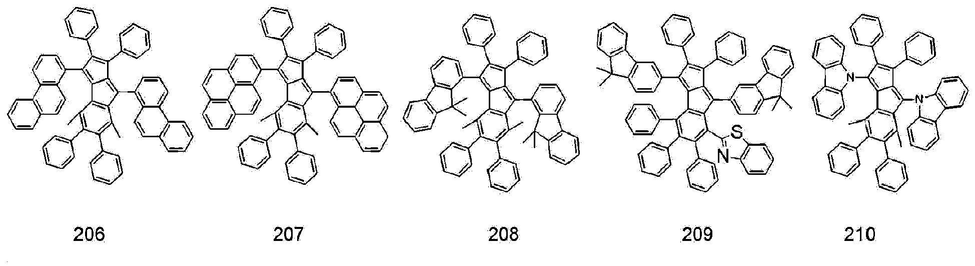

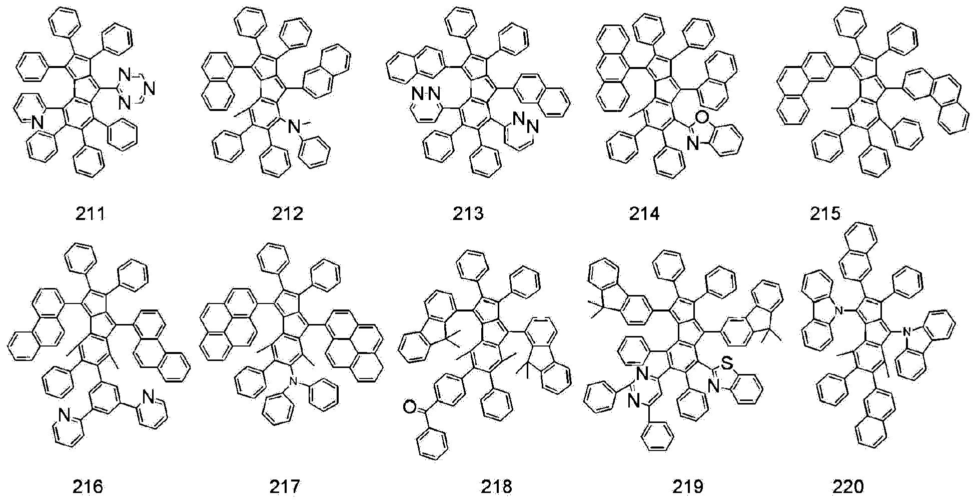

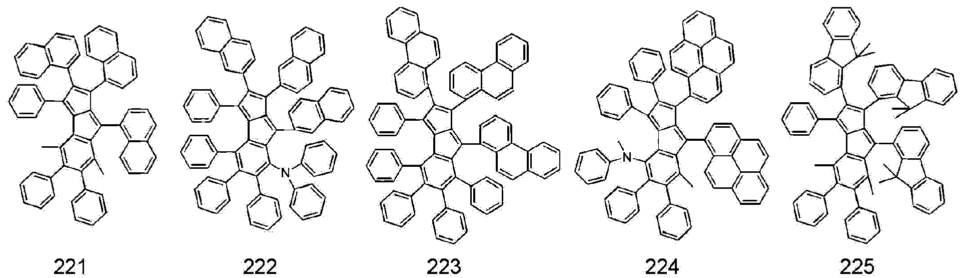

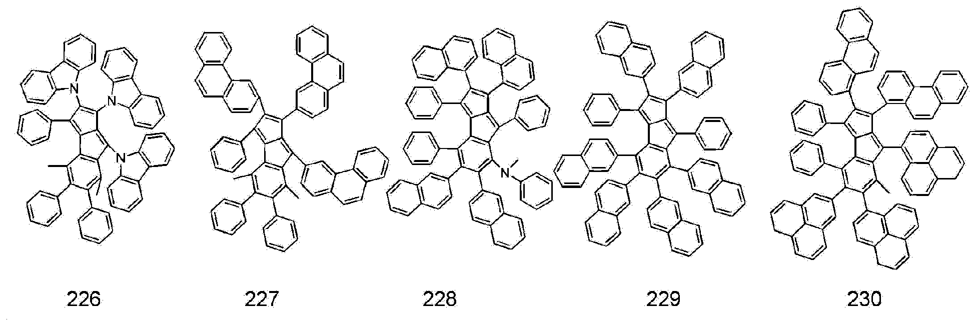

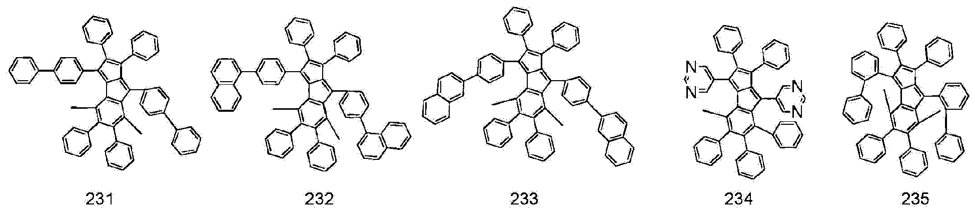

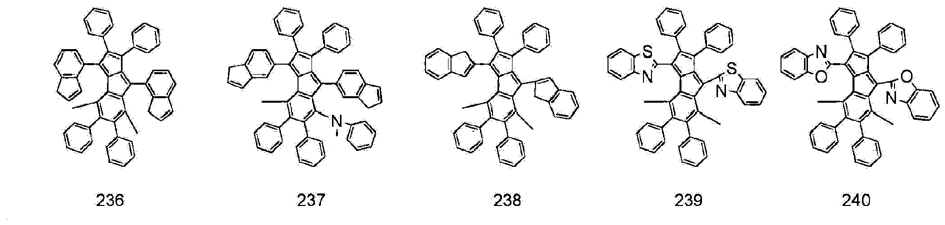

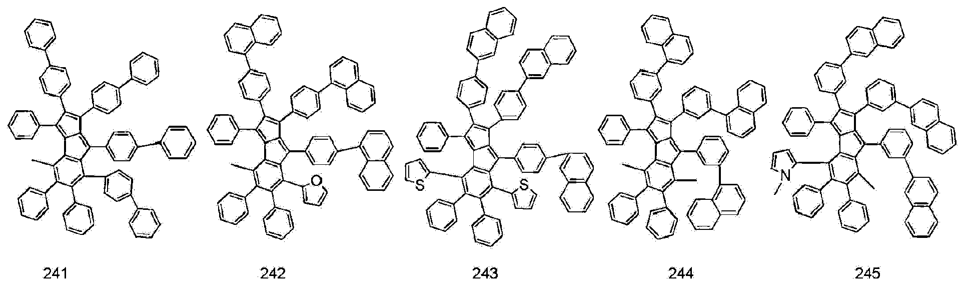

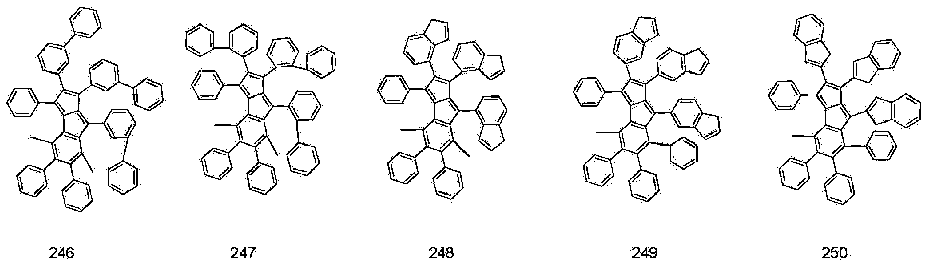

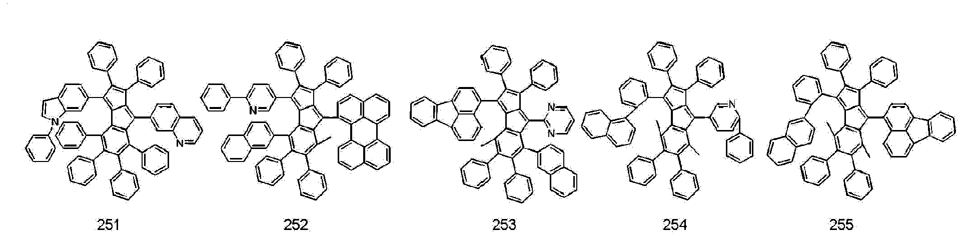

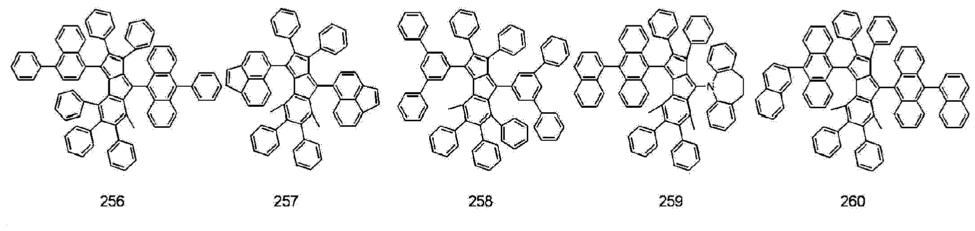

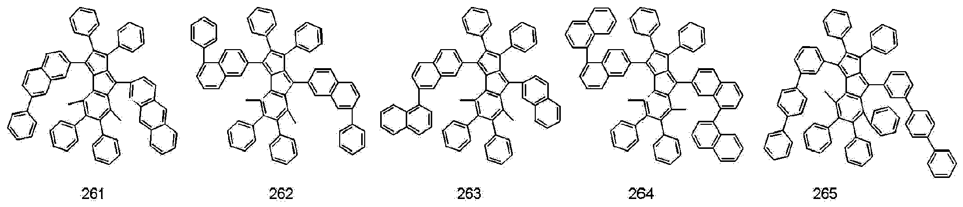

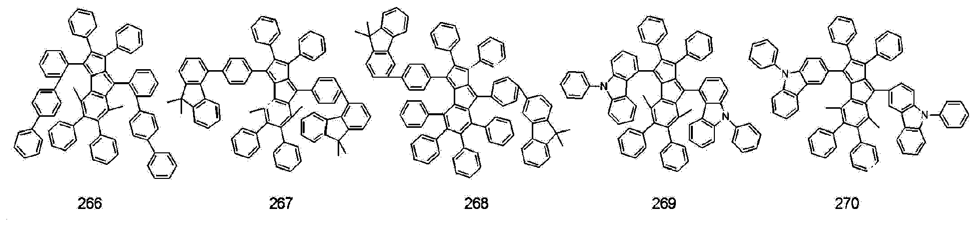

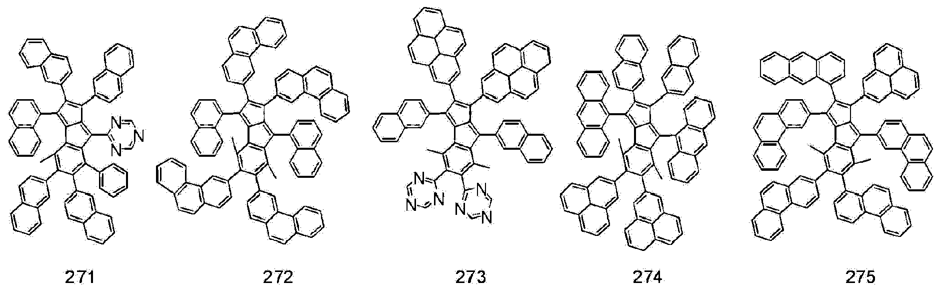

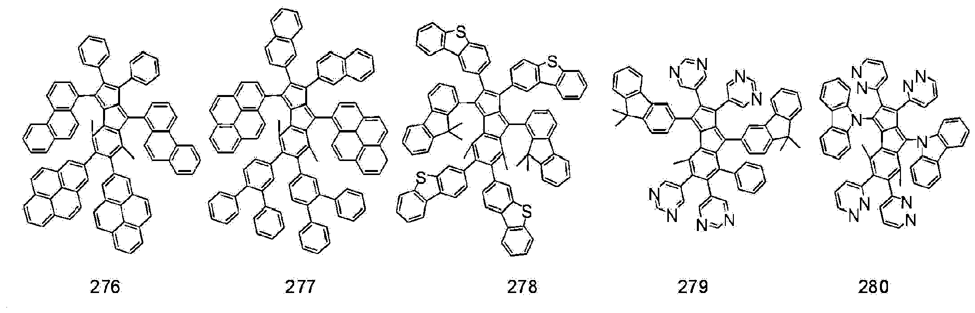

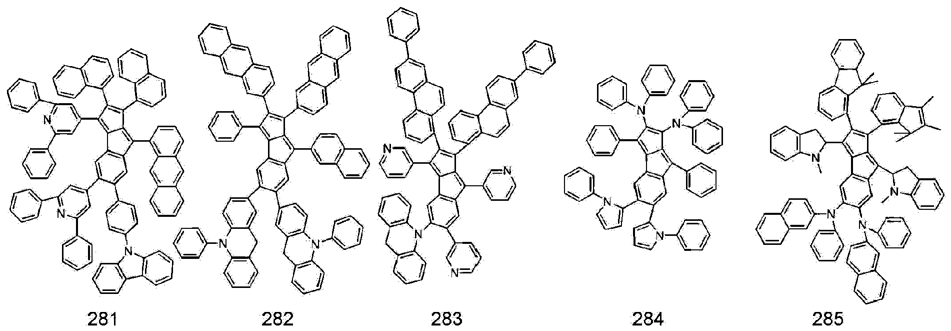

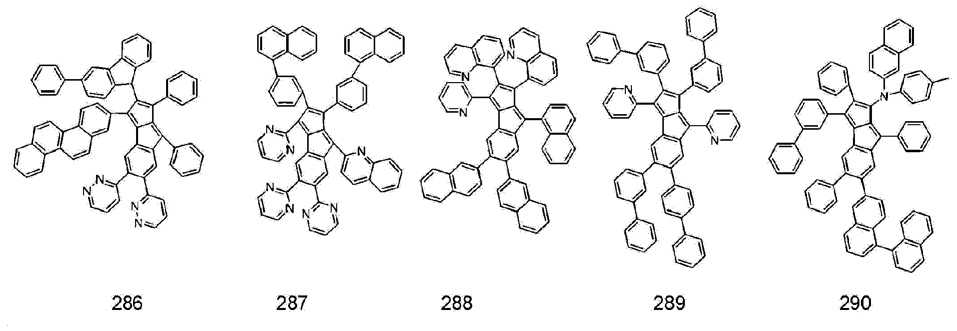

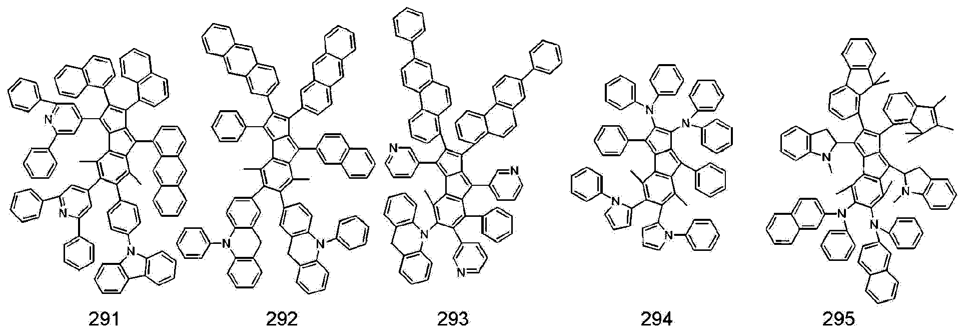

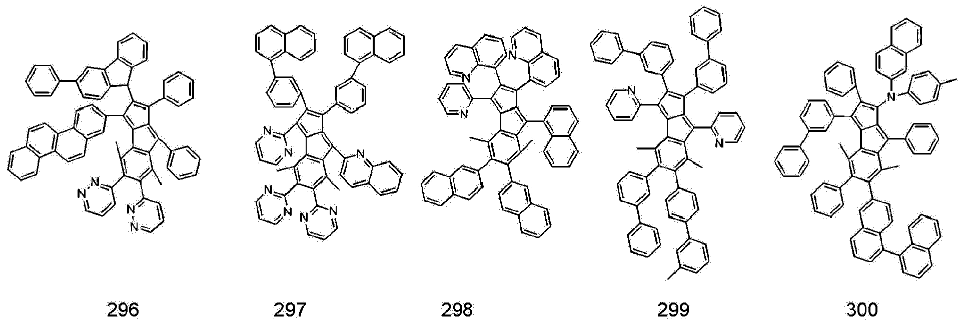

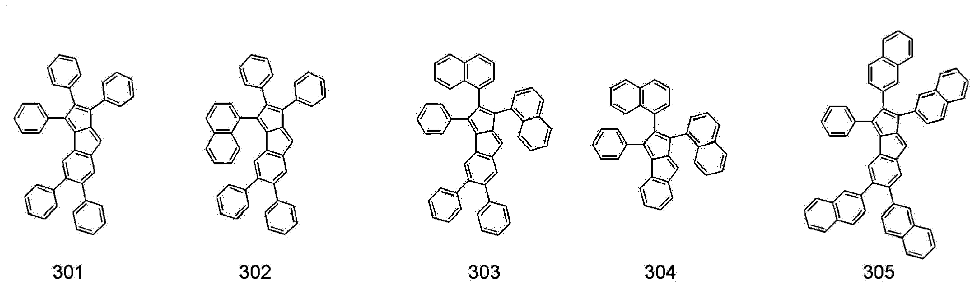

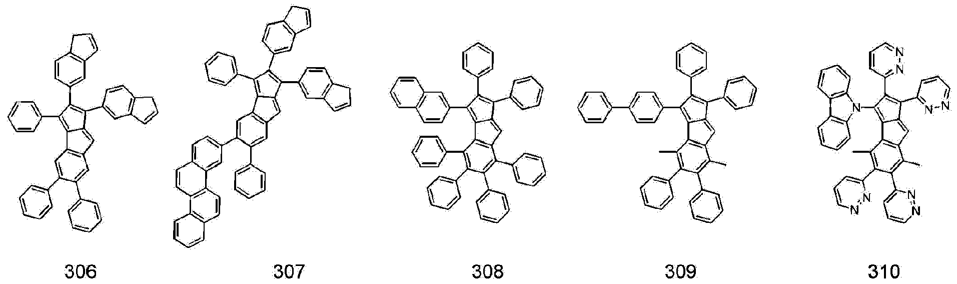

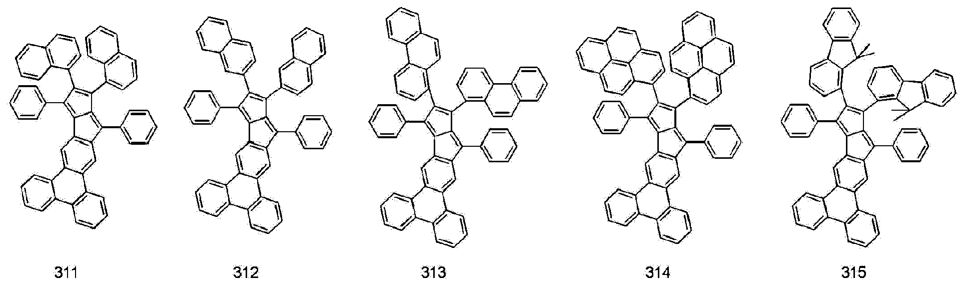

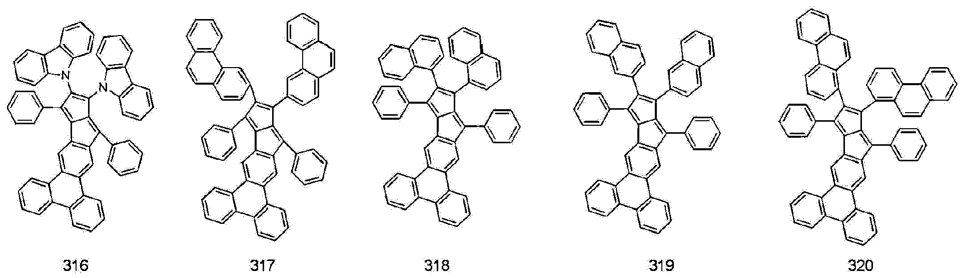

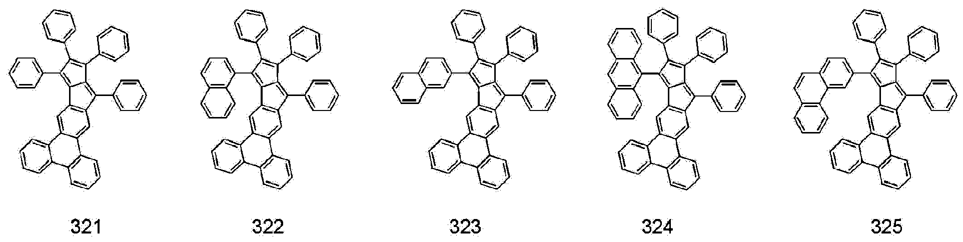

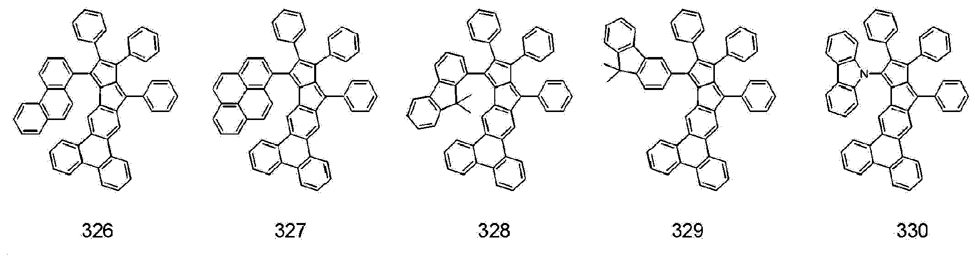

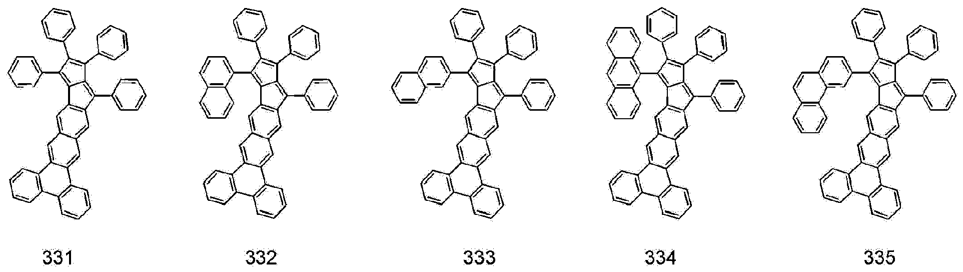

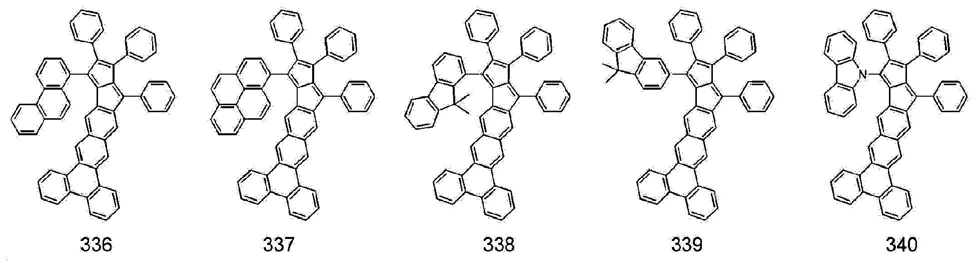

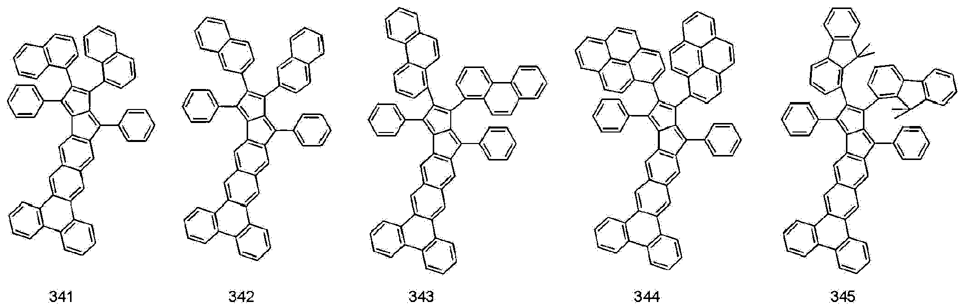

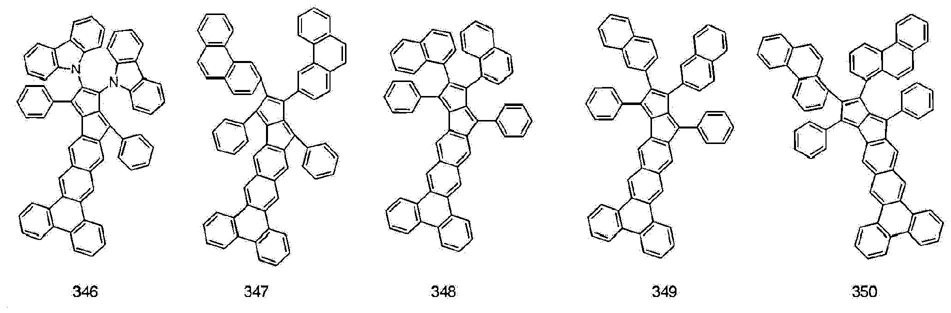

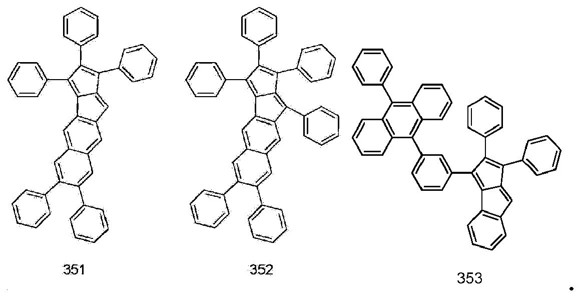

9. An organic light emitting device comprising:
an anode;
a cathode; and
one or more organic material layers provided between the anode and the cathode,
wherein, one or more layers of the organic material layers include the compound of Chemical Formula 1 according to any one of Claims 1 to 8.

10. The organic light emitting device of Claim 9, wherein the organic material layer including the compound of Chemical Formula 1 is a light emitting layer.
